# EUROPEAN PATENT APPLICATION

(11) **EP 1 441 010 A1**
(43) Date of publication of application: **28.07.2004**
(21) Application number: 04076071.2
(22) Date of filing: 11.04.1997
(51) Int. Cl.: C09B 11/00, C09B 11/24, G01N 1/30, C07C 209/36, C07C 245/20, C07C 37/00

(54) **Fluorinated xanthene derivatives**

(30) Priority: 12.04.1996 US 631202
(62) Divisional of application: 97922299.9
(71) Applicant: Molecular Probes, Inc., Eugene, OR 97402-9132 (US)
(72) Inventor: Gee, Kyle R., Springfield, OR 97477 (US); Klaubert, Dieter H., Eugene, OR 97405 (US); Haugland, Richard P., Eugene, OR 97405 (US); Poot, Martin, Seattle, WA 98105 (US); Sun, Wei-Chuan, Eugene, OR 97405 (US); Mao, Fei, Eugene, OR 97405 (US)
(74) Representative: Harrison Goddard Foote

(57) **Abstract**

The family of dyes of the invention are fluoresceins and rhodols that are directly substituted on one or more aromatic carbons by fluorine. These fluorine-substituted fluorescent dyes possess greater photostability and have lower sensitivity to pH changes in the physiological range of 6-8 than do non-fluorinated dyes, exhibit less quenching when conjugated to a substance, and possess additional advantages. The dyes of the invention are useful as detectable tracers and for preparing conjugates of organic and inorganic substances.

## Description

### FIELD OF THE INVENTION

The invention relates to novel fluorinated xanthene dyes (including fluorescein and rhodol dyes), reactive dye derivatives, dye-conjugates and dyes that are enzyme substrates; and to their use. Additionally a facile synthesis for fluorinated resorcinols and aminophenols is provided.

### BACKGROUND OF THE INVENTION

Fluorescent dyes are known to be particularly suitable for biological applications in which a highly sensitive detection reagent is desirable. Fluorescent dyes are used to impart both visible color and fluorescence to other materials. The dyes of this invention are fluorine-substituted analogs of xanthene-based dyes that are typically fluorescein or rhodol derivatives.

"Fluorescein" dyes include derivatives of 3*H*-xanthen-6-ol-3-one that are substituted at the 9-position by a 2-carboxyphenyl group, while "rhodol dyes" include derivatives of 6-amino-3*H*-xanthen-3-one that are substituted at the 9-position by a 2-carboxyphenyl group.

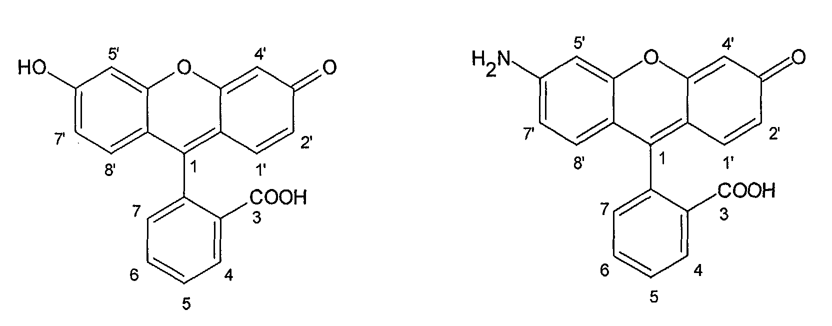

Rhodols and fluoresceins are typically substituted at the 1-position by a derivative capable of forming a 5- or 6-membered lactone or lactam ring:

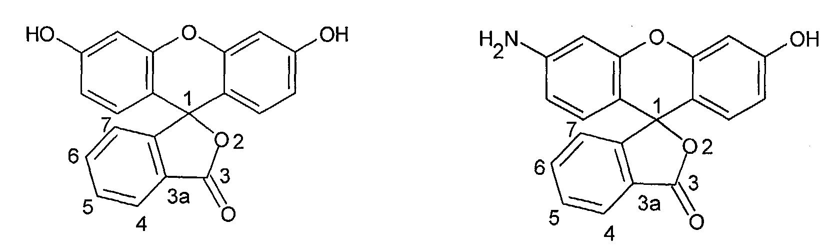

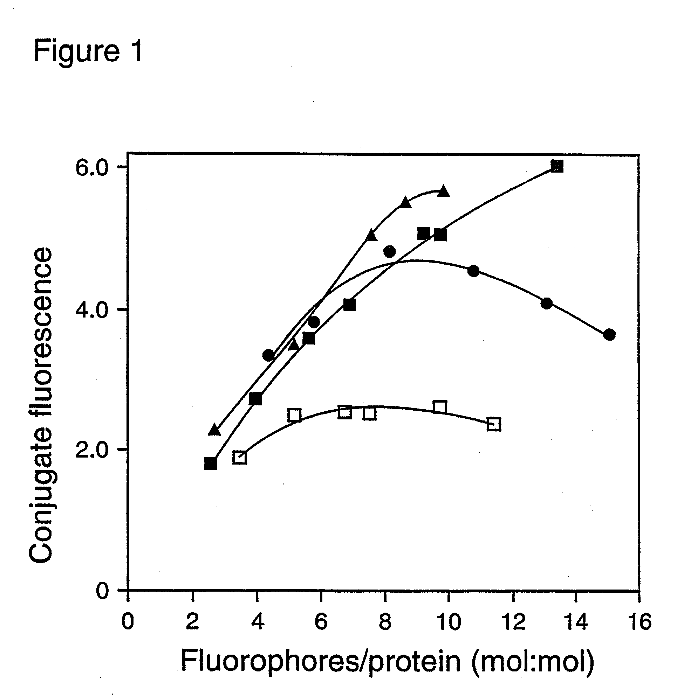

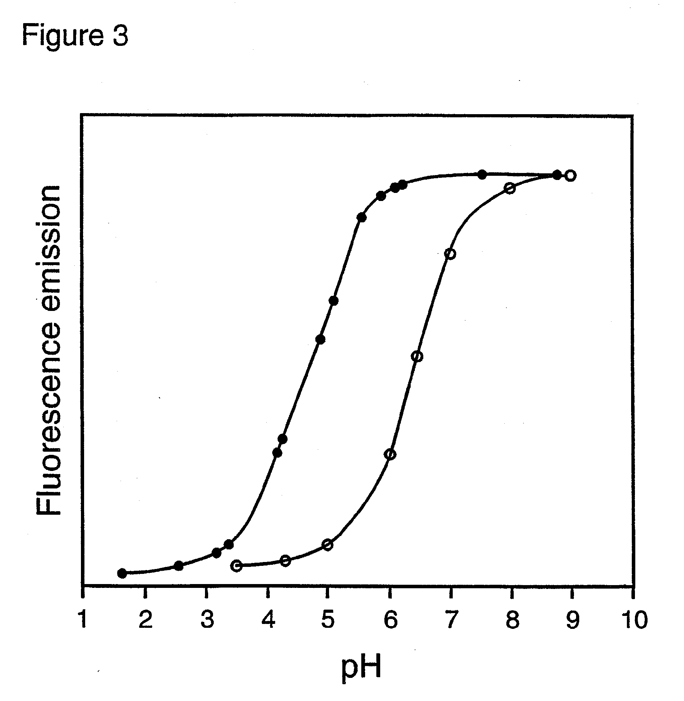

Despite the known tendency of halogenation to shift the wavelength of fluoresceins, new fluorinated fluoresceins retain the favorable characteristics of fluorescein, including high absorbance and fluorescence, excitation at 488 nm (e.g. argon laser), and compatibility with standard microscope filters; with demonstrated advantages over fluorescein, including greater photostability and a significantly lower pKₐ (>1.5 pH units on the otherwise unsubstituted dyes, see Figure 3). The improved properties of the fluorinated dyes are retained in their conjugates and there is decreased quenching of dye on conjugates (e.g. Figure 1). The new materials are suitable for use in known applications of fluoresceins and rhodols except those requiring sensitivity to pH changes near pH 7. Additionally, polyfluorination of the phenyl substituent present in most fluorescein and rhodol dyes provides a novel route to reactive derivatives and conjugates. Certain fluorinated xanthenes are unexpectedly well-retained in cells.

The unique chemistry of fluorine is incompatible with methods that are typically used to prepare chlorine, bromine or iodine substituted fluorescein derivatives, and efficient methods for preparing fluororesorcinol and fluoroaminophenol intermediates have not previously been described. A novel means of preparing 1'- and/or 8'-substituted xanthenes is also described.

### DESCRIPTION OF DRAWINGS

Figure 1: Fluorescence versus degree of substitution for dye-conjugates of goat anti-mouse immunoglobulin G (GAM IgG). Fluorescence is determined by measuring the quantum yield of the dye-conjugate relative to the free dye and multiplying by the number of fluorophores per protein: Compound **109** (▲), Compound **64** (■), Compound **61** (●) and fluorescein isothiocyanate (FITC, □).
Figure 2: Relative photostability of dye-conjugates of GAM IgG. The conjugates of fluorinated dyes exhibit enhanced photostability relative to non-fluorinated dyes: Compound **59** (**■**), Compound **109** (▲), Compound **35** (□) and 6-carboxyfluorescein (●).
Figure 3: The effect of fluorination on pH-dependent fluorescence: Compound **35** (●), and 6-carboxyfluorescein (○) (excitation/emission at 490/520 nm).
Figure 4: Relative photostability of phalloidin conjugates of 6-carboxymethylthio-2',4,5,7,7'-pentafluorofluorescein (■) and 6-carboxyfluorescein (●).
Figure 5: Retention of fluorescence in cells treated with 1) Compound **25**, 2) fluorescein diacetate (FDA) and 3) Compound **26.**

### SUMMARY OF THE INVENTION AND DESCRIPTION OF PREFERRED EMBODIMENTS

The dyes of the invention are fluoresceins and rhodols that are directly substituted at one or more aromatic carbons by fluorine. In describing the dyes, carboxy means -COOH, biologically compatible salt of a carboxylic acid, or a biologically compatible ester of a carboxylic acid; sulfo means -SO₃H, or a biologically compatible salt of a sulfonic acid. A biologically compatible salt means a salt that is not deleterious to biological systems, including K⁺, Na⁺, Cs⁺, Li⁺, Ca²⁺, Mg²⁺, and NR₄⁺ salts, where R = H, C₁-C₄ alkyl, or C₂-C₄ alkanol or combinations thereof. A biologically compatible ester means a readily hydrolyzable ester used in biological systems, e.g. α-acyloxyalkyl esters (typically -CH₂-O-(C=O)-R¹⁸, where R¹⁸ is C₁₋₄ alkyl, especially acetoxymethyl (CH₃CO₂CH₂-) esters).

In one embodiment, the dyes have the formula

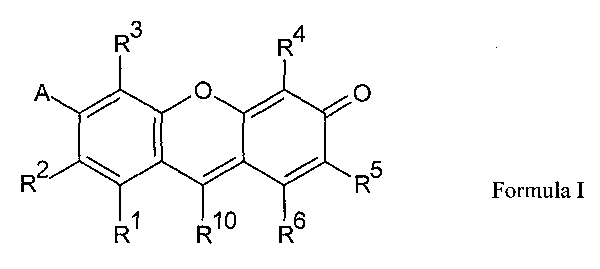

Substituents R¹ and R⁶ are independently H, F, Cl, Br, I, C₁-C₁₈ alkyl or C₁-C₁₈ alkoxy. Typically, R¹ and R⁶ are H or F.

Substituents R², R³, R⁴ and R⁵ are independently H, F, Cl, Br, I or CN, or a C₁-C₁₈ alkyl, alkoxy, or alkylthio group. An alkyl, alkoxy, or alkylthio dye substituent is optionally further substituted by F, Cl, Br, I, carboxy, sulfo, amino, alkylamino, dialkylamino or alkoxy, the alkyl portions of each independently having 1-6 carbons. Additionally, dye substituents R³ and R⁴ are optionally each -CH₂-N-(CR¹⁹HCOOR¹⁷)₂, where R¹⁷ is H, a biologically compatible counterion, a linear or branched alkyl having 1-6 carbons, or an α-acyloxyalkyl ester, and R¹⁹ is H or C₁-C₆ alkyl.

Substituent A is -NR⁸R⁹ or -OR⁷, where R⁷ is H, C₁-C₁₈ alkyl or C₁-C₁₈ acyl that is optionally substituted by amino, hydroxy or carboxy, or R⁷ is a trialkylsilyl moiety, the alkyl portions of which have 1-6 carbons. Substituents R⁸ and R⁹ are independently H, an alkyl having 1-6 carbons, or acyl having 1-18 carbons. Where one or both of R⁸ and R⁹ are alkyl or acyl, the alkyl portions are optionally further substituted by carboxy or sulfo. Additionally, R⁸ when taken in combination with R², or R⁹ when taken in combination with R³, or both, form a saturated 5- or 6-membered ring that is optionally substituted by one or more methyls. Alternatively, R⁸ and R⁹, when taken in combination, form a saturated 5- or 6-membered heterocycle that is a piperidine, a morpholine, a pyrrolidine or a piperazine that is optionally substituted by methyl, or carboxy.

Substituent R¹⁰ is F, carboxy, or a C₁-C₁₈ alkyl, alkenyl or alkynyl group. Where R¹⁰ is alkyl, it is optionally substituted by F, Cl, Br, carboxy, sulfo, amino, alkylamino or dialkylamino where the alkyl portions of each substituent have 1-6 carbons. Alternatively, R¹⁰ is an aryl: 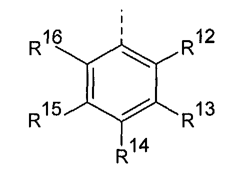 (sometimes referred to as the "bottom ring"). Substituents R¹², R¹³, R¹⁴, R¹⁵ and R¹⁶ are independently H, F, Cl, Br, I, carboxy, sulfo, CN, nitro, hydroxy, azido, amino or hydrazino, or C₁-C₁₈ alkyl, alkoxy, alkylthio, alkylamino, alkylester, alkylamido or arylamido, the alkyl or aryl portions of which are optionally substituted by F, Cl, Br, I, hydroxy, carboxy, sulfo, amino, alkylamino, dialkylamino or alkoxy, the alkyl portions of which have 1-6 carbons. Alternatively, any two adjacent substituents of R¹³, R¹⁴, R¹⁵ and R¹⁶, when taken in combination, form a fused 6-membered aromatic ring (making R¹⁰ a naphthyl) that is optionally further substituted by carboxy.

In one embodiment, R¹⁰ is a substituted aryl, preferably phenyl, R¹² is a carboxy, and R¹³, R¹⁴, R¹⁵ and R¹⁶ are independently H, F, or carboxy. In another embodiment, R¹² is a carboxy or a sulfo, and none of R¹³, R¹⁴, R¹⁵ or R¹⁶ is F. In yet another embodiment, three of R¹³, R¹⁴, R¹⁵ and R¹⁶ are F, and R¹² is a carboxy or a sulfo.

In another embodiment, the dyes have the formula 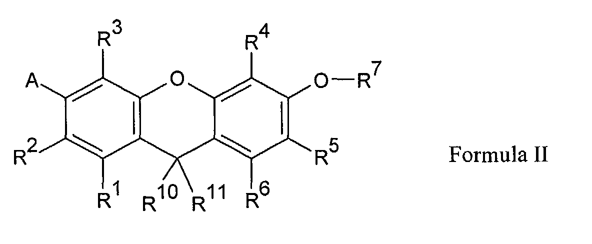 where substituents R¹⁻⁷, R¹⁰, and A are as defined previously, and R¹¹ is H, hydroxy, CN or C₁₋₆ alkoxy, or R¹⁰ and R¹¹ form a 5-membered spirolactone ring, or R¹¹ and R¹² form a 5- or 6-membered spirolactone ring, or a 5- or 6-membered sultone ring, for example (additional substituents are not shown): 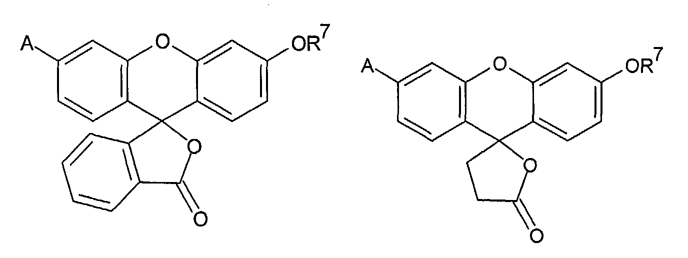 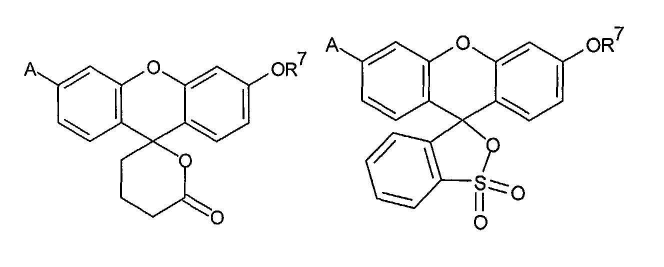 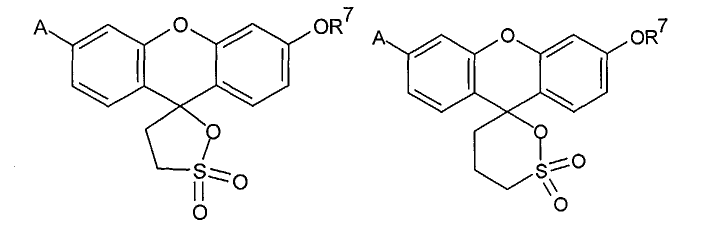 The methylene carbons of the spirolactone ring or spirosultone ring are optionally and independently substituted by H, F or CH₃.

Alternatively, R¹⁰ together with R¹¹ is a carbonyl oxygen, according to the simplified formula below (additional substituents are not shown).

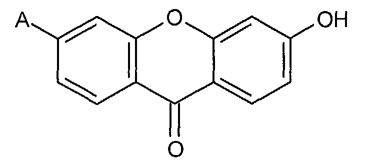

Dye embodiments that incorporate a spirolactone ring are representative of a structural isomer that may exist in equilibrium with the isomer wherein R¹² is a carboxylic acid, or R¹⁰ is a propionic or butyric acid. Dyes that incorporate a spirosultone ring may exist in equilibrium with the isomer wherein R¹² is a sulfonic acid, or R¹⁰ is a sulfonic acid-substituted ethyl or propyl. Isomers that incorporate a spirolactone or spirosultone ring are non-fluorescent until the ring is opened.

Where A is OR⁷, R¹⁰ is aryl and R¹² is carboxy, the described dye is a fluorescein (Formula I) or a dihydrofluorescein (Formula II). Where A is NR⁸R⁹, R¹⁰ is aryl and R¹² is carboxy, the described dye is a rhodol (Formula I) or a dihydrorhodol (Formula II).

For all embodiments of the invention, at least one of R¹, R², R³, R⁴, R⁵, R⁶, R¹⁰, R¹², R¹³, R¹⁴, R¹⁵ or R¹⁶ is fluorine (F). The fluorinated xanthenes optionally possess a reactive group that is bound to the fluorophore by a single bond or a linking moiety, and may be used to prepare a dye-conjugate. The dyes optionally contain an R⁷ substituent that makes the dye photoactivatible or more cell-permeant, or makes the dye a substrate for an enzyme, or modifies its spectral properties.

In one embodiment, one or more of R¹, R², R³, R⁴, R⁵ and R⁶ is F. In an additional embodiment, where at least one of R², R³, R⁴ and R⁵ is F, none of R¹², R¹³, R¹⁴, R¹⁵ and R¹⁶ is F. In yet another embodiment, where at least one of R², R³, R⁴ and R⁵ is F, at least one of R¹², R¹³, R¹⁴, R¹⁵ and R¹⁶ is F. In another embodiment at least one of the substituents (other than R³ and R⁴) is F. Alternatively, R¹ and R⁶ are H, and R², R³, R⁴, and R⁵ are H, F, Cl, Br, I, or C₁-C₆ alkoxy, provided that at least one of the substituents is F. In another embodiment, at least one of R¹², R¹³, R¹⁴, R¹⁵ and R¹⁶ is F. In one embodiment, four of R¹², R¹³, R¹⁴, R¹⁵ and R¹⁶ is F. In another embodiment, each of R¹³, R¹⁴, R¹⁵ and R¹⁶ is F. In another embodiment, where at least one of R¹², R¹³, R¹⁴, R¹⁵ and R¹⁶ is F, none of R¹, R², R³, R⁴, R⁵ and R⁶ is F. In yet another embodiment, R¹⁰ is F.

Typically, where A = OR⁷, either R² and R⁵ are F, or R³ and R⁴ are F, or R², R³, R⁴ and R⁵ are all F. Where A = NR⁸R⁹, preferably R⁵ is F, resulting in a drop in the pKa; where R² is F, the pKa is comparable to that of the non-fluorinated compound. For both A = NR⁸R⁹ and A= OR⁷, where each of R¹³ through R¹⁶ is fluorinated, the resulting dyes react readily with nucleophiles, and where in addition R¹¹ and R¹², taken in combination, are spirolactone, the resulting dyes are well retained in cells or attach readily to biological molecules.

Preferred dyes possess a quantum yield in aqueous solution (pH = 6) of greater than 0.50. Preferably these dyes possess a pKa of less than 5.0. The extinction coefficient of preferred dyes measured at a wavelength greater than 490 nm at pH 6.0 is greater than 60,000 cm⁻¹M⁻¹. Preferably, the fluorinated dyes exhibit a fluorescence emission maximum that is shifted less than 15 nm relative to that of the non-fluorinated analog. Spectral properties of selected dyes are given in Table 1.

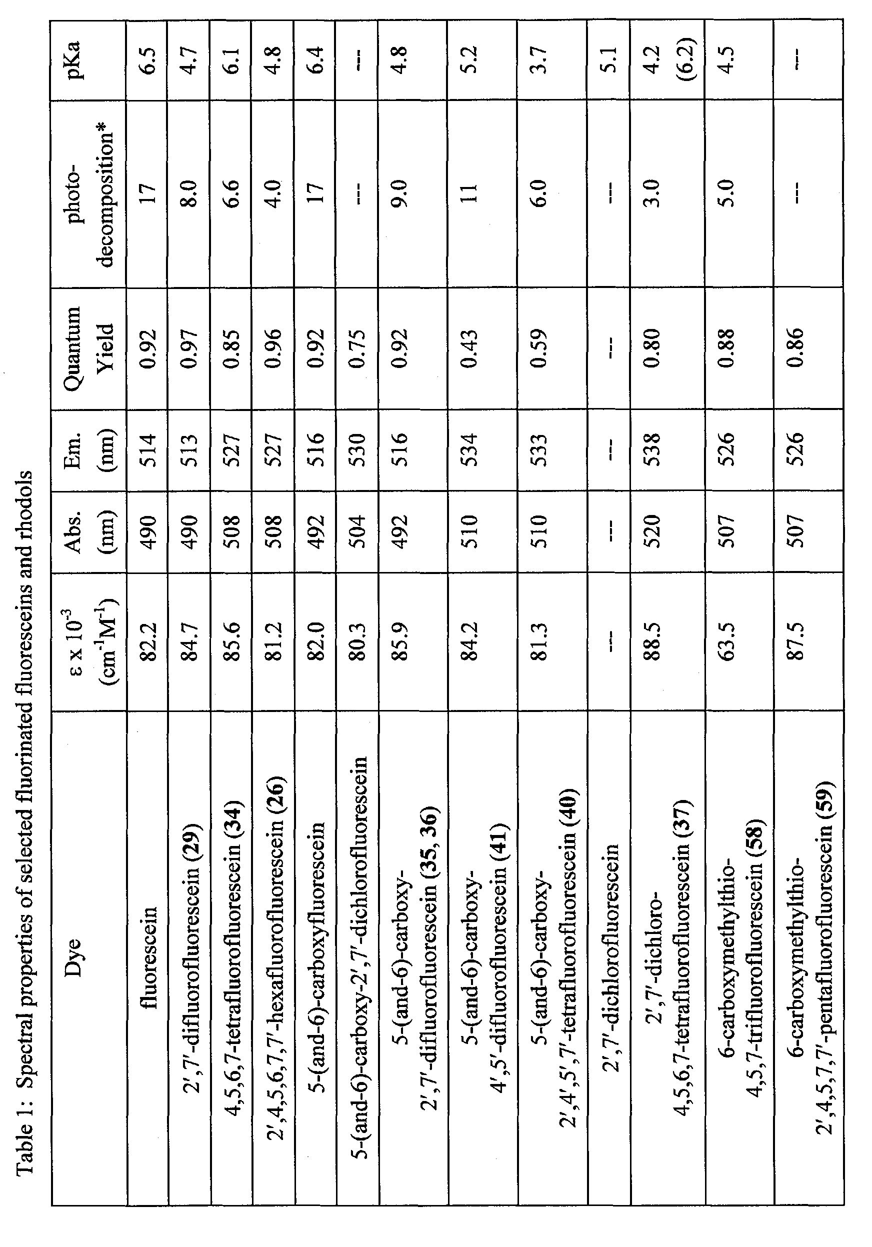

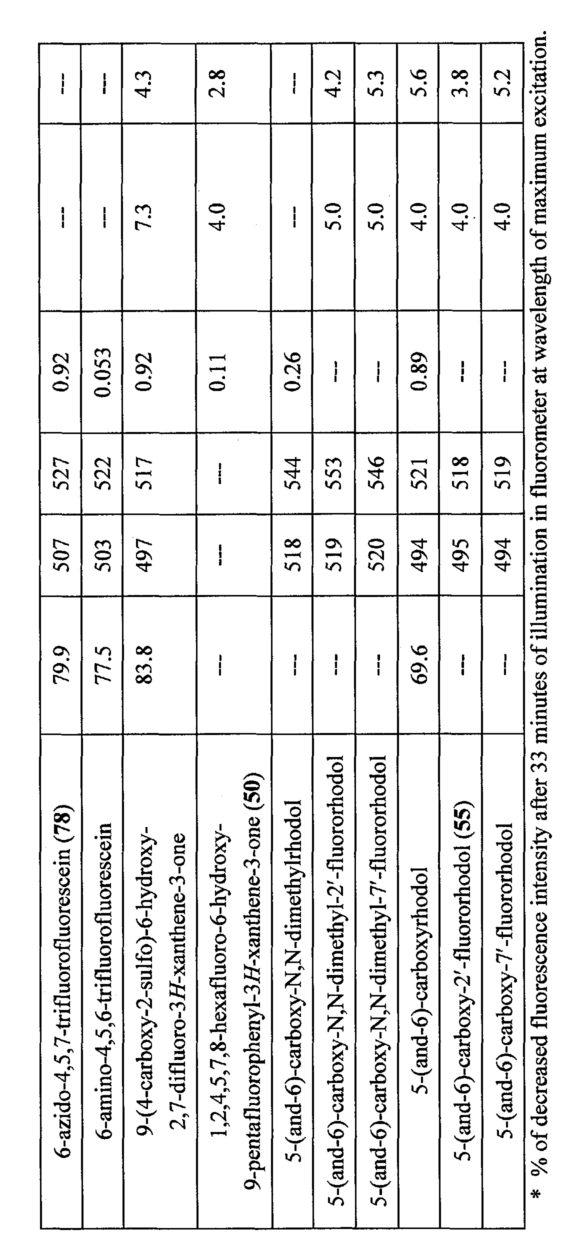

### Conjugates of Reactive Dyes

One embodiment of the fluorinated fluorophore contains at least one group -L-Rₓ, where Rₓ is the reactive group that is attached to fluorophore by a covalent linkage L. In certain embodiments (see Table 9) Rₓ is attached to the dye by multiple intervening atoms that serve as a spacer. The dyes with a reactive group (Rₓ) fluorescently label a wide variety of organic or inorganic substances that contain or are modified to contain functional groups with suitable reactivity, resulting in chemical attachment of the conjugated substance (S_{c}), represented by -L-S_{c}. The reactive group and functional group are typically an electrophile and a nucleophile that can generate a covalent linkage. Alternatively, the reactive group is a photoactivatable group, and becomes chemically reactive only after illumination with light of an appropriate wavelength. Selected examples of functional groups and linkages are shown in Table 2, where the reaction of an electrophilic group and a nucleophilic group yields a covalent linkage.

**TABLE 2:**

| Examples of some routes to useful covalent linkages | | |
|---|---|---|
| Electrophilic Group | Nucleophilic Group | Resulting Covalent Linkage |
| activated esters* | amines/anilines | carboxamides |
| acyl azides** | amines/anilines | carboxamides |
| acyl halides | amines/anilines | carboxamides |
| acyl halides | alcohols/phenols | esters |
| acyl nitriles | alcohols/phenols | esters |
| acyl nitriles | amines/anilines | carboxamides |
| aldehydes | amines/anilines | imines |
| aldehydes or ketones | hydrazines | hydrazones |
| aldehydes or ketones | hydroxylamines | oximes |
| alkyl halides | amines/anilines | alkyl amines |
| alkyl halides | carboxylic acids | esters |
| alkyl halides | thiols | thioethers |
| alkyl halides | alcohols/phenols | ethers |
| alkyl sulfonates | thiols | thioethers |
| alkyl sulfonates | carboxylic acids | esters |
| alkyl sulfonates | alcohols/phenols | ethers |
| anhydrides | alcohols/phenols | esters |
| anhydrides | amines/anilines | carboxamides |
| aryl halides | thiols | thiophenols |
| aryl halides | amines | aryl amines |
| aziridines | thiols | thioethers |
| boronates | glycols | boronate esters |
| carboxylic acids | amines/anilines | carboxamides |
| carboxylic acids | alcohols | esters |
| carboxylic acids | hydrazines | hydrazides |
| carbodiimides | carboxylic acids | N-acylureas or anhydrides |
| diazoalkanes | carboxylic acids | esters |
| epoxides | thiols | thioethers |
| haloacetamides | thiols | thioethers |
| halotriazines | amines/anilines | aminotriazines |
| halotriazines | alcohols/phenols | triazinyl ethers |
| imido esters | amines/anilines | amidines |
| isocyanates | amines/anilines | ureas |
| isocyanates | alcohols/phenols | urethanes |
| isothiocyanates | amines/anilines | thioureas |
| maleimides | thiols | thioethers |
| phosphoramidites | alcohols | phosphite esters |
| silyl halides | alcohols | silyl ethers |
| sulfonate esters | amines/anilines | alkyl amines |
| sulfonate esters | thiols | thioethers |
| sulfonate esters | carboxylic acids | esters |
| sulfonate esters | alcohols | ethers |
| sulfonyl halides | amines/anilines | sulfonamides |
| sulfonyl halides | phenols/alcohols | sulfonate esters |

| | | |
|---|---|---|
| * Activated esters, as understood in the art, generally have the formula -COΩ, where Ω is a good leaving group (e.g. oxysuccinimidyl (-OC₄H₄O₂) oxysulfosuccinimidyl (-OC₄H₃O₂-SO₃H), -1-oxybenzotriazolyl (-OC₆H₄N₃); or an aryloxy group or aryloxy substituted one or more times by electron withdrawing substituents such as nitro, fluoro, chloro, cyano, or trifluoromethyl, or combinations thereof, used to form activated aryl esters; or a carboxylic acid activated by a carbodiimide to form an anhydride or mixed anhydride -OCOR^{a} or -OCNR^{a}NHR^{b}, where R^{a} and R^{b}, which may be the same or different, are C₁-C₆ alkyl, C₁-C₆ perfluoroalkyl, or C₁-C₆ alkoxy; or cyclohexyl, 3-dimethylaminopropyl, or N-morpholinoethyl). | | |
| * Acyl azides can also rearrange to isocyanates | | |

The covalent linkage L binds the reactive group Rₓ or conjugated substance S_{c} to the fluorophore, either directly (L is a single bond) or with a combination of stable chemical bonds, optionally including single, double, triple or aromatic carbon-carbon bonds, as well as carbon-nitrogen bonds, nitrogen-nitrogen bonds, carbon-oxygen bonds and carbon-sulfur bonds. L typically includes ether, thioether, carboxamide, sulfonamide, urea, urethane or hydrazine moieties. Preferred L moieties have 1-20 nonhydrogen atoms selected from the group consisting of C, N, O and S; and are composed of any combination of ether, thioether, amine, ester, carboxamide, sulfonamide, hydrazide bonds and aromatic or heteroaromatic bonds. Preferably L is a combination of single carbon-carbon bonds and carboxamide or thioether bonds. The longest linear segment of the linkage L preferably contains 4-10 nonhydrogen atoms including one or two heteroatoms. Examples of L include substituted or unsubstituted polymethylene, arylene, alkylarylene or arylenealkyl. In one embodiment, L contains 1-6 carbon atoms; in another, L is a thioether linkage. In yet another embodiment, L has the formula -(CH₂)ₐ(CONH(CH₂)_{b})_{z}-, where a has any value from 0-5, b has any value from 1-5 and z is 0 or 1.

The group Rₓ is bound directly to the fluorophore at any of R²-R⁵ or R⁷-R¹⁶, preferably at one of R¹³-R¹⁶, more preferably at R¹⁴ or R¹⁵, or is present as a substituent on an alkyl, alkoxy, alkylthio or alkylamino substituent. In one embodiment, exactly one of R², R³, R⁴, R⁵, R⁷, R⁹, R¹⁰, R¹¹, R¹², R¹³, R¹⁴, R¹⁵ or R¹⁶ is a -L-S_{c} moiety. In another embodiment, exactly one of R¹³, R¹⁴, R¹⁵, or R¹⁶ is an -L-S_{c} moiety. Where exactly one of R¹³, R¹⁴, R¹⁵, or R¹⁶ is a -L-S_{c} moiety, typically either each of the remaining of R¹³, R¹⁴, R¹⁵, or R¹⁶ is fluorine, or each of the remaining of R¹³, R¹⁴, R¹⁵, or R¹⁶ is hydrogen. In one embodiment, exactly one of R¹³, R¹⁴, R¹⁵ and R¹⁶ is a -L-Rₓ moiety. In another embodiment, exactly one of R², R³, R⁴, R⁵, R⁷, R⁸, R⁹ or R¹⁰ is a -L-Rₓ moiety. In another embodiment, where R¹² is a carboxylic acid or sulfonic acid, there is an additional -L-Rₓ moiety on the fluorinated dye.

The selection of covalent linkage to attach the fluorophore to the conjugated substance typically depends on the functional group on the substance to be conjugated. The types of functional groups typically present on the organic or inorganic substances include, but are not limited to, amines, thiols, alcohols, phenols, aldehydes, ketones, phosphates, imidazoles, hydrazines, hydroxylamines, disubstituted amines, halides, epoxides, sulfonate esters, purines, pyrimidines, carboxylic acids, or a combination of these groups. A single type of reactive site may be available on the substance (typical for polysaccharides), or a variety of sites may occur (e.g. amines, thiols, alcohols, phenols), as is typical for proteins. A conjugated substance may be conjugated to more than one fluorophore, which may be the same or different, or to a substance that is additionally modified by a hapten. Although some selectivity can be obtained by careful control of the reaction conditions, selectivity of labeling is best obtained by selection of an appropriate reactive dye.

Typically, Rₓ will react with an amine, a thiol or an alcohol. In one embodiment, Rₓ is an acrylamide, an activated ester of a carboxylic acid, an acyl azide, an acyl nitrile, an aldehyde, an alkyl halide, an amine, an anhydride, an aniline, an aryl halide, an azide, an aziridine, a boronate, a carboxylic acid, a diazoalkane, a haloacetamide, a halotriazine, a hydrazine, an imido ester, an isocyanate, an isothiocyanate, a maleimide, a phosphoramidite, a sulfonyl halide, or a thiol group. Preferably, Rₓ is a carboxylic acid, a succinimidyl ester, an amine, a haloacetamide, an alkyl halide, a sulfonyl halide, an isothiocyanate, a maleimide group or an azidoperfluorobenzamido group.

Substitution by a haloalkyl, haloacetamide, halomethylbenzamide allow the retention of fluorinated fluorophores in cells or organelles according to methods previously described (US Pats. 5,362,628 and 5,576,424 (in cells); and US Pat. 5,459,268 and UK 9420661.2 (in mitochondria)). Dyes fluorinated on the bottom ring yield adducts of glutathione (e.g. Compound 25) and protein that are retained in cells.

Useful dye-conjugates include conjugates of antigens, steroids, vitamins, drugs, haptens, metabolites, toxins, environmental pollutants, amino acids, peptides, proteins, nucleic acids, nucleic acid polymers, carbohydrates, lipids, ion-complexing moieties, and polymers. Alternatively, these are conjugates of cells, cellular systems, cellular fragments, or subcellular particles. Examples include virus particles, bacterial particles, virus components, biological cells (such as animal cells, plant cells, bacteria, or yeast), or cellular components. Fluorinated reactive dyes label reactive sites at the cell surface, in cell membranes, organelles, or cytoplasm; or derivatize low molecular weight compounds for analysis by capillary zone electrophoresis, HPLC or other separation techniques. Preferably the conjugated substance is an amino acid, peptide protein, polysaccharide, ion-complexing moiety, nucleotide, oligonucleotide, nucleic acid, hapten, drug, lipid, phospholipid, lipoprotein, lipopolysaccharide, liposome, lipophilic polymer, polymer, polymeric microparticle, biological cell or virus.

In one embodiment, the conjugated substance (S_{c}) is an amino acid (including those that are protected or are substituted by phosphates, carbohydrates, or C₁ to C₂₂ carboxylic acids), or is a polymer of amino acids such as a peptide or protein. Preferred conjugates of peptides contain at least five amino acids, more preferably 5 to 36 amino acids. Preferred peptides include, but are not limited to, neuropeptides, cytokines, toxins, protease substrates, and protein kinase substrates. Preferred protein conjugates include enzymes, antibodies, lectins, glycoproteins, histones, albumins, lipoproteins, avidin, streptavidin, protein A, protein G, phycobiliproteins and other fluorescent proteins, hormones, toxins and growth factors. Typically, the conjugated protein is an antibody, an antibody fragment, avidin, streptavidin, a toxin, a lectin, or a growth factor. Dye-peptide and protein conjugates include those labeled with a dye of the invention in combination with a second fluorescent or non-fluorescent dye to form an energy-transfer pair.

In another embodiment, the conjugated substance (S_{c}) is a nucleic acid base, nucleoside, nucleotide or a nucleic acid polymer, optionally containing an additional linker or spacer for attachment of a fluorophore or other ligand, such as an alkynyl linkage (US Pat. 5,047,519), an aminoallyl linkage (US Pat. 4,711,955) or other linkage. Preferably, the conjugated nucleotide is a nucleoside triphosphate or a deoxynucleoside triphosphate or a dideoxynucleoside triphosphate.

Preferred nucleic acid conjugates are labeled, single- or multi-stranded, natural or synthetic DNA or RNA oligonucleotides, or DNA/RNA hybrids, or incorporating an unusual linker such as morpholine derivatized phosphates (AntiVirals, Inc., Corvallis OR), or peptide nucleic acids such as *N*-(2-aminoethyl)glycine units, where the nucleic acid contains fewer than 50 nucleotides, more typically fewer than 25 nucleotides. Typically, the dye is attached via one or more purine or pyrimidine bases through an amide, ester, ether or thioether bond; or is attached to the phosphate or carbohydrate by a bond that is an ester, thioester, amide, ether or thioether. Alternatively, at least one dye of the invention is conjugated to an oligonucleotide that is simultaneously labeled with at least a second dye to form a fluorescence energy-transfer pair, or to a hapten such as biotin or digoxigenin, or to an enzyme such as alkaline phosphatase, or to a protein such as an antibody. Nucleotide conjugates of the invention are readily incorporated by DNA polymerase and can be used for *in situ* hybridization (see below) and nucleic acid sequencing (e.g., US Pats. 5,332,666; 5,171,534; and 4,997,928; and WO Appl. 94/05688).

In another embodiment, the conjugated substance (S_{c}) is a carbohydrate that is typically a polysaccharide, such as dextran, FICOL, heparin, glycogen, amylopectin, mannan, inulin, starch, agarose and cellulose. Preferred polysaccharide conjugates are dextran or FICOL conjugates.

In another embodiment, the conjugated substance (S_{c}) is a lipid (typically having 6-25 carbons), including glycolipids, phospholipids, and sphingolipids. Alternatively, the conjugated substance is a lipid vesicle, such as a liposome, or is a lipoprotein (see below). The lipophilic moiety may be used to retain the compounds in cells, as described in US Pat. 5,208,148.

Conjugates having an ion-complexing moiety serve as an indicator of calcium, sodium, magnesium, potassium, or other biologically important metal ion. Preferred ion-complexing moieties are crown ether, including diaryldiaza crown ethers (US Pat. 5,405,975); BAPTA (US Pat. 5,453,517, US Pat. 5,516,911, and US Pat. 5,049,673); derivatives APTRA (AM. J. PHYSIOL. **256**, C540 (1989)); and pyridine- and phenanthroline-based metal ion chelators; preferably a diaryldiaza crown ether or BAPTA chelator. The ion indicators are optionally conjugated to plastic or biological polymers such as dextrans to improve their utility as sensors. Alternatively, the dye itself acts as an indicator of H⁺ at pH values within 1.5 pH units of the individual dye's pKa. Those dye embodiments having fluorine at R² and R⁵ are most useful as pH indicators over the range of pH 4-6.

Finally, the conjugates are optionally dye-conjugates of polymers, polymeric particles, polymeric microparticles including magnetic and non-magnetic microspheres, polymeric membranes, conducting and non-conducting metals and non-metals, and glass and plastic surfaces and particles. Conjugates are optionally prepared by copolymerization of a fluorinated dye that contains an appropriate functionality while preparing the polymer, or by chemical modification of a polymer that contains functional groups with suitable chemical reactivity. Other types of reactions that are useful for preparing dye-conjugates of polymers include catalyzed polymerizations or copolymerizations of alkenes and reactions of dienes with dienophiles, transesterifications or transaminations. In another embodiment, the conjugated substance is a glass or silica, which may be formed into an optical fiber or other structure.

The preparation of dye conjugates using reactive dyes is well documented, e.g. by R. Haugland, MOLECULAR PROBES HANDBOOK OF FLUORESCENT PROBES AND RESEARCH CHEMICALS, Sets 1-7 (1992); and Brinkley, BIOCONJUGATE CHEM., 3, 2 (1992). Conjugates typically result from mixing appropriate fluorinated reactive dyes and the substance to be conjugated in a suitable solvent in which both are soluble. For those reactive dyes that are photoactivated, conjugation requires illumination of the reaction mixture to activate the reactive dye. The dye-biomolecule conjugate is used in solution or lyophilized.

Labeled members of a specific binding pair are used as fluorescent probes for the complementary member of that specific binding pair, each specific binding pair member having an area on the surface or in a cavity which specifically binds to and is complementary with a particular spatial and polar organization of the other. Representative specific binding pairs are shown in Table 3. Such probes optionally contain a BLOCK that is removed by an enzyme or light, or R¹¹ is H and the compound fluoresces on oxidation.

**Table 3.**

| Representative Specific Binding Pairs | |
|---|---|
| antigen | antibody |
| biotin | avidin (or streptavidin or anti-biotin) |
| IgG* | protein A or protein G |
| drug | drug receptor |
| toxin | toxin receptor |
| carbohydrate | lectin or carbohydrate receptor |
| peptide | peptide receptor |
| protein | protein receptor |
| enzyme substrate | enzyme |
| DNA (RNA) | aDNA (aRNA)† |
| hormone | hormone receptor |
| ion | chelator |

| | |
|---|---|
| * IgG is an immunoglobulin | |
| ^{†} aDNA and aRNA are the antisense (complementary) strands used for hybridization | |

### BLOCKED Dyes and Other Substrates

In embodiments where R⁷, R⁸ or R⁹ is a BLOCK moiety (which may be the same or different) that substantially alters the fluorescence of the fluorophore, removal of BLOCK restores the fluorescence of the parent dye. Typically BLOCK is a monovalent moiety derived by removal of a hydroxy group from phosphate or sulfate, or a biologically compatible salt thereof; or from a carboxy group of an aliphatic or aromatic carboxylic acid or of an amino acid, protected amino acid, peptide, or protected peptide; or from an alcohol or a mono- or polysaccharide. Typically, the BLOCK-fluorophore bond at R⁷ is an ether or ester bond, and at R⁸ or R⁹ is an amide bond. Alternatively, BLOCK is a photolabile caging group on a dye or on a dye conjugate. At R⁷, the cage is typically a substituted or unsubstituted derivative of *o*-nitroarylmethine (including α-carboxy o-nitroarylmethine and bis-(5-t-butoxycarbonylmethoxy)-2-nitrobenzyl (Compound **101**)), of 2-methoxy-5-nitrophenyl, or of desyl.

Where cleavage of -BLOCK yields a detectable response indicative of the presence of enzyme activity, the compound is an enzyme substrate. Enzymes that are known to cleave these conjugated moieties include microsomal dealkylases (for example, cytochrome P450 enzymes), glycosidases (for example β-galactosidase, β-glucosidase, α-fucosidase, β-glucosaminidase), phosphatases, sulfatases, esterases, lipases, guanidinobenzoatases and others. Conjugates of rhodol dyes that are amino acid or peptide amides are typically useful as peptidase substrates. Fluorinated fluorescein diacetate compounds readily enter intact cells where the acetate moieties are cleaved by intracellular esterases in viable cells, restoring intrinsic fluorescence and indicating viability. Similarly, fluorinated dyes that are substituted by acetoxymethyl esters (such as fluorinated analogs of calcein-AM, e.g. Compound **99**) readily enter intact cells and can serve as cell tracers or viability indicators, particularly where R¹¹ is H.

Any of the substituents described above for retaining the dyes in cells can be used to retain the substrates in cells. Preferred enzyme substrates are fluorinated on the bottom ring. Particularly preferred substrates have two identical BLOCK moieties that are acetate or glycoside, such as β-D-galactopyranoside, that are at least tetrafluorinated on the bottom ring.

Dye compounds having a pKa <6 in an aqueous solution, more preferably less than about 5, are preferred substrates in an acidic environment (e.g. an acidic β-galactosidase such as β-D-galactopyranoside, or in acidic organelles, such as substrates for lysosomal glycosidases (see Table 14). Substrates where -BLOCK is cleavable by a phosphatase enzyme are useful to detect both acid and alkaline phosphatase. Preferred substrates for detection of enzymes with maximal turnover rates below pH 7 are fluorinated at the 2' and 7' positions.

In another substrate embodiment, dyes where R¹¹ is H are substrates for oxidative enzymes and other oxidizing agents, e.g. peroxidase enzymes. Unlike their non-fluorinated analogs, fluorinated dihydrofluorescein and dihydrorhodol are stable in aqueous solutions without the use of blocking groups that typically reduce solubility. For example, Compound **84** (4,5,6,7-tetrafluorodihydrofluorescein) is soluble in water to at least 2 mM.

### Methods of Use

The new dyes are generally utilized to stain a sample to give a detectable optical response under desired conditions by combining the sample of interest with a solution of dye (prepared according to methods generally known in the art) for a period of time sufficient for the dye compound to yield a detectable optical response under the desired conditions. The required concentration for the dye solution (typically nanomolar to micromolar) is determined by systematic variation in dye or dye-conjugate concentration until satisfactory dye staining is accomplished.

The dye compounds are most advantageously used to stain samples with biological components. The sample may comprise heterogeneous mixtures of components (including intact cells, cell extracts, bacteria, viruses, organelles, and mixtures thereof), or a single component or homogeneous group of components (e.g. natural or synthetic amino acid, nucleic acid or carbohydrate polymers, or lipid membrane complexes). These dyes are generally non-toxic to living cells and other biological components, within the concentrations of use, although those fluorinated dyes that are additionally substituted one or more times by Br or I are efficient photosensitizers.

The sample is optionally combined with other solutions in the course of staining, including wash solutions, permeabilization and/or fixation solutions, and other solutions containing additional detection reagents, according to known methods. With selected embodiments that are well retained in cells, the cells retain considerable fluorescent staining after fixation. Where the additional detection reagent has spectral properties that differ from those of the subject dye compounds, multi-color applications are possible.

After or during staining, the sample is illuminated to give a detectable optical response, and observed with a means for detecting the optical response, using a fluorometer, microscope, microplate reader, flow cytometer, laser scanner, hand lamp or other equipment. Typically the optical response is a change in fluorescence, such as a change in the intensity or excitation or emission wavelength distribution of fluorescence, fluorescence lifetime, fluorescence polarization, or a combination thereof.

Staining is used to determine the presence, quantity, or the spatial or temporal distribution of components or a mechanism in a sample, according to known methods. The dyes of the invention are well-suited to the preparation of a kit comprising a reactive fluorinated dye and instructions for conjugating the dye to any substance possessing an appropriate functional group, and optionally for recovering or purifying the materials labeled thereby, including, but not limited to, biological polymers (e.g. proteins, oligonucleotides or carbohydrates), polymeric resins and plastics (e.g. polystyrene), metals, glasses, and other organic or inorganic substances. Fluorinated xanthene dyes are also useful haptens because fluorination does not interfere with the recognition of the fluorophore by the anti-fluorescein antibody, typically resulting in fluorescence quenching of the dye (see Table 15).

### Dye Synthesis

The first step in preparing a fluorinated fluorescein or rhodol dye is the preparation of an appropriately substituted resorcinol or aminophenol, respectively. While methods for preparing fluorinated resorcinols are known (Patrick et al. J. ORG. CHEM. **51**, 3242 (1986); Lerman et al. J. ORG. CHEM. **49**, 806 (1984)), fluorinated resorcinols and aminophenols are more conveniently synthesized from a (commercially available) polyfluoronitrobenzene or alkoxy-substituted derivative. The fluorine atoms *ortho* and *para* to the nitro are displaced with two equivalents of alkoxide or benzyloxide. The nitro group is then reduced, followed by diazotization and reductive dediazonization (see Table 4). Alternatively, the diazonium cations are isolated as pure salts, followed by reduction with sodium borohydride. Dealkylation with BBr₃ or another ether-cleaving reagent, or in the case of benzyl ethers catalytic hydrogenolysis, affords pure fluorinated resorcinols. Other substituents are optionally present during the synthesis provided they survive the synthetic method. For example, alkyl, carboxyalkyl, chloro, bromo, iodo, alkoxy and hydroxy moieties.

Fluorinated aminophenols are prepared similarly: Alkyl-substituted amine nucleophiles are first substituted onto a polyfluoronitrobenzene, in sequence with alkoxide or benzyloxide. Reduction followed by diazotization and hydrodediazoniation results in a fluorinated aminophenol, which is then used to prepare a fluorinated rhodol. Alternatively, commercially available fluorinated nitrophenyl ketones and aldehydes are converted to the corresponding phenolic esters using Baeyer-Villiger oxidation chemistry. Reductive amination or direct alkylation yields the desired N-substituted aminophenol. Deacylation of the phenolic hydroxyl gives the desired fluorinated aminophenol (see Table 5).

Xanthylium dyes having fluorine substituents on the xanthene portion of the dye are typically prepared by condensation of a fluorinated resorcinol with a phthalic acid, phthalic anhydride, sulfobenzoic acid or sulfobenzoic anhydride (such as phthalic anhydride, trimellitic anhydride, nitrophthalic anhydride, o-sulfobenzoic anhydride, sulfoterephthalic acid), or with benzaldehydes (when followed by oxidation) or with aliphatic dicarboxylic acids or anhydrides such as a succinic or glutaric anhydride. The condensation is optionally catalyzed (such as by ZnCl₂ or methanesulfonic acid), and yields the desired fluorinated xanthylium dye after aqueous workup.

Fluorinated xanthylium dyes are also prepared by condensing two equivalents of a resorcinol with a fluorinated benzenecarbonyl compound, such as tetrafluorophthalic anhydride, tetrafluorophthalic acid or fluorophthalic acid derivatives, pentafluorobenzoic acid, and fluorinated benzaldehydes such as pentafluorobenzaldehyde, although an oxidation step is required after using fluorinated benzaldehydes to give the fluorescent xanthylium dye. The resulting dyes are fluorinated on the aryl ring bound to the xanthene ring system. Where the aryl ring is fluorinated at the 4- and 6-positions, nucleophiles such as reduced glutathione, mercaptoacetic acid, mercaptoethylamine and azide can displace fluoride ion at the 4- or 6-positions, providing a synthetic route for subsequent conjugation chemistry (see Table 8). For example, a mercaptoacetic acid adduct is converted into a succinimidyl ester, or a mercaptoethyl amino is converted into isothiocyanate, maleimide or haloacetamide. Alternatively the azide group is reduced to an amine, which is then acylated with iodoacetic anhydride to provide an iodoacetamide.

Fluorinated xanthylium dyes are also obtained beginning with polyfluorinated benzonitriles. Addition of an alkoxide or a benzyloxide to the benzonitrile displaces the fluorine atoms *ortho-* and *para-* to the nitrile group. Addition of an arylorganometallic reagent, such as pentafluorophenylmagnesium chloride, converts the nitrile group to an imine, which is subsequently hydrolyzed, affording a substituted benzophenone with alkoxy groups at the 2- and 4-positions. The alkoxy groups are dealkylated, for example with hydrobromic acid and acetic acid. Treatment with NaOH in methanol then gives a xanthenone, in which the 2-hydroxy group has displaced the 2'-fluorine atom; concomitantly, an alkoxy group displaces the 4'-fluorine atom. The 3-hydroxy group in the resulting xanthone is typically protected by a base-stable protecting group, such as a 2-methoxyethylmethyl ether (MEM ether), and then an arylorganometallic reagent such as pentafluorophenyl magnesium chloride or an alkyl lithium reagent is added to the xanthone carbonyl group. Subsequent treatment with hydrobromic and acetic acid dehydrates the resulting tertiary carbinol to the desired xanthylium dye, with concomitant conversion of the 6-methoxy group to a hydroxyl group.

For asymmetric xanthylium dyes such as rhodols and unsymmetrical xanthylium dyes such as rhodols and unsymmetrical fluoresceins, condensation can be performed using one equivalent each of the appropriate substituted or unsubstituted resorcinol with one equivalent of a different resorcinol (as in Khanna et al., U.S. Patent No. 4,439,359 (1984)), aminophenol and with one equivalent of the appropriate phthalic acid derivative or benzaldehyde. The synthesis may be performed in a concerted fashion or stepwise, as described in U.S. Patent No. 5,227,487 to Haugland et al. (1993) (see Table 7).

Modifications of fluorinated xanthylium dyes are performed according to known methods for modifying fluoresceins. For example, the fluorinated fluorescein can be halogenated with an appropriate halogenating agent, such as liquid bromine (e.g. Compound **31**). Where the 4' and/or 5' positions of a fluorinated fluorescein are occupied by hydrogen atoms, those positions can be substituted with aminomethyl groups using Mannich conditions (as in Kirkemo et al., U.S. Patent No. 4,510,251, supra), in particular where the substituted amine is iminodiacetic acid or an amino acid. Where a bottom-ring carboxylate is present, it can be converted into a chloromethyl or bromomethyl substituent by reduction followed by treatment with HCl or HBr. Where two isomeric carboxylates are present, they are optionally separated (General Method J) or used as a mixture of isomers. Reduced xanthylium dyes of Formula 2 are prepared by reduction of the xanthenone with zinc dust or borohydride in organic solvents (see Table 11). These dihydrofluorinated dyes serve as substrates for enzymes that take up electrons, or in the detection of oxidizing agents, reactive oxygen species or nitric oxides. Preparation of other enzyme substrates includes acylation of phenolic hydroxyls with phosphate to give phosphatase substrates, with carboxylic acids to give esterase substrates, alkylation to give dealkylase substrates, and with carbohydrates to yield glycosidase substrates. In addition, rhodols having at least one hydrogen atom at the rhodol nitrogen are optionally acylated by methods well known in the art to yield amido derivatives or treated with sulfonyl halides to yield sulfonamides.

The dihydroxanthene and xanthylium versions of the dyes of the invention are freely interconvertible by well-known oxidation or reduction reagents, including borohydrides, aluminum hydrides, hydrogen/catalyst, and dithionites. A variety of oxidizing agents mediate the oxidation of dihydroxanthenes, including molecular oxygen in the presence or absence of a catalyst, nitric oxide, peroxynitrite, dichromate, triphenylcarbenium and chloranil. The xanthenes are also oxidized by enzyme action, including horseradish peroxidase in combination with peroxides or by nitric oxide.

The examples below are given so as to illustrate the practice of this invention. They are not intended to limit or define the entire scope of this invention.

### Examples

### Preparation of Fluorinated Resorcinols:

### General Method A

Sodium methoxide (1.0 M) is prepared by adding sodium metal portionwise to anhydrous methanol (Aldrich) under nitrogen at 0 °C. To a neat fluorinated nitrobenzene (1.0 equiv.) under nitrogen at room temperature is added sodium methoxide solution (2.2 equiv.) over 5-10 minutes. The reaction mixture is stirred at room temperature. Once TLC analysis shows the reaction is complete (1-24 hours), several drops of 1 M citric acid are added. Water is added, followed by extraction with ether. The organic layer is washed with brine, dried over Na₂SO₄, evaporated and recrystallized from hexanes/CH₂Cl₂ to give the desired dimethoxyfluoronitrobenzene.

### General Method B

The nitro group of the fluoronitrobenzene is reduced by catalytic hydrogenation at 40 psi in ethanol/ethyl acetate over 10% Pd/C. When TLC analysis shows the reaction is complete, the catalyst is removed by filtration and the filtrate is evaporated to give the pure amine-substituted fluorinated benzene.

### General Method C

The nitro group of the fluoronitrobenzene is reduced by homogeneous reduction in refluxing ethyl acetate/ethanol (2:1, 0.10 M) using stannous chloride dihydrate (5 equiv.). Reaction progress is monitored by TLC. The reaction is cooled, poured into water, and neutralized to pH 7 with 1 M NaOH. The mixture is extracted with ethyl acetate, washed with brine, dried over Na₂SO₄, and evaporated. The residue is purified by flash chromatography.

### General Method D

The desired aminofluorobenzene in water/HCl (2:1, 0.3 M) is chilled in ice and treated with a cold solution of NaNO₂ (1.05 equiv.) in water. The diazonium salt solution is stirred 15 minutes, then hypophosphorous acid (50% aqueous solution, 20 equiv.) is added over 5 minutes. The mixture is stirred at room temperature for two hours, then diluted with water. The mixture is neutralized with aqueous Na₂CO₃ or NaOH, and extracted twice with ether. The organic extract is washed with water, then brine, and dried over Na₂SO₄. The solution is evaporated and the residue is purified by flash chromatography.

### General Method E

A solution of the fluorinated dimethoxybenzene in anhydrous CH₂Cl₂ (0.3 M) at room temperature under nitrogen is treated with BBr₃ (3.0 equiv., 1.0 M in CH₂Cl₂) via syringe over five minutes. TLC shows that the reaction takes 24-48 hours to reach completion; an additional 0.5 equiv. of BBr₃ solution is sometimes necessary to reach completion. The solution is carefully quenched with water, and stirred to dissolve any precipitate. The solution is extracted with ether, the extracts are washed with brine, dried over Na₂SO₄ and evaporated and purified by sublimation to yield the desired fluorinated resorcinol.

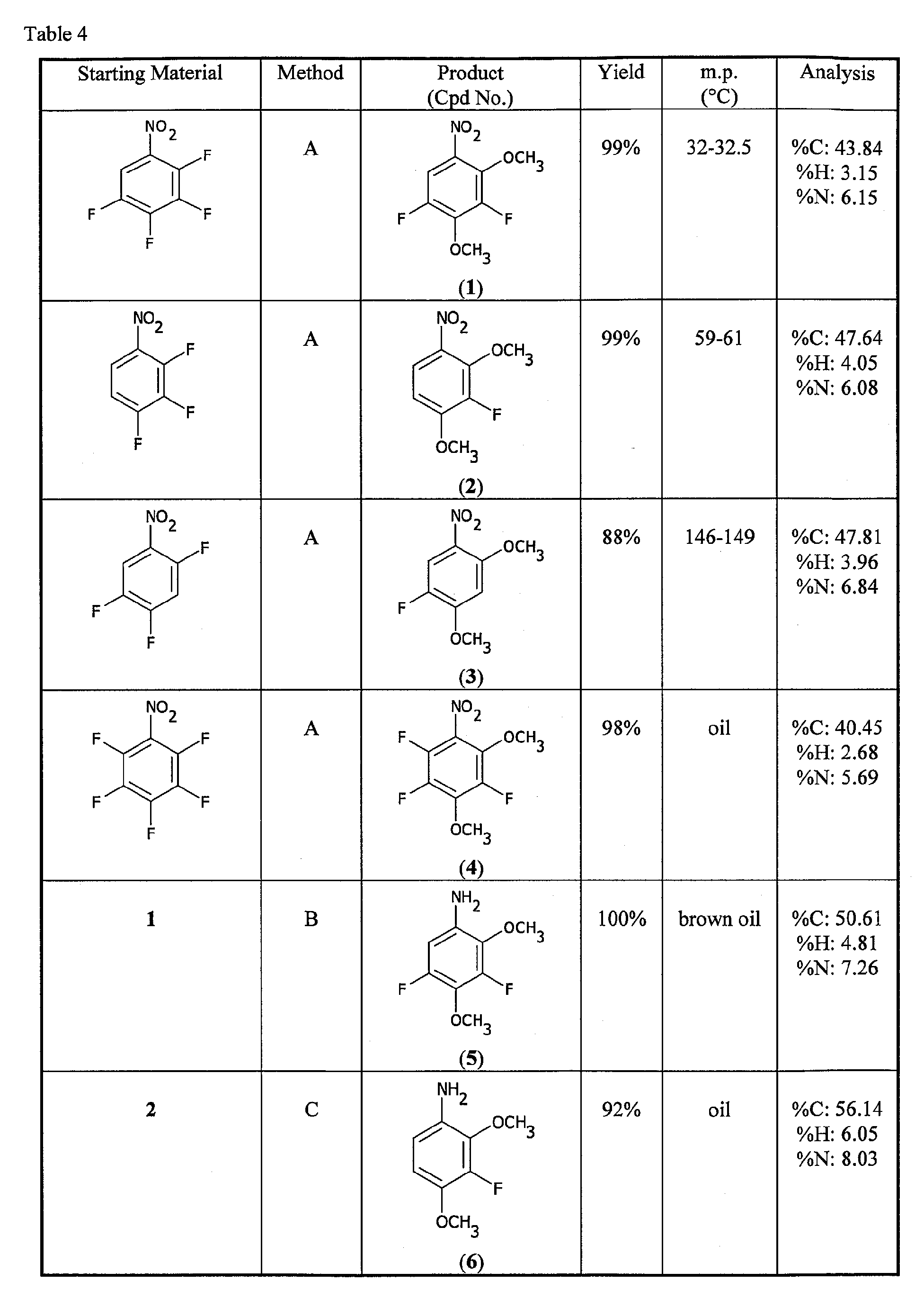

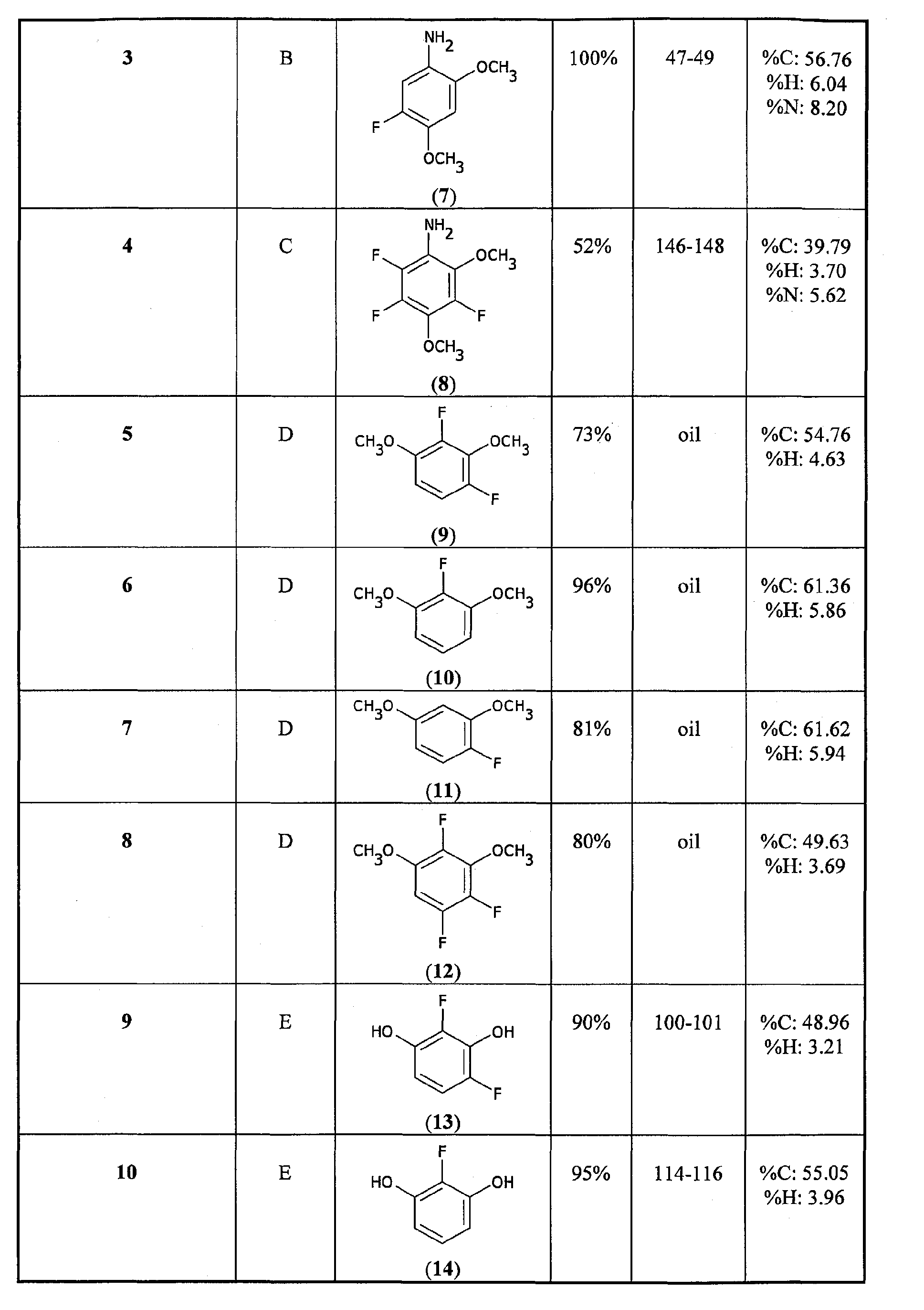

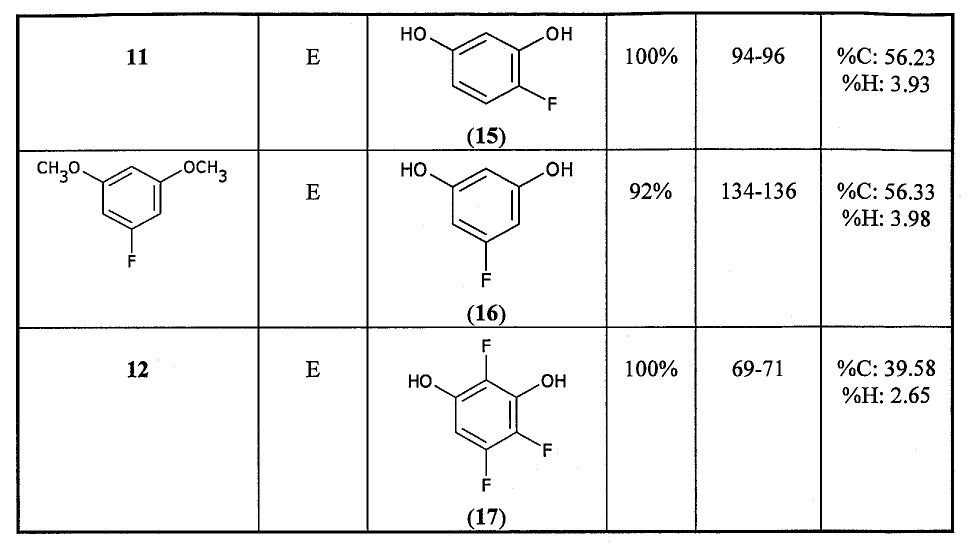

### Preparation of Fluorinated Aminophenols

### General Method F

An ethanol solution of a secondary amine (3 equiv.) and a fluorinated nitrobenzene is either heated in a sealed tube or heated at reflux (1-24 hrs). The solution is evaporated and the resulting solid is recrystallized or chromatographed to give the pure fluoronitroaniline. The fluorinated aminophenol is then prepared as in General Method A for fluorinated resorcinols.

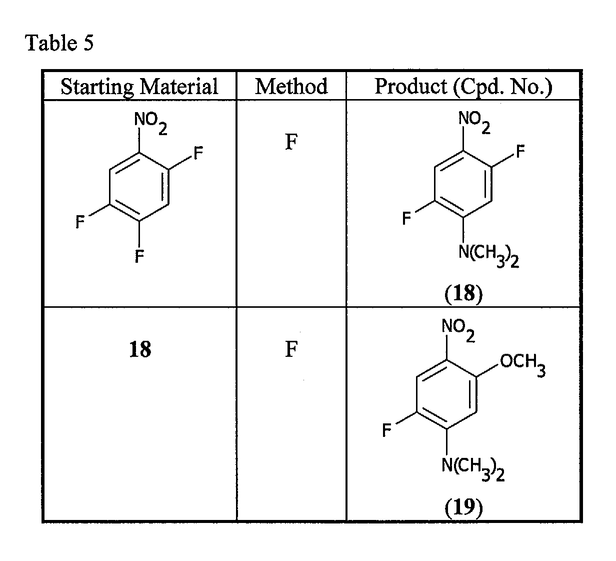

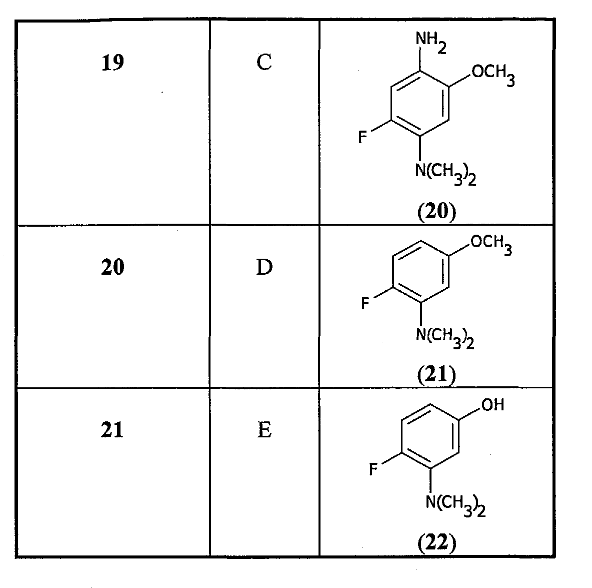

### General Method G

Fluorinated aminophenols are generated from commercially available fluorinated phenyl ketones and aldehydes by nitrating the phenyl ketone or aldehyde and converting it into the corresponding nitrophenol using Baeyer-Villiger oxidation chemistry. Reduction followed by reductive amination yields the N-substituted aminophenol. Amines may also be treated with other alkylating agents known in the art, such as alkyl halides.

### Preparation of 4-fluoro-3-nitrophenol (23):

To 15 mL of conc. H₂SO₄ at 0 °C is added 6.9 g (0.05 mmol) of 4-fluoroacetophenone. To the solution is added a mixture of 4 mL HNO₃ and 6 mL conc. H₂SO₄. The reaction is stirred at 0-5 °C for 3 hrs. The reaction is poured into ice water, and extracted with CHCl₃. The organic layer is washed with water, dried over Na₂SO₄, filtered and evaporated. The residue is purified by column chromatography, yielding 6.0 g of 4-fluoro-3-nitroacetophenone (60% yield).

Conc. H₂SO₄ (200 mL) and acetic acid (120 mL) are mixed at 0 °C. To the solution is added 15 g (0.082 mol) of 4-fluoro-3-nitroacetophenone followed by 36 mL of 36% peracetic acid. The reaction is stirred at room temperature for 4 hours. Water (500 mL) is added, and the product is extracted into diethyl ether. The ether fraction is washed with water, dried over Na₂SO₄, filtered and evaporated. The residue is purified by column chromatography, giving 3.2 g of Compound 23 (20% yield). mp: 86-87 °C. Anal. Found: C, 45.80; H, 2.51; N, 8.69.

### Preparation of 3-amino-4-fluorophenol (24):

A mixture of 4-fluoro-3-nitrophenol (2.70 g, 17.2 mmol) and Pd/C (10 %, 0.31 g) in 60 mL ethyl acetate is hydrogenated under 50 psi of H₂. After 1 hour, the catalyst is removed by filtration. The filtrate is evaporated and the residue sublimed to yield 2.18 g (100 %) of Compound **24** as a white solid. mp: 142-144 °C. Anal. Found: C, 57.28; H, 4.86; N, 10.65.

### Preparation of Fluorinated Fluoresceins

### General Method H

A fluorinated resorcinol (2 equiv.), a phthalic acid or anhydride (1 equiv.) and anhydrous ZnCl₂ (2.5 equiv.) are fused at ca. 170-180 °C for 30 minutes with stirring. The cooled mixture is suspended in water and suction filtered. The solid, containing the fluorinated fluorescein, is further purified by dissolving it in methanol/water, followed by filtration through diatomaceous earth and evaporation.

### General Method H'

Alternatively, the crude fluorinated fluorescein of Method H is converted to a diacetate in acetic anhydride and pyridine, heating briefly, and the mixture is subjected to aqueous workup and chromatographic purification or recrystallization of the organic-soluble product(s).

### General Method I

Alternatively, a desired fluorinated resorcinol (2 equiv.) and a phthalic anhydride (1 equiv.) are heated at 80-85 °C for 48 hrs. in methanesulfonic acid, as a 10 % w/v solution. The cooled solution is poured into 7 volumes of an ice/water mixture. The precipitate is collected, washed with cold water, dried *in vacuo* at 60 °C, and purified as described in Method H.

### General Method I'

Bromine (6 equiv.) is added dropwise to a solution of a fluorinated dye (1 equiv.) in MeOH. The mixture is stirred for 3 hours then evaporated. The residue is stirred with acetic anhydride and pyridine for 1 hour, diluted with ethyl acetate, washed with 1 M citric acid, then brine, and evaporated. The residue is purified by column chromatography to yield the brominated dye.

### General Method J

Isolation of carboxyfluorescein isomers: The fluorinated carboxyfluorescein (1 equiv.), pyridine (4 equiv.), and acetic anhydride (100 equiv.) are heated for 5 minutes at 80 °C. The reaction is cooled to -4 °C for 24 hours. The crystalline precipitate (the pyridinium salt of the 6-isomer diacetate) is collected, washed with acetic anhydride, ether, and dried to yield an off-white powder. The filtrate is stirred with an equal volume of water for 30 min and extracted with ethyl acetate. The combined extract is washed with brine, dried, evaporated, and recrystallized from CH₂Cl₂ to yield the 5-isomer diacetate as the free acid. The filtrate is concentrated, redissolved in toluene, 1.5 equiv. of pyridine is added, and the solution is allowed to stand at 20 °C for 15 hours. The resulting precipitate is collected, washed with toluene, ether, and dried to yield additional 6-isomer diacetate as the pyridinium salt. The filtrate is diluted with ethyl acetate, washed twice with 1 M citric acid, brine, dried, concentrated and recrystallized from CH₂Cl₂ to yield a second crop of the 5-isomer diacetate as the free acid.

### General Method K

Hydrolysis of a fluorinated fluorescein diacetate: A 0.1 M solution of the diacetate in THF/MeOH/water (4:4:2) is treated with NH₄OH (10 equiv.) at 20 °C. After 2 hours the reaction is poured into 7 volume of ice water and acidified with HCl to pH 2. The precipitate is collected, washed with cold water, and dried at 60 °C to yield the product.

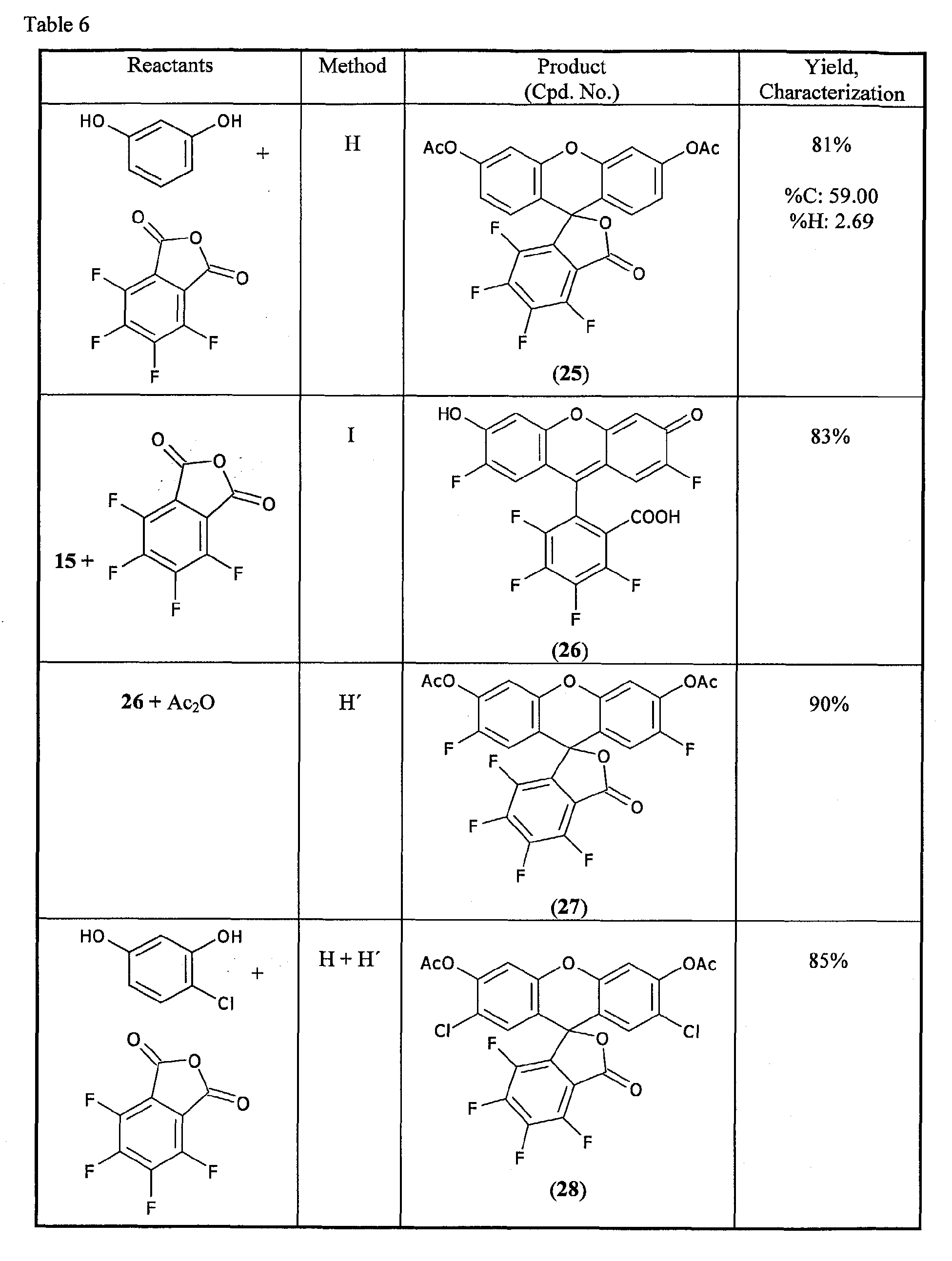

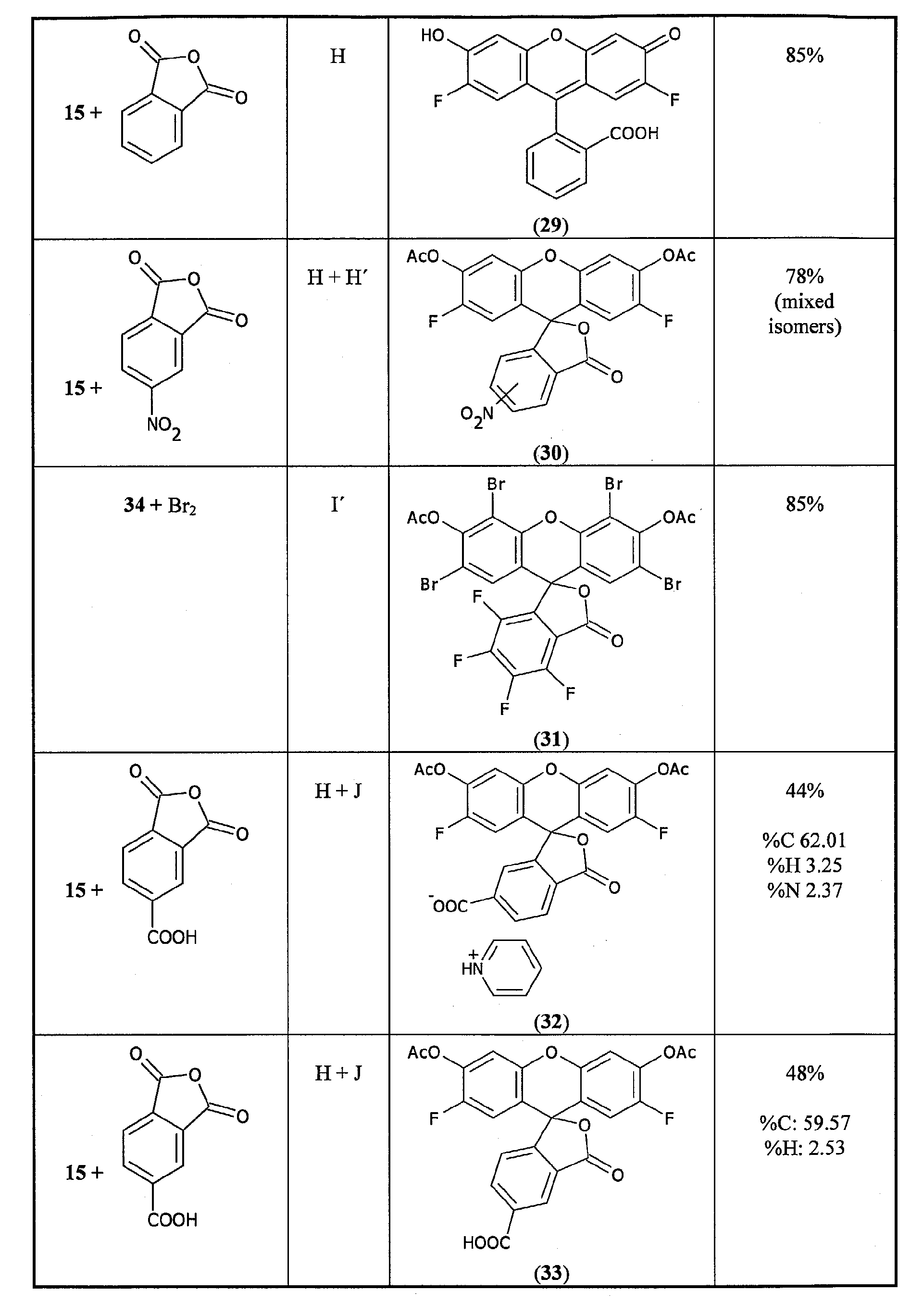

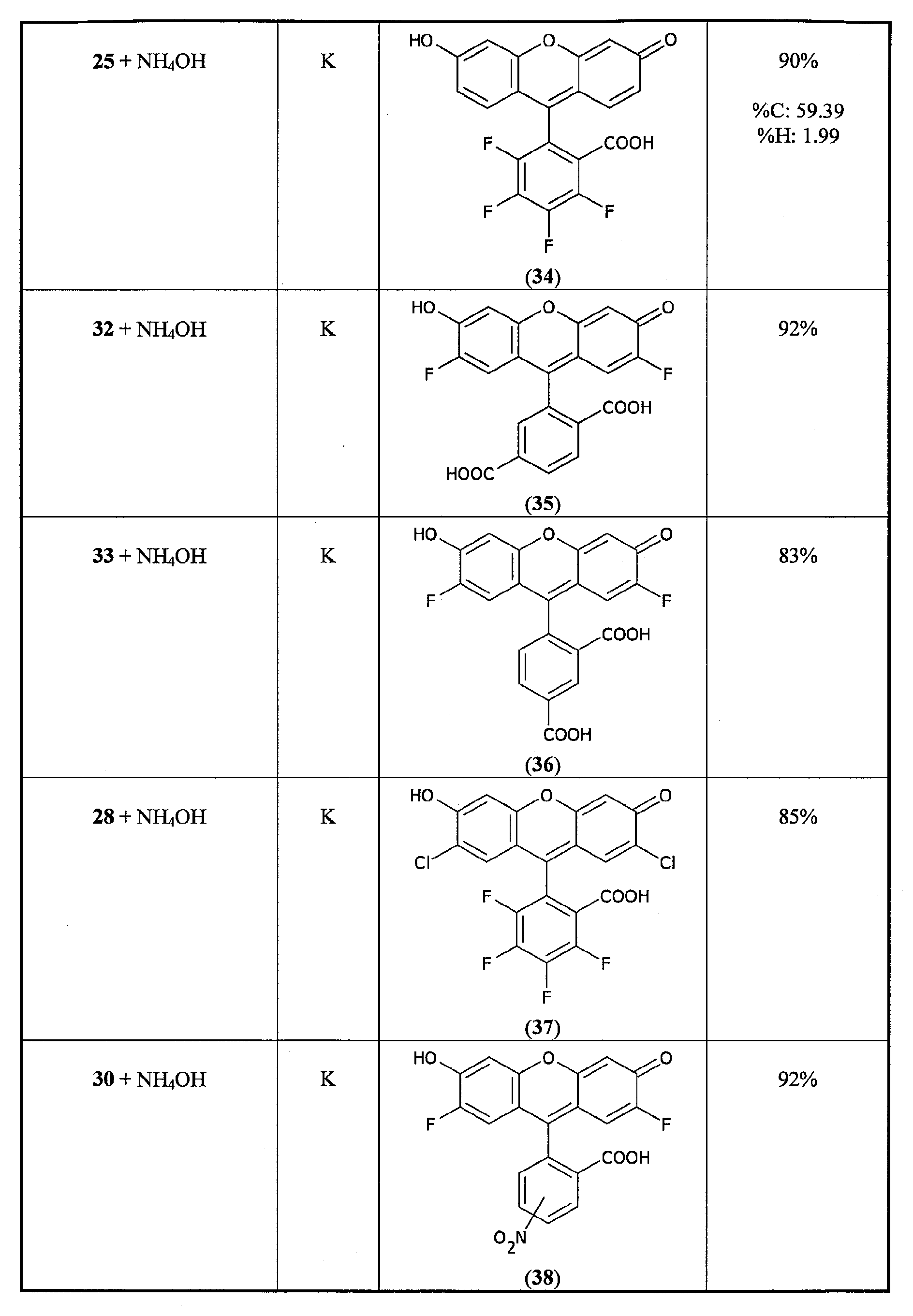

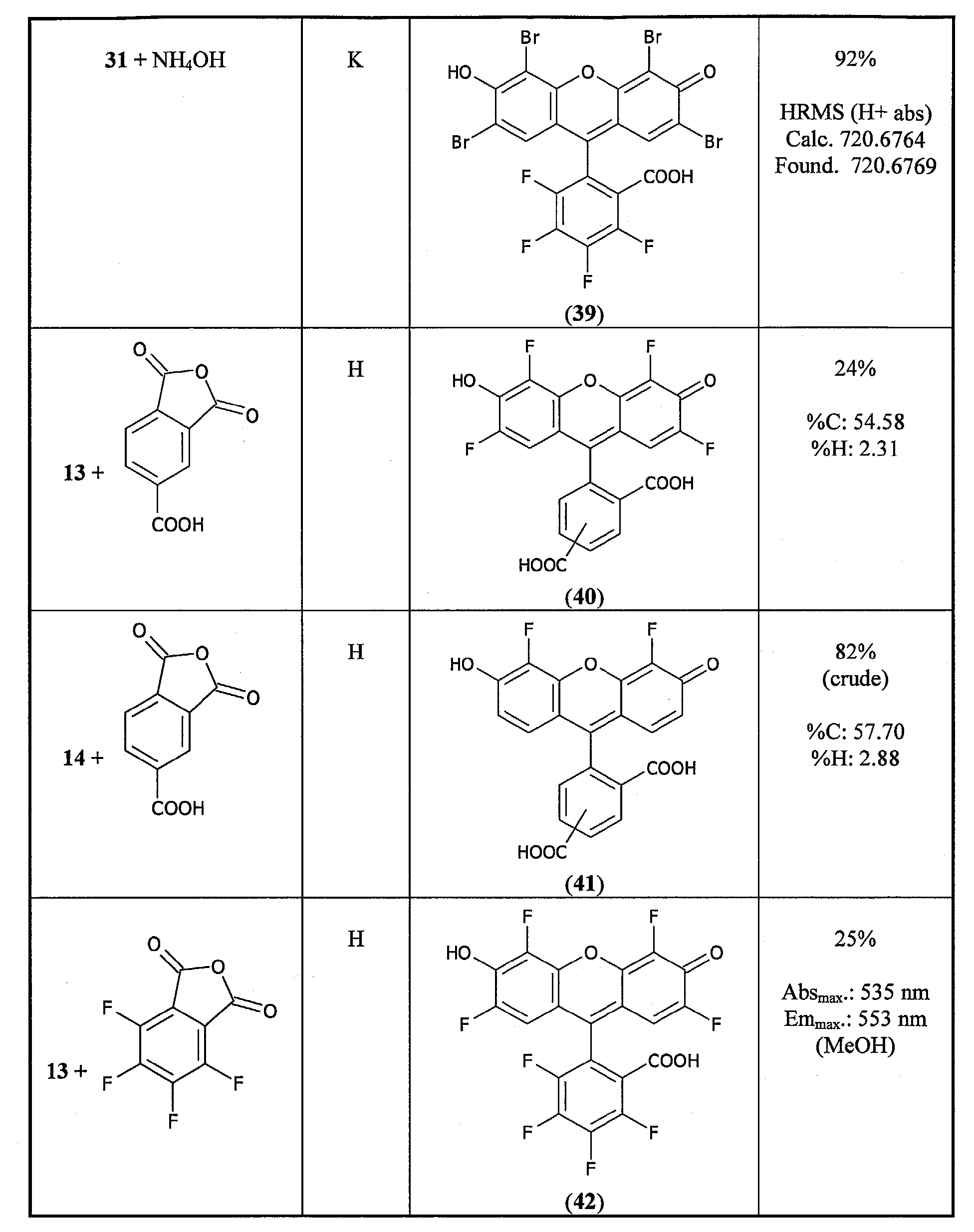

### Preparation of 1',8'-Substituted Fluorinated Fluoresceins:

1',8'-Substituted fluorinated fluoresceins are prepared from polyfluorinated benzonitriles as described in the specification. The reaction scheme below exemplifies the preparation of 1,2,4,5,7,8-hexafluoro-6-hydroxy-9-pentafluorophenylxanthen-3-one. Selection of appropriately substituted benzonitrile and arylorganometallic groups will result in the desired fluorinated dye.

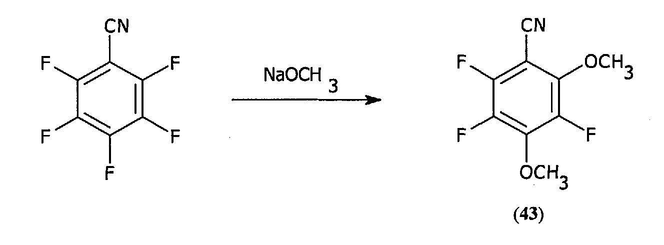

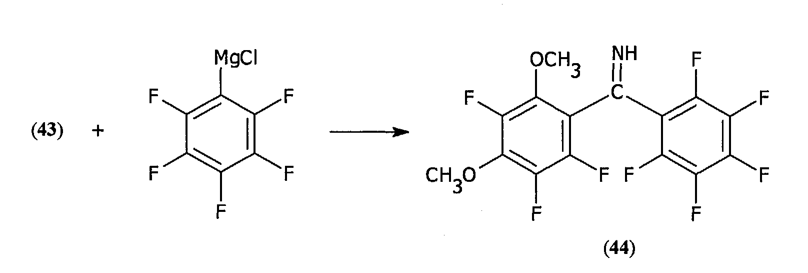

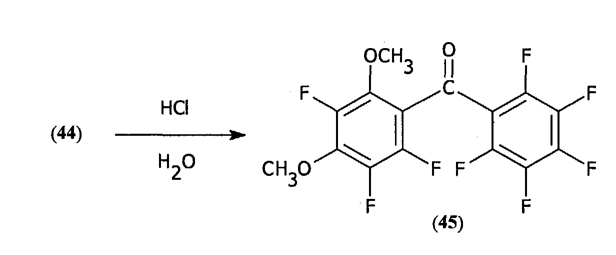

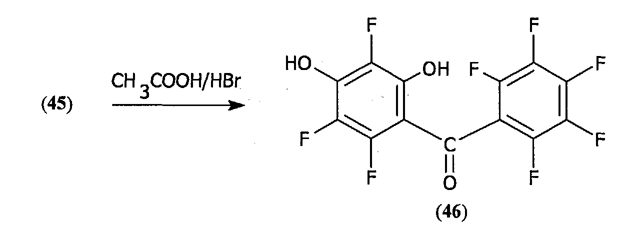

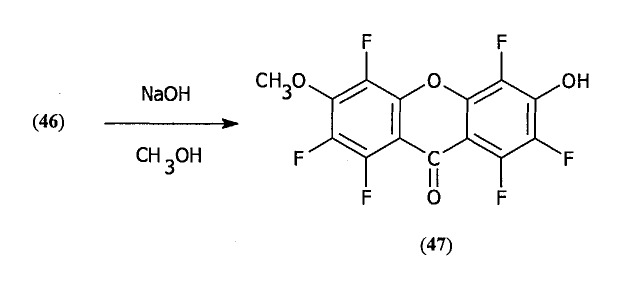

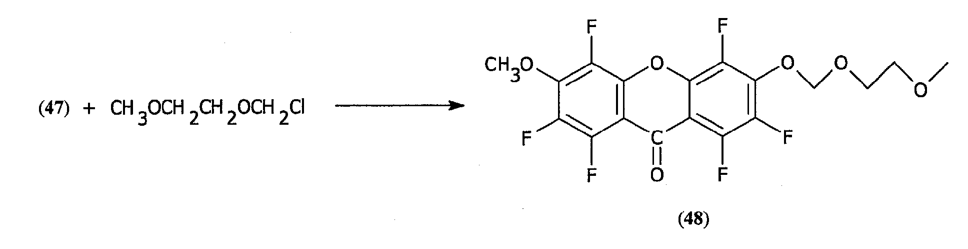

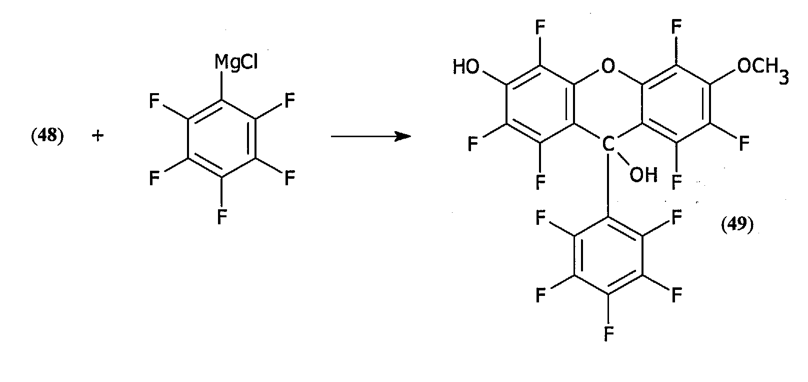

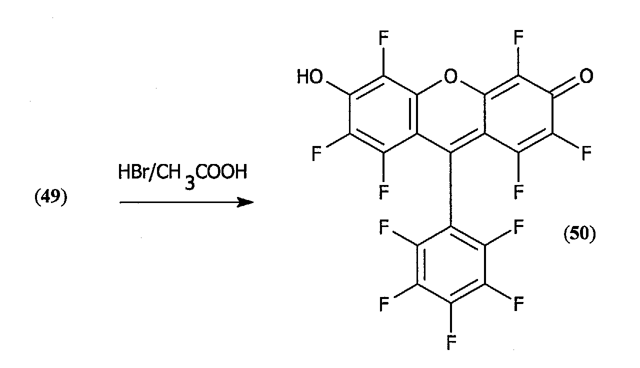

### Preparation of Fluorinated Rhodols:

### General Method L

Equimolar mixtures of the two appropriate electron rich aromatic components (i.e. a resorcinol, and a 3-aminophenol) are condensed with one equivalent of a phthalic acid, phthalic anhydride or sulfoterephthalic acid, either as a melt at 150-200 °C in the presence of one equivalent of anhydrous zinc chloride, with subsequent work-up consisting of mixing with water, followed by filtration, or as a solution in warm (80 °C) methanesulfonic acid, with subsequent work-up consisting of pouring the reaction solution into an excess of water, followed by filtration. In both methods the desired unsymmetrical fluorescein and rhodol dye products are separated from the undesired symmetrical dye products by either flash chromatography or preparative thin-layer chromatography.

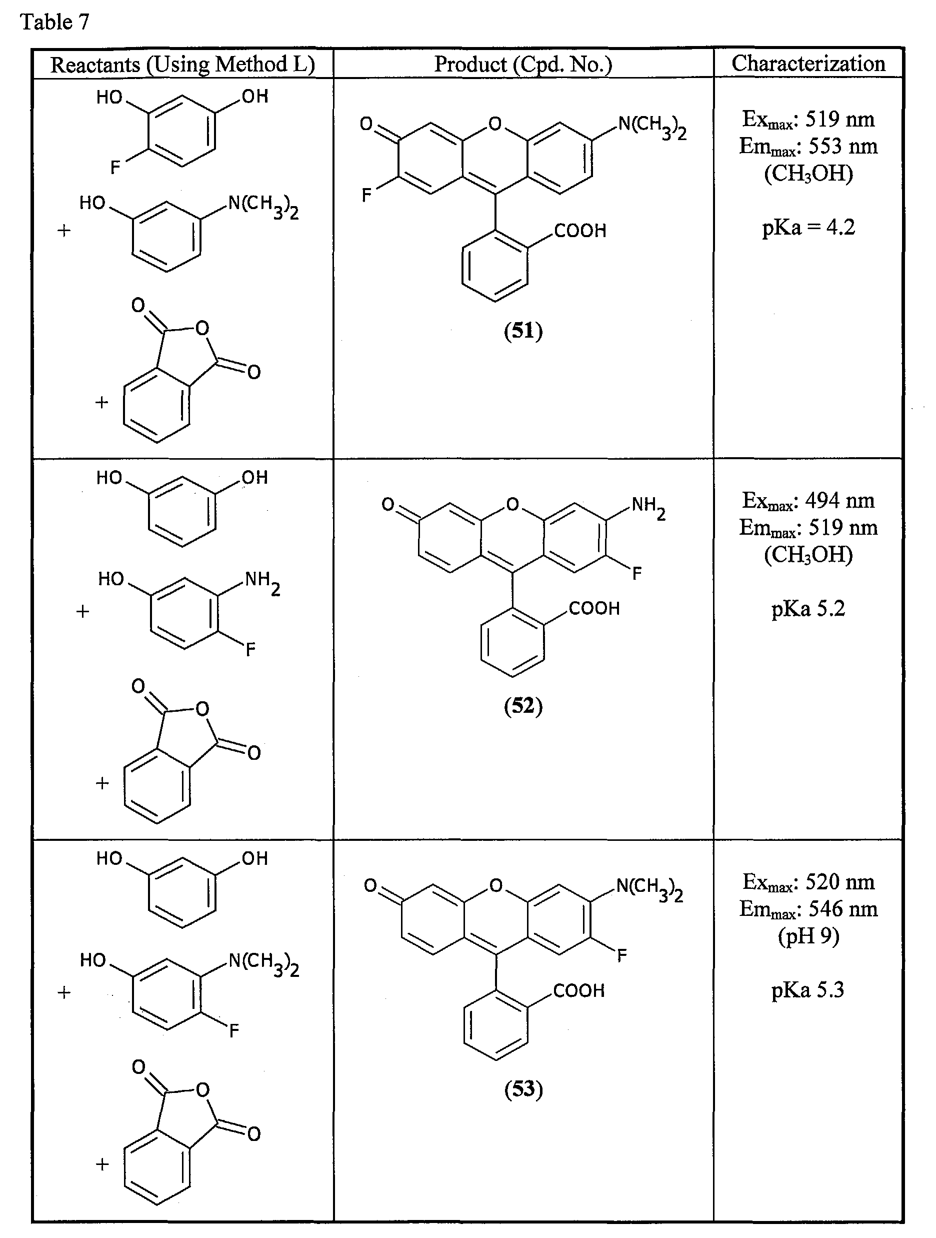

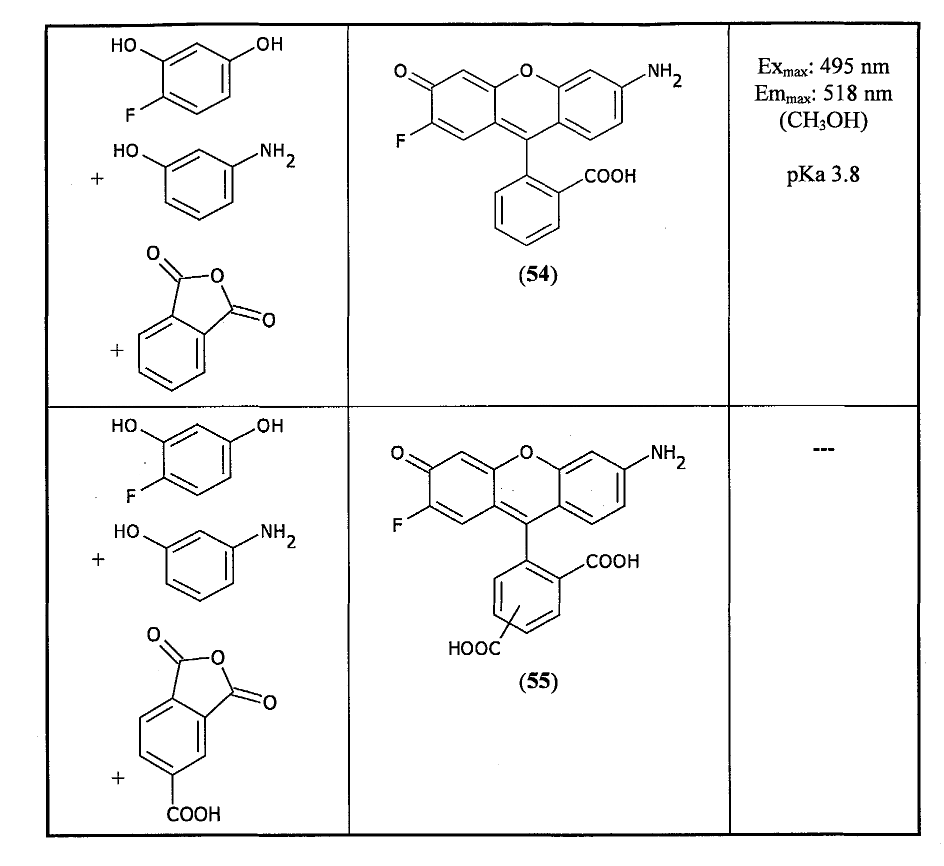

### Preparation of Fluorinated Xanthenes

### Preparation of 2,7-difluoro-6-hydroxy-9-trifluoromethylxanthene-3-one (56):

4-Fluororesorcinol (Compound **15**, 0.10 g, 0.78 mmol) is condensed with trifluoroacetic acid (45 mg, 0.39 mmol) at 80 °C in methanesulfonic acid. The desired product is precipitated from the reaction solution by the addition of an excess of water. The product is filtered and dried to give Compound **56** as a reddish powder.

### Preparation of 2,7-difluoro-6-hydroxy-1-(1-naphthyl)xanthene-3-one (57):

Two equivalents of 4-fluororesorcinol (Compound **15**) are condensed with one equivalent of naphthalene-1-carboxaldehyde in warm methanesulfonic acid. When the reaction is judged complete by TLC analysis, the intermediate product is precipitated from the reaction solution with water. The crude solid is filtered and dried. The intermediate is dissolved in chloroform and treated with excess chloramine-T. When oxidation is complete, as judged by TLC, the volatiles are removed and the crude product is purified by chromatography to give Compound 57.

### Preparation of Derivatized Fluorinated Fluorophores:

### General method M for the preparation of thioether derivatives:

A solution of the desired "bottom-ring" fluorinated fluorescein (1 equiv.) and mercaptoacetic acid (1.2 equiv.) in DMF, as 5% w/v solution, is heated at 80 °C for 40 minutes. The reaction is poured into ice water, extracted with ethyl acetate, washed with brine, dried, evaporated and purified using silica gel column chromatography eluting with CHCl₃:methanol:acetic acid (85:15:0.3) then THF:trifluoroacetic acid (100:0.5). Fractions containing pure product are combined and evaporated. The residue is dissolved in a minimum of THF, and filtered into petroleum ether (bp. 30-60 °C). The precipitate is collected, washed with petroleum ether, and dried.

### General method N for the preparation of succinimidyl ester derivatives:

To a pyridine solution of the appropriate fluorinated fluorescein is added 1.3 equiv. of succinimidyl trifluoroacetate (STFA, prepared from *N*-hydroxysuccinimide and trifluoroacetic anhydride). Additional STFA (2 x 1 equiv.) is added to force the reaction to completion. After 16 hours at 20 °C, the reaction mixture is diluted with ether, washed with 1 M citric acid, with brine, dried and evaporated. The residue is purified using column chromatography eluting with CHCl₃:methanol:acetic acid (95:5:0.1 to 80:20:0.1 stepping gradient) to yield the desired product.

Alternatively, a carboxylic acid-substituted fluorinated fluorescein is coupled to N-hydroxysuccinimide using a carbodiimide coupling agent, such as 1-ethyl-3-(3-dimethylaminopropyl)-carbodiimide hydrochloride (EDAC) or *N,N*-dicyclohexylcarbodiimide (DCC), by methods well-known in the art.

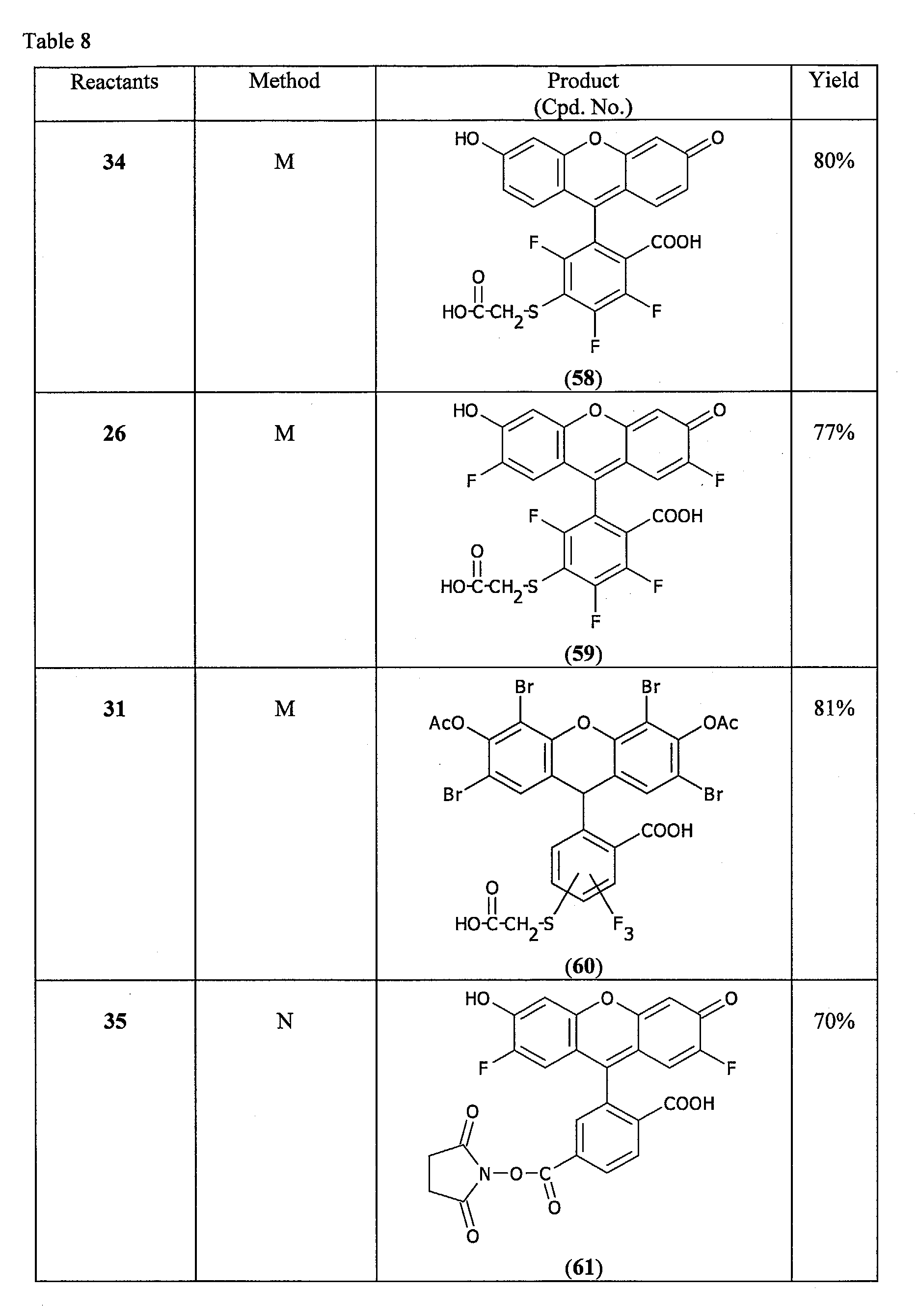

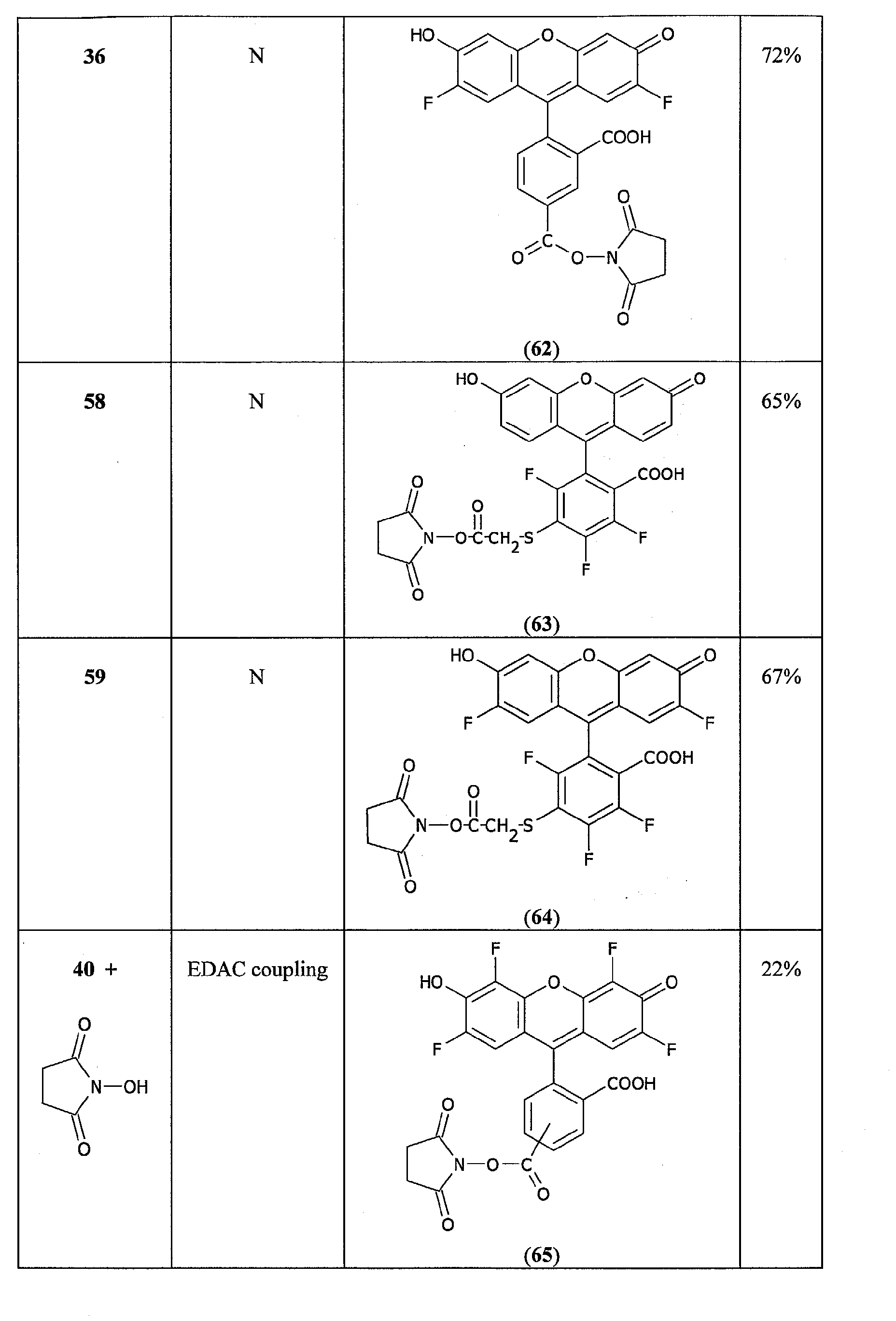

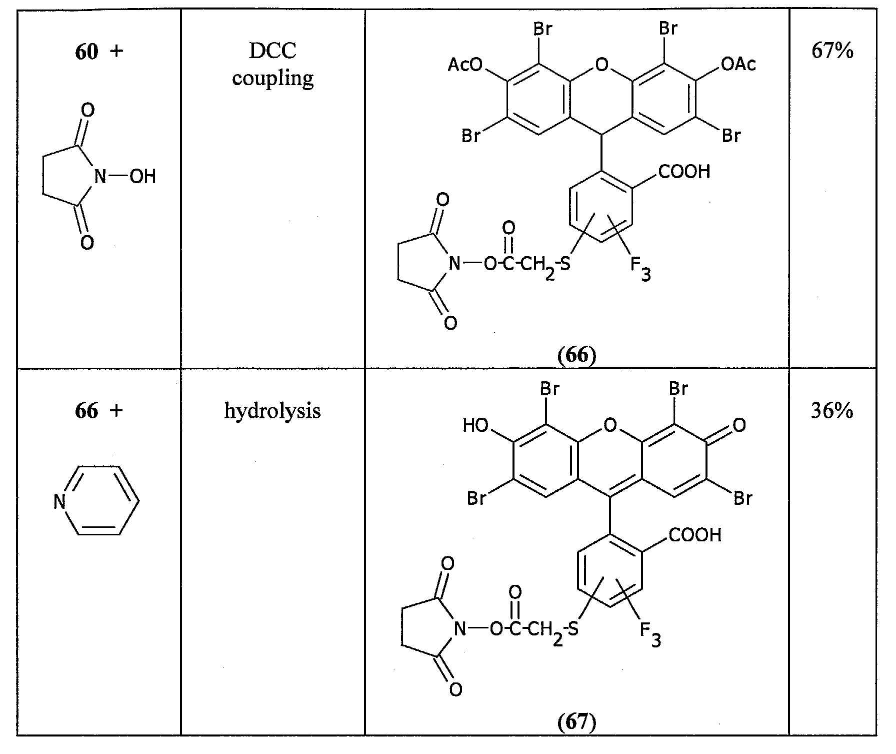

Other derivatives of fluorinated fluorescein are prepared by methods known in the art, as shown in Table 9

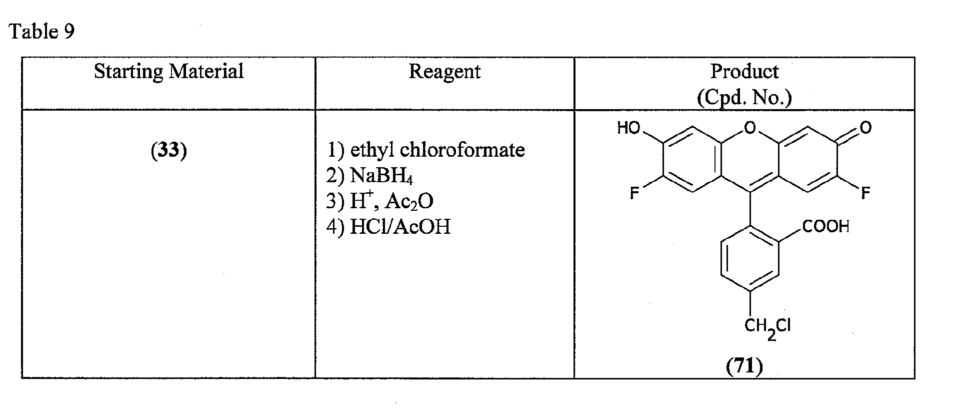

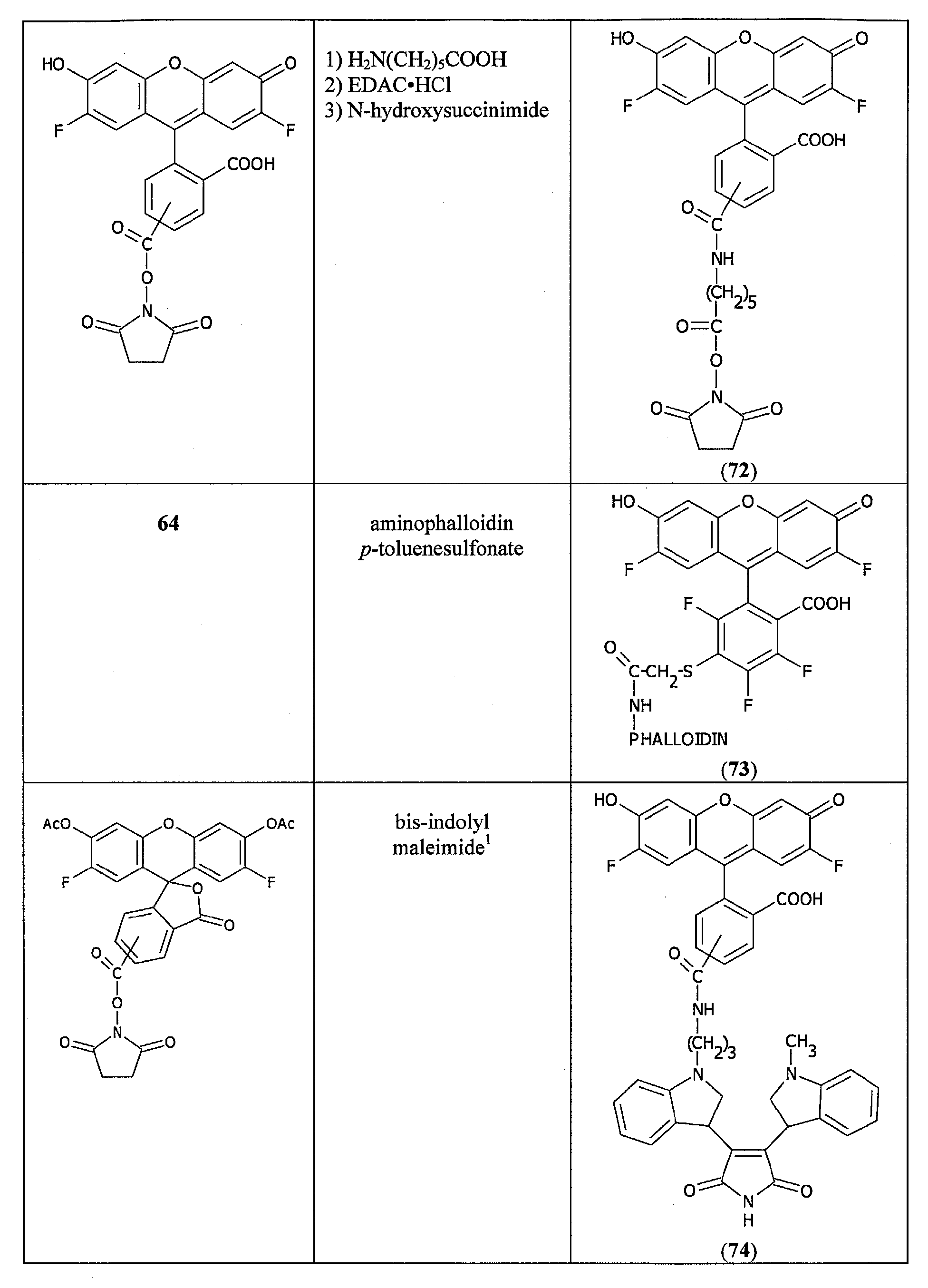

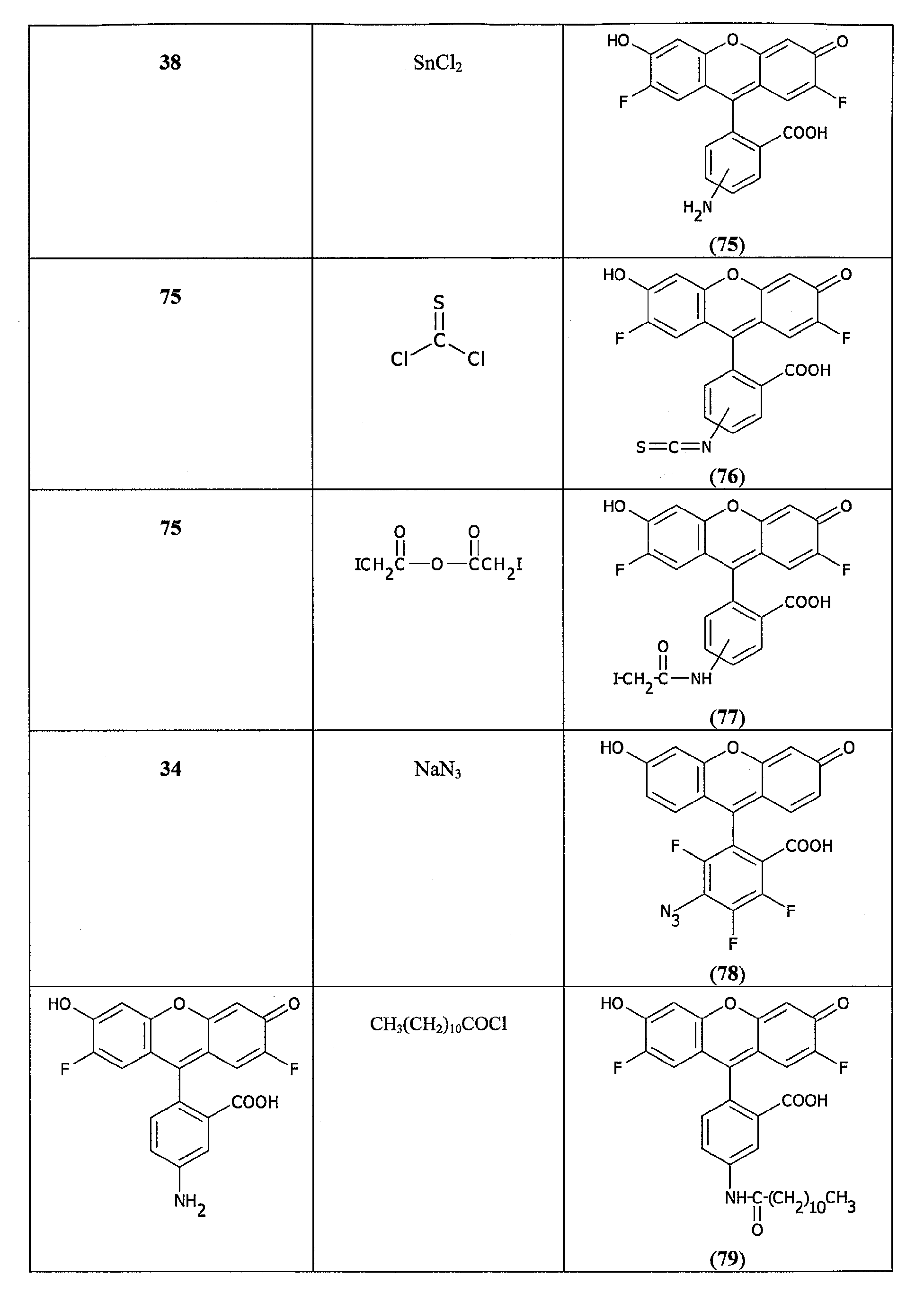

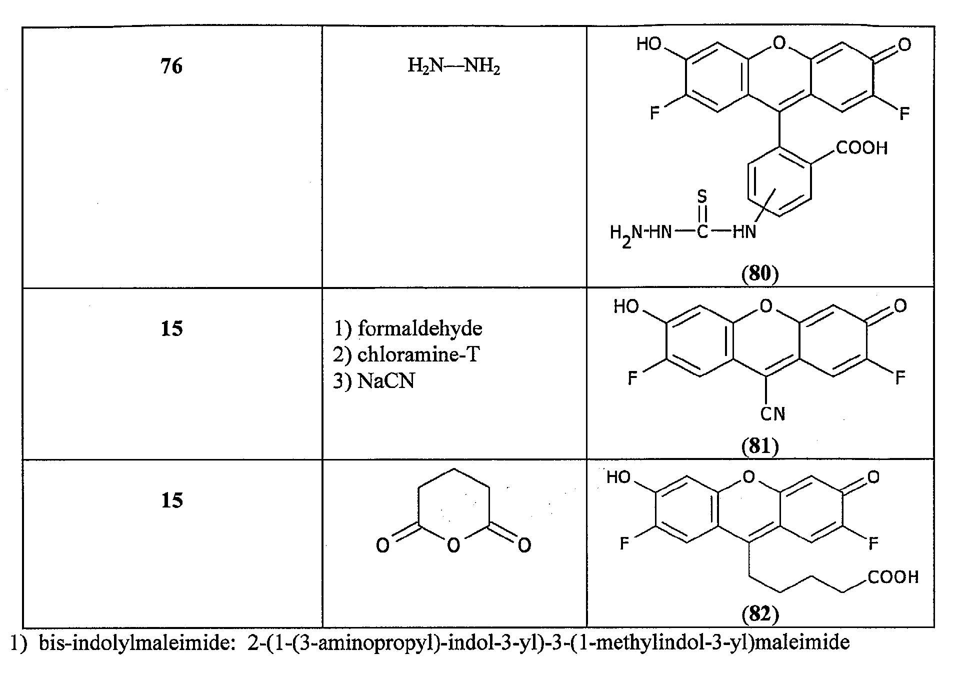

### Preparation of Fluorinated Sulfonefluoresceins and Their Analogs:

In a modification of General Method H, the condensation of an appropriately fluorinated resorcinol with sulfoterephthalic acid gives the desired fluorinated sulfonefluorescein.

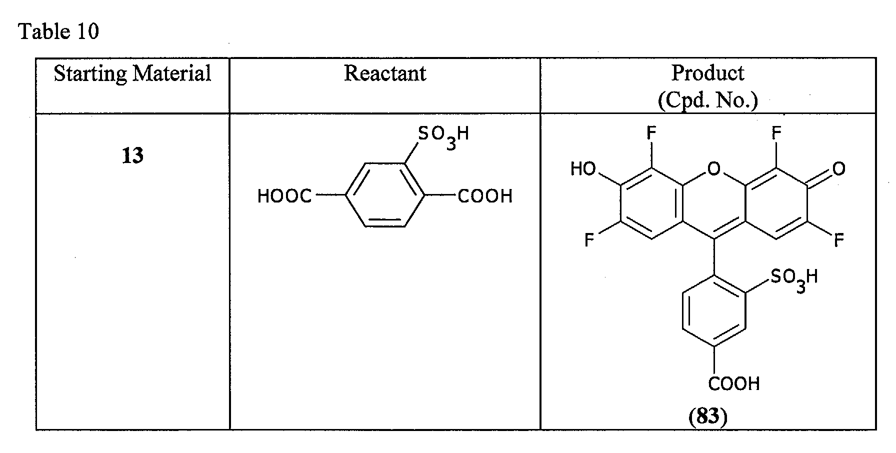

### Preparation of Fluorinated Dihydrofluoresceins:

Fluorinated fluoresceins are readily converted to their reduced dihydrofluorescein analogs. The appropriate fluorinated fluorescein is dissolved in acetic acid or acetic acid/methanol and treated with zinc dust under air at room temperature. After stirring 1-3 days, the mixture is filtered and the crude product is purified via chromatography on SEPHADEX LH-20.

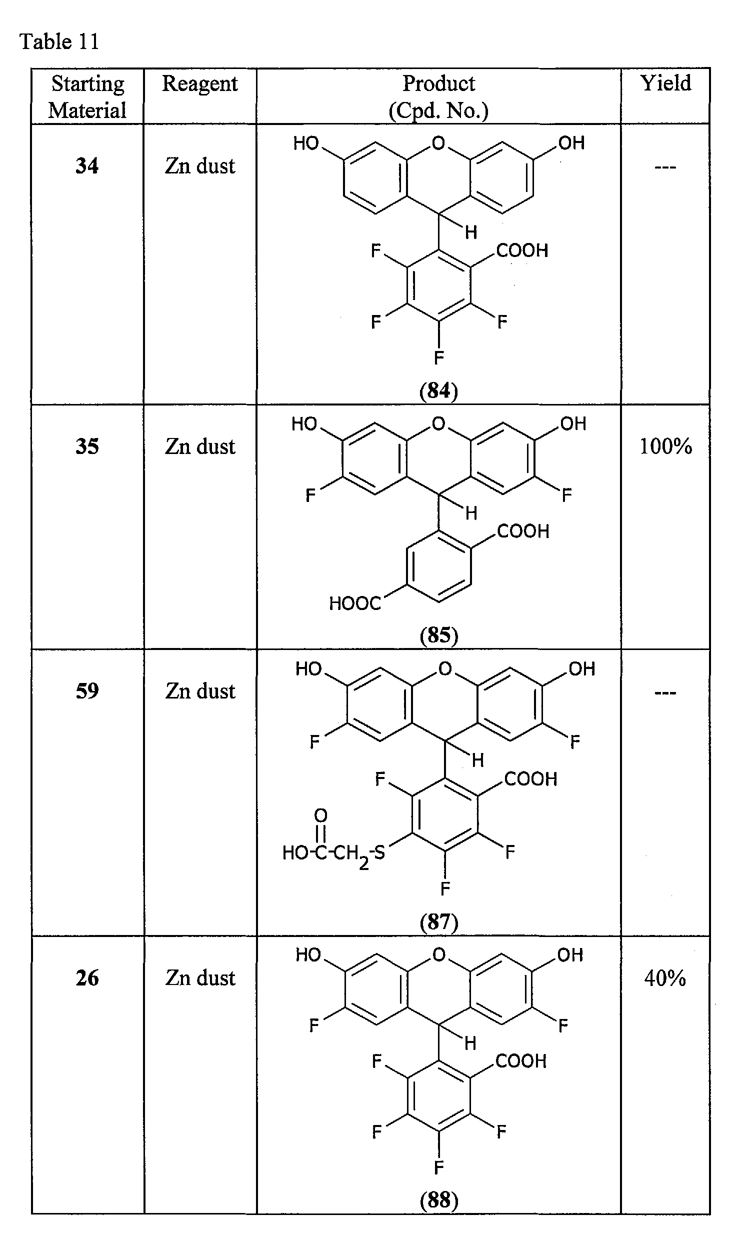

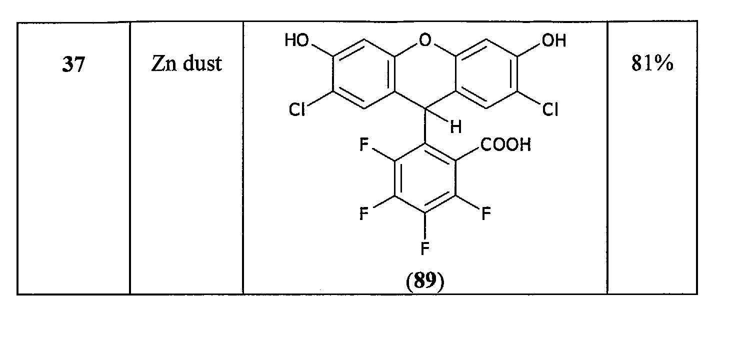

### Preparation of Fluorinated β-Galactosidase Substrates:

### General Method O

A mixture of the desired fluorinated fluorescein (1 equivalent), tetra-O-acetylbromogalactose (1.5 equivalents), and silver (I) oxide (1.5 equivalents) in anhydrous THF (0.02 M in dye) is stirred at room temperature under nitrogen. The reaction takes 72-96 hours to complete; more silver oxide is added as necessary. The mixture is filtered, and evaporated concentrated. The residue is purified via flash chromatography to give pure mono-alkylated product. Using this method, 2',7'-difluorofluorescein, tetra-O-acetylgalactoside (**90**) is prepared from Compound **29** in 92% yield, and 4,5,6,7-tetrafluorofluorescein, tetra-O-acetylgalactoside (**91**) is prepared from Compound **34** in 55% yield.

### General Method P

A mixture of the desired fluorinated fluorescein mono(tetra-O-acetyl)galactoside (1 equivalent), tetraacetobromogalactose (1.5 equivalents) and cadmium carbonate (1.5 equivalents) in dry toluene is heated at reflux for four days. The cooled reaction mixture is filtered and the filtrate concentrated. The residue is chromatographically purified to give the nonfluorescent protected galactosidase substrates. Using this method, 2',7'-difluorofluorescein, bis-tetra-O-acetylgalactoside (**92**) is prepared from Compound **90** in 62% yield, and 4,5,6,7-tetrafluorofluorescein, bis-tetra-O-acetylgalactoside (**93**) is prepared from Compound **91** in 16% yield.

The acetyl protecting groups are removed by reaction of the protected galactosidase with less than one equiv. of sodium methoxide or lithium hydroxide. The reaction is quenched with aqueous citric acid, and the product is purified by preparative TLC or chromatography on SEPHADEX LH-20. In this manner 2',7'-difluorofluorescein, bis-O-galactoside (94) is prepared from Compound **92**, and 4,5,6,7-tetrafluorofluorescein, bis-O-galactoside (**95**) is prepared from Compound **93** in 25% yield.

### Preparation of Alkaline Phosphatase Substrates:

### General Method Q

The general scheme for preparation of fluorinated fluorescein or rhodol phosphatase substrates typically requires initial phosphorylation of the fluorophore with phosphorus oxychloride. Typically, the fluorescein dye is dissolved in pyridine under nitrogen at 0 °C. To the fluorescein solution is added a pyridine solution of POCl₃. After the reaction is judged complete by TLC, the reaction is quenched with crushed ice and neutralized to pH 7.0 with NH₄OH. The pyridine is extracted with chloroform, and the aqueous phase is lyophilized. The crude material is purified on SEPHADEX LH20, eluting with water. Pure product fractions are pooled, frozen and lyophilized to give pure fluorinated fluorescein diphosphates as their tetraammonium salts, as pale yellow solids.

Using Method Q, 2',7'-difluorofluorescein (**29**) is converted into 2',7'-difluorofluorescein diphosphate, tetraammonium salt (**96**), and 2',4,5,6,7,7'-hexafluorofluorescein (26) is converted into 2',4,5,6,7,7'-hexafluorofluorescein diphosphate, tetraammonium salt (**97**).

### Preparation of Fluorinated Analogs of Calcein-AM:

### General Method R

The fluorinated dye is dissolved in ethanol and 6 M KOH is added to make a 0.2 M solution. The resulting solution is treated with 3 equiv. of iminodiacetic acid, followed by aqueous formaldehyde (4 equiv.). The solution is heated at 65 °C overnight, then doubled in volume with water/ethanol. The pH is lowered to 2.0 using aqueous HCl, and the precipitate collected by filtration. The intermediate is partially purified by trituration with acetone, followed by filtration, suspension in DMF and treatment with 10 equiv. diisopropylethylamine (DIEA) and acetic anhydride (4 equiv.). After 30 minutes, more DIEA (7 equiv.) is added, followed by water dropwise until homogeneity is achieved. Bromomethyl acetate (15 equiv.) is added, and the solution is stirred overnight. The mixture is partitioned between ethyl acetate and water. The organic layer is washed with brine, concentrated and then purified by preparative TLC, using hexanes:ethyl acetate as eluant, giving the pure product as a colorless oil.

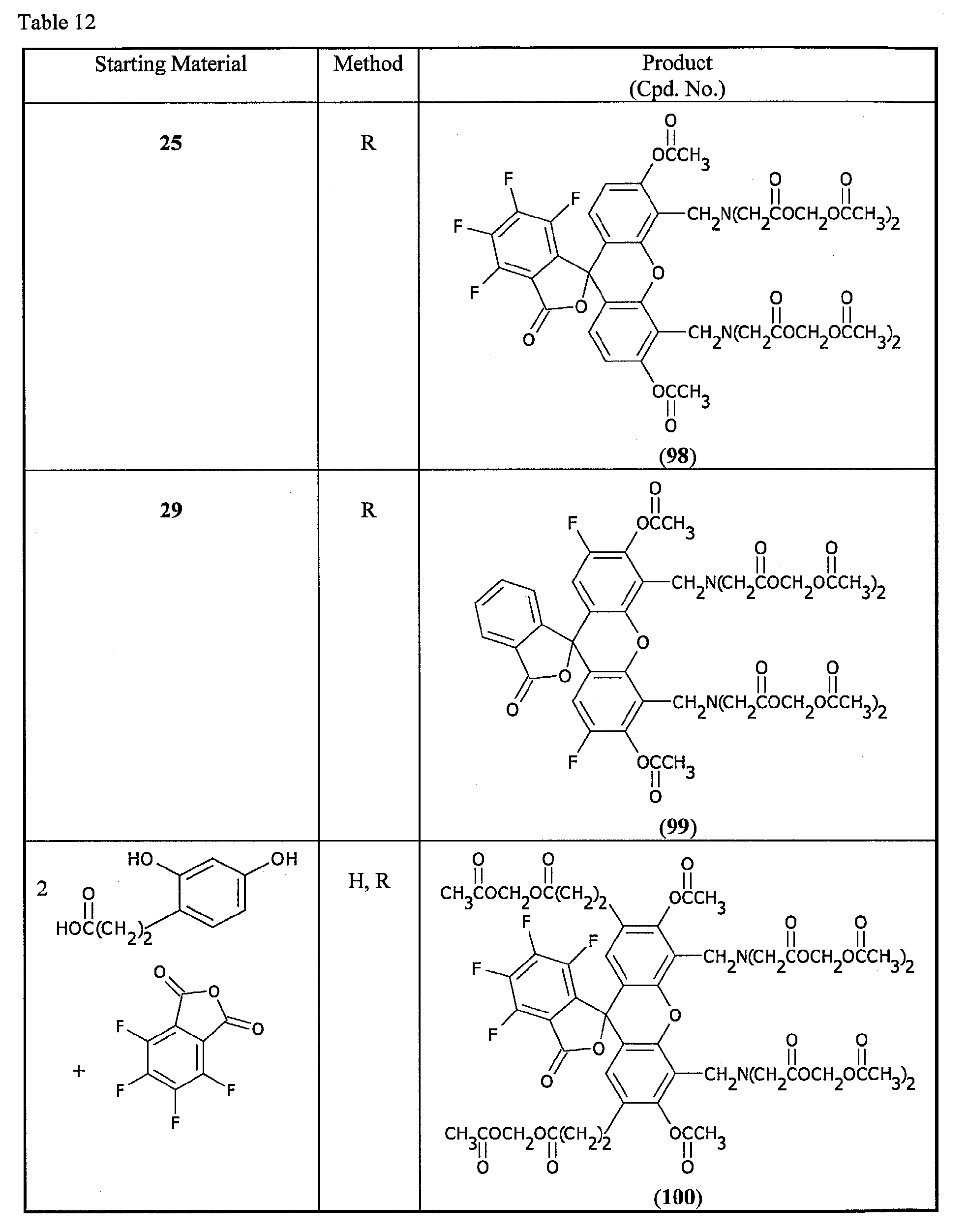

### Preparation of caged fluorinated fluorophores

Fluorinated fluorophores are caged using methods well-known in the art for caging fluoresceins. For example, treatment of 2',4,5,6,7,7'-hexafluorofluorescein (**26**) with 5-(t-butoxycarbonylmethoxy)-2-nitrobenzyl iodide and Ag₂O, followed by purification using chromatography yields non-fluorescent bis-(5-t-butoxycarbonylmethoxy)-2-nitrobenzyl-caged 2',4,5,6,7,7'hexafluorofluorescein (**101**) in 46% yield. The product is initially quenching, but becomes fluorescent upon irradiation.

### Preparation of ion indicators incorporating fluorinated fluorophores

Ion indicators incorporating fluorinated fluorophores are readily prepared utilizing published methods.

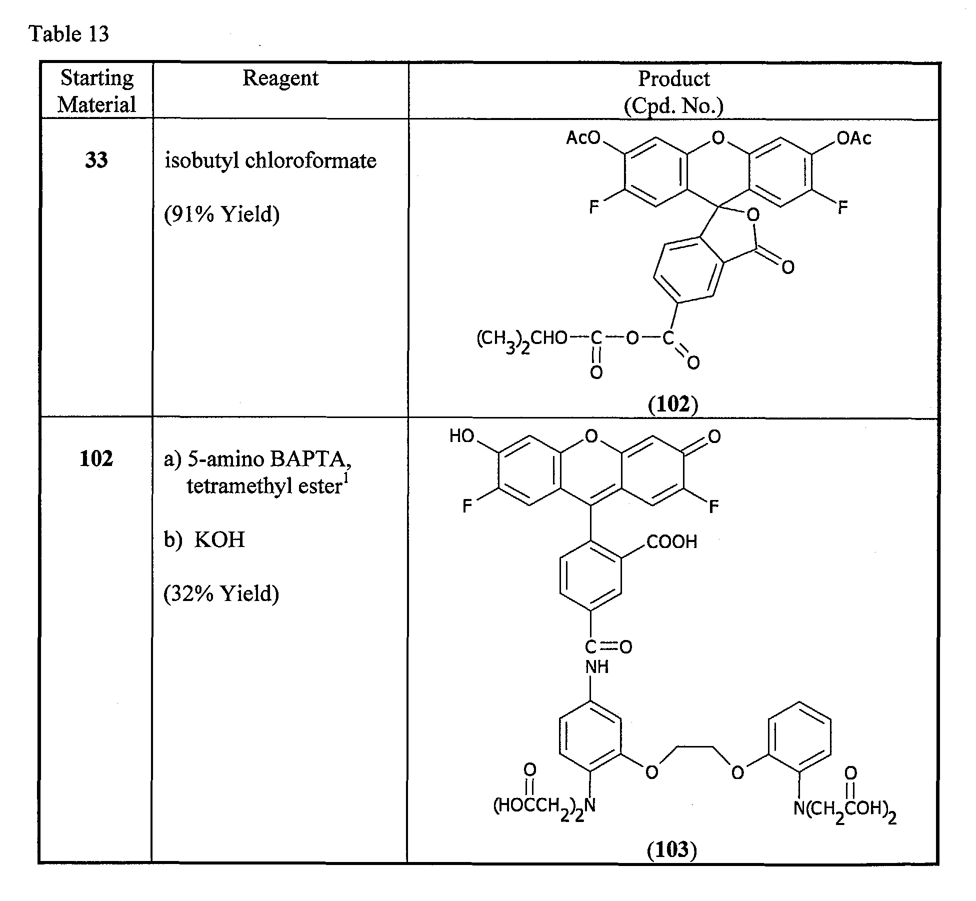

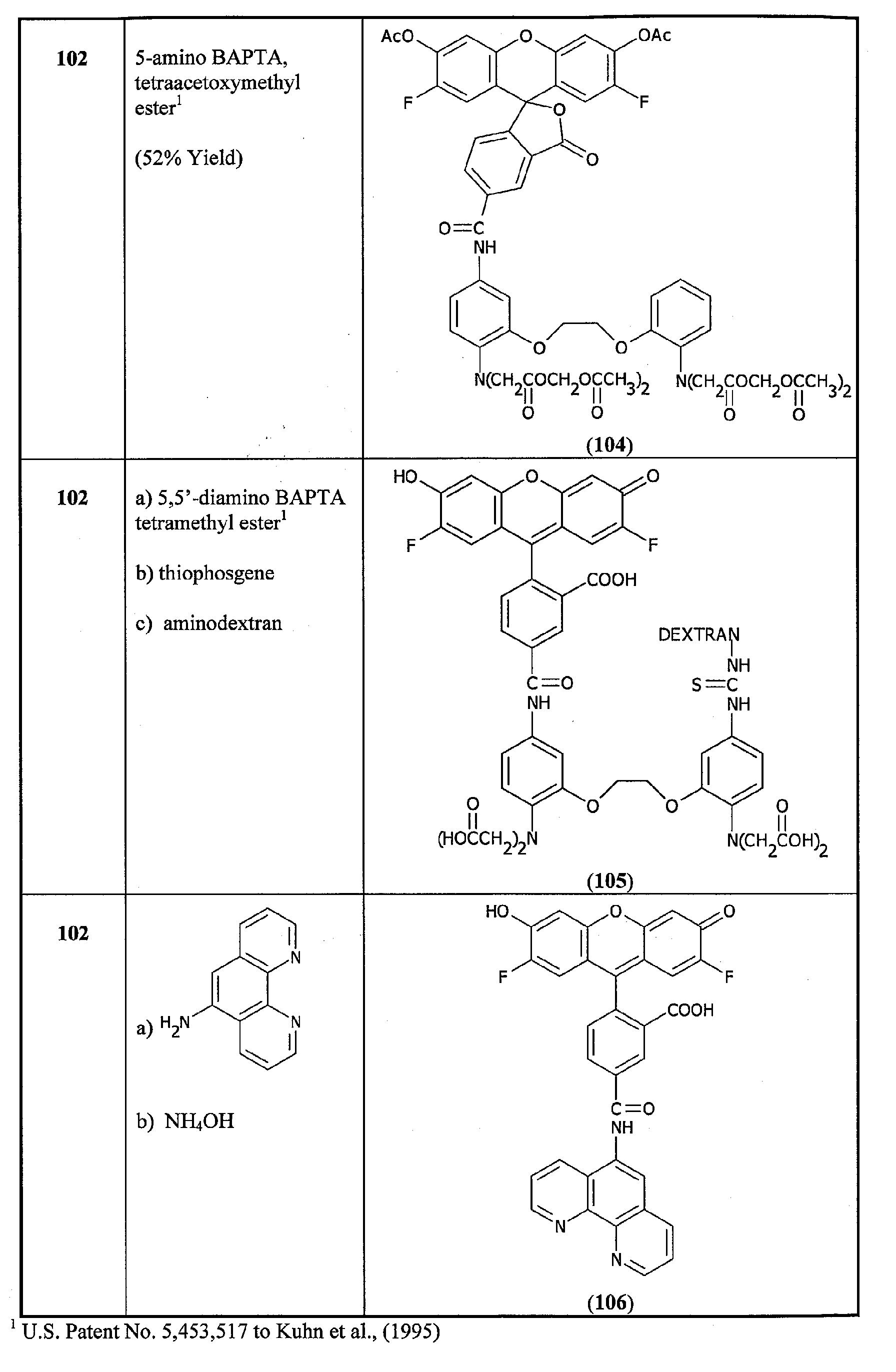

### Preparation of a nucleotide conjugate of 2',7'-difluorofluorescein-5-(and-6)-carboxylic acid:

To 2 mg of 5-(3-aminoallyl)-2'-deoxyuridine-5'-triphosphate, ammonium salt in 100 µL water is added 3 mg of Compound **72** in 100 of DMF, followed by 5 µL triethylamine. After 3 hours at room temperature, the solution is evaporated and the residue is purified on lipophilic SEPHADEX using water for elution. The first green fluorescent fractions are combined and lyophilized to give the fluorescent nucleotide conjugate as an orange solid (Compound **107**).

Alternatively a fluorescent conjugate (Compound **108**) of deoxyuridine-5'-triphosphate is prepared using 5-(3-amino-1-propynyl)-2'-deoxyuridine-5'-triphosphate in place of 5-(3-aminoallyl)-2'-deoxyuridine-5'-triphosphate (as described in Hobbs, Jr. et al, *supra*).

### Preparation of an oligonucleotide conjugate of 2',4',5',7'-tetrafluorofluorescein-5-(and-6)-carboxylic acid:

A sample of 500 µg of a 5'-amine modified, 24-base M13 primer sequence is dissolved in 220 µL 0.1 M NaHCO₃, pH 8.5. To this is added 1 mg of Compound **65** in 35 µL DMF. After 16 hours at room temperature, 15 µL of 5 M NaCl and 3 volumes of cold 100% ethanol are added. The mixture is cooled to -20°C, centrifuged, the ethanol supernate is decanted, and the pellet is rinsed and dissolved in 100 µL H₂O. The labeled oligonucleotide is purified by HPLC on a 300A C8 reverse phase column using a ramp gradient of 0.1 M triethylammonium acetate (pH ∼7) and acetonitrile (15→60% over 30 min). The desired peak is collected and evaporated to give the fluorescent oligonucleotide.

### Preparation of a drug conjugate of 2',7'-difluorofluorescein-5-(and-6)-isothiocyanate:

A fluorescent dopamine D₂ antagonist is prepared as follows:

To 10 mg of *N*-(*p*-aminophenethyl)spiperone (Amlaiky et al., FEBS LETT, **176**, 436 (1984)), and 10 µL *N*,*N*-diisopropylethylamine in 1 mL of DMF is added 15 mg of 2',7'-difluorofluorescein-5-(and-6)-isothiocyanate (Compound **76**, Example 77). After 3 hours, the reaction mixture is poured into 5 mL diethylether. The precipitate is collected by centrifugation and purified by chromatography on silica gel using 10% methanol in chloroform to give the pure product as an orange solid.

### Protein conjugates of fluorinated dyes:

A series of dye conjugates of goat anti-mouse IgG or streptavidin are prepared separately using Compound **64**, Compound **61**, 9-(4-carboxy-2-sulfophenyl)-2,7-difluoro-6-hydroxy-3*H*-xanthene-3-one, succinimidyl ester (Compound **109**); and fluorescein isothiocyanate as follows:

A solution of the desired protein is prepared at 10 mg/mL in 0.1 M sodium bicarbonate. The labeling reagents are dissolved in DMF at 10 mg/mL. Predetermined amounts of the labeling reagents are slowly added to the protein solutions with stirring. A molar ratio of 10 equiv. of dye to 1 equiv. of protein is typical, though the optimal amount varies with the particular labeling reagent and the protein being labeled. The reaction mixture is incubated at room temperature for one hour, or on ice for several hours. The dye-protein conjugate is separated from other reagents on CELLUFINE GH-25 equilibrated with PBS. The initial, protein-containing colored band is collected and the degree of labeling is determined by the absorbance at the absorbance maximum of each fluorophore, using the extinction coefficient of 68,000 cm⁻¹M⁻¹ for fluorescein at pH 8, and of 70,000 cm⁻¹M⁻¹ for the other three fluorinated fluorophores at their absorption maxima. The protein concentration is determined from the optical density at 280 nm corrected for the dye absorption at the same wavelength.

### Total fluorescence of selected dye-protein conjugates as a function of degree of substitution:

A series of goat anti-mouse IgG conjugates is prepared as described above using Compound **64**, Compound **61**, Compound **109**; and fluorescein isothiocyanate so as to yield derivatives with similar degrees of substitution. Fluorescence of the conjugates of the fluorinated fluoresceins is higher than that of FITC. Furthermore, fluorescence of antibody conjugates of Compound **59** and Compound **109** do not quench appreciably, even at high degrees of substitution, as shown in Figure 1.

### Labeling β-galactosidase with a fluorinated fluorescein diacetate:

*Escherichia coli* β-galactosidase (3 mg) in 150 µL phosphate-buffered saline pH 7.5 is treated with 13.6 µL of 1 mg/mL stock solution of Compound **25** in DMSO. After 1 hour the pH is raised to 10 with Na₂CO₃ for 2 hours to remove the acetates. Unreacted dye is removed on a spin column. The degree of substitution is estimated at 8.6 using ε = 85,600 cm⁻¹M⁻¹ at 515 nm. Direct labeling of β-galactosidase with Compound **34** at the same dye:protein ratio results in minimal protein labeling.

### Labeling and use of wheat germ agglutinin with 2',7'-difluorofluorescein:

Wheat germ agglutinin (25 mg, EY Laboratories) is dissolved in 5 mL sodium carbonate buffer pH 9.0 containing 5 mM N-acetylglucosamine to protect the active site. To this is added 3.5 mg of Compound **76**. After 1 hr at room temperature the solution is purified as described above for protein conjugates. Followed by lyophilization, a degree of substitution of 2-3 dyes per molecule is determined from the absorption at 490 nm. When used according to Sizemore et al. (U.S. Patent No. 5,137,810) the conjugate can distinguish between Gram-positive and Gram-negative bacteria.

### Labeling of actin filaments with Compound 73 and photobleaching:

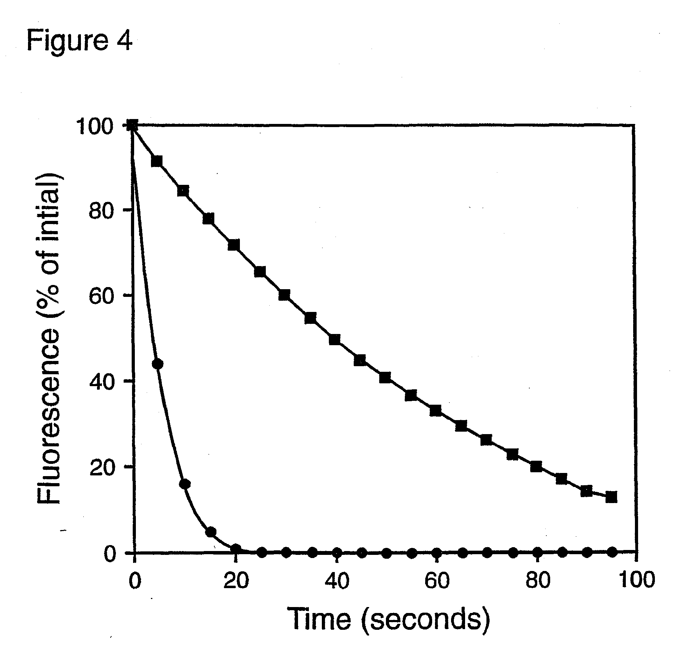

CRE BAG 2 fibroblasts are fixed with formaldehyde, permeabilized with acetone and then stained with the fluorescent phallotoxins fluorescein phalloidin and Compound **73**. The stained cells exhibit green fluorescent F-actin filaments. Each sample is continuously illuminated and viewed on a fluorescence microscope. Relative photobleaching, as shown in Figure 4, clearly demonstrates the superior photostability of the fluorinated dye-conjugate.

Once the F-actin filaments are stained, additional cellular components are optionally stained using other dye-conjugates having spectral properties that are readily distinguishable from those of the fluorinated dye-conjugate. For example, cell nuclei are stained fluorescent blue using DAPI, while cell antigens are stained red using a fluorescent antibody conjugates of a rhodamine or carbocyanine dye, such as TEXAS RED dye or CY-5 dye, respectively. Both the staining and subsequent visualization of the discrete cell components may be performed simultaneously or sequentially.

### Preparation and use of a fluorescent α-bungarotoxin:

α-Bungarotoxin (1 mg) in 25 µL 0.1 M NaHCO₃ is treated with 1.5 equivalents of Compound **63** at room temperature for 2 hours. The product is purified on CM-SEPHADEX. Staining of acetylcholine receptors and detection of their resulting fluorescence is comparable to that of fluorescein-conjugated α-bungarotoxin, except that the fluorescence of the fluorinated dye-conjugate is more resistant to photobleaching.

### Preparation of a dextran conjugate of Compound 76:

Compound 76 is treated with cyanuric chloride to yield the dichlorotriazine adduct (Compound **110**), which is used to label the free hydroxyl groups of a polysaccharide.

A 70,000 MW dextran (50 mg) is dissolved in 2.5 mL of 0.2 M Na₂CO₃ (pH 9.5). Compound **110** (20 mg in 1 mL DMSO) is added. The solution is heated to 50 °C for 6 hours while maintaining the pH at 9.5-10.0 with 1 M NaOH. The dye-dextran conjugate is purified on SEPHADEX G-15 using 30 mM ammonium acetate. The first colored band is collected and lyophilized.

### Preparation of aminodextran conjugates of fluorinated fluoresceins:

70,000 MW aminodextran (50 mg) is dissolved at 10 mg/mL in 0.1 M NaHCO₃. Compound **61** is added to a give dye/dextran ratio of 12. After 6 hours, the conjugate is purified on SEPHADEX G-50 eluting with water and the product is lyophilized. Typically ∼6 moles of dye are conjugated to 70,000 g dextran.

### Preparation of fluorescent liposomes:

Fluorescent liposomes containing polar derivatives, such as Compound **36** or 2',7'-difluorocalcein (prepared by hydrolysis of Compound **99**) on their interior are prepared and used essentially as described in J. BIOL. CHEM. 257, 13892 (1982) and PROC. NATL. ACAD. SCI. USA 75, 4194 (1978). Alternatively, liposomes containing lipophilic fluorinated fluoresceins within their membranes such as Compound **79** are prepared by codissolving the fluorescent lipid and the unlabeled phospholipid(s) that make up the liposome before forming the liposome dispersion essentially as described by Szoka, Jr. et al. (ANN. REV. BIOPHYS. BIOENG. 9, 467 (1980)).

### Preparations of a fluorescent low density lipoprotein:

Covalent conjugates of human low density lipoproteins (LDL), which are known to be taken up by macrophage, endothelial and other cells that possess "scavenger" receptors specific for the modified LDL, are prepared using the methods described for preparing protein conjugates, with purification by gel filtration. Binding of the fluorescent conjugates can be detected by either fluorescence microscopy or flow cytometry. Alternatively, LDL labeled in its lipid domain can be prepared by incubation of the LDL with the lipophilic fluorescent dye dispersed in buffer, followed by gel filtration.

### Preparation of fluorescent conjugates of bacteria:

Heat-killed *Escherichia coli* are suspended at 10 mg/mL in pH 8-9 buffer then incubated with 0.5-1.0 mg/mL of an amine-reactive fluorinated dye. After 30-60 minutes the labeled bacteria are centrifuged and washed with buffer to remove any unconjugated dye. Labeled bacteria that are opsonized are taken up by macrophage, as determined by flow cytometry.

### Utility of protein conjugates as immunoreagents and resistance to photobleaching:

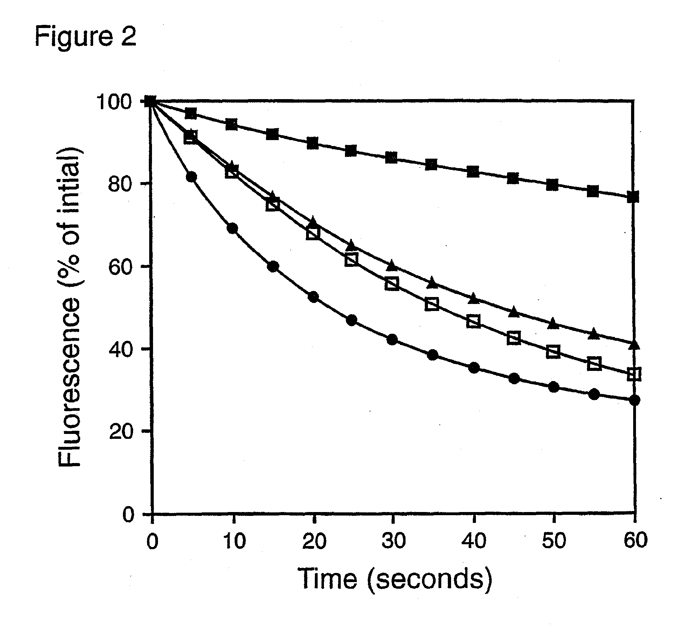

Antibody conjugates of Compound **59**; Compound **61**; Compound **109**; and fluorescein, succinimidyl ester are prepared with degrees of substitution of approximately 4-6. INOVA slides are rehydrated in 1% bovine serum albumin (BSA) in phosphate buffered saline (PBS) for 30 minutes. The slide is drained thoroughly. Human autoantibody is applied, the slide is incubated 30 minutes and rinsed with PBS. Mouse anti-human antibody is applied and the slide is incubated 30 minutes and rinsed with PBS. Each green fluorescent goat anti-mouse antibody conjugate is applied as a 10 µg/mL solution, diluted in 1% BSA/PBS. After 30 minutes, the labeled slides are rinsed in PBS, then in 50 mM Tris pH 8.0, mounted in 50 mM Tris pH 8.0, and viewed through a longpass fluorescein filter. All samples give predominantly nuclear staining. One image of the slide is acquired every 5 seconds for 100 seconds with coninuous illumination of the specimen, using a fluorescein long-pass filter. Three fields of cells are bleached using this method, and the photobleaching values are normalized and averaged. The average of three runs for each conjugate is then normalized and plotted. The results are shown in Figure 2. It is observed that antibody conjugates of the fluorinated dyes are significantly more photostable than the fluorescein conjugate.

### In situ hybridization of an RNA probe prepared using fluorescent nucleotide conjugates:

A UTP conjugate of Compound **72** is prepared using 5-(3-aminoallyl)-uridine-5'-triphosphate, ammonium salt (Sigma Chemical).

Mouse fibroblasts are fixed and prepared for mRNA *in situ* hybridization using standard procedures. A fluorophore-labeled RNA probe is prepared by *in vitro* transcription of a plasmid containing the mouse actin structural gene cloned downstream of a phage T3 RNA polymerase promoter. Labeling reactions consist of combining 2 µL DNA template (1 µg DNA), 1 µL each of 10 mM ATP, CTP and GTP, 0.75 µL 10 mM UTP, 2.5 µL 1 mM fluorescent UTP-Compound **72** conjugate, 2 µL 10X transcription buffer (400 mM Tris, pH 8.0, 100 mM MgCl₂, 20 mM spermidine, 100 mM NaCl), 1 µL T3 RNA polymerase (40 units/µL), 1 µL 2 mg/mL BSA, and 8.75 µL water. Reactions are incubated at 37 °C for two hours.

The DNA template is removed by treatment of the reaction with 20 units DNase I for 15 minutes, at 37°C. The RNA transcript is purified by extraction with an equal volume of phenol:chloroform, 1:1, then by chromatography through SEPHADEX G50. Labeled RNA is denatured for 5 minutes at 50°C, then hybridized to cellular preparations using standard procedures. When preparations are washed and viewed through a fluorescein filter on a fluorescence microscope, cells expressing actin mRNA show bright green fluorescence.

### The use of Compound 96 for the detection of acid phosphatase activity:

The rate of fluorescent product generation is measured by exciting a solution of 10 µM Compound **96** or fluorescein diphosphate (FDP) at 490 nm while monitoring the emission at 515 nm in a fluorometer in the presence of 0.1 units of prostatic acid phosphatase at pH 5. Under these conditions, acid phosphatase produces about a 6.5 times greater change in signal using Compound **96** than FDP.

### The use of Compound 94 for the detection of acid β-galactosidase activity:

The fluorescence resulting from the action of acid β-galactosidase from bovine testes on fluorescein digalactoside (FDG) and Compound **94** is compared. The fluorescence increase at 512 nm when excited at 490 nm is measured versus time for 50 µM substrate with 4.6 nM enzyme in assay buffer (200 mM sodium phosphate + 75 mM citric acid, pH 4.5). The initial rate of fluorescence production from Compound **94** is 3.0 times that obtained using FDG.

### The use of 4,5,7-trifluoro-6-methoxyfluorescein digalactoside (TFMFDG) and Compound 95 for the detection of endogenous (lysosomal) β-galactosidase activity in intact cells:

NIH 3T3 cells that are not transfected with exogenous β-galactosidase are cultured in Dulbecco's Modified Minimal Essential Medium supplemented with 10% fetal bovine serum and 2 mM L-glutamine. Stock solutions of TFMFDG and Compound **95** in PBS are added to the cell culture medium at 1 µM. After 30 minutes at 37 °C the cells are put on ice and assayed immediately by flow cytometry using a FACS-Vantage instrument equipped with an argon-ion laser (488 nm excitation). Fluorescence signals in FL1 normalized for the known fluorogenic substrate FDG show that TFMFDG and Compound **95** give signals 48.1 and 4.1 times that of FDG, respectively (Table 14).

**Table 14**

| **Substrate** | **Fluorescence Emission** |
|---|---|
| fluorescein digalactoside | 1.0 |
| Compound **95** | 4.1 |
| TFMFDG | 48.1 |

### The use of Compound 84 for the detection of horseradish peroxidase (HRP) activity in vitro:

A 2 mM solution (0.5 mL) of Compound **84** is prepared in water. An equal volume of OPD buffer (0.5 M Phosphate-citrate buffer) without hydrogen peroxide (pH 5.5, 0.5 mL) is added. Minor background fluorescence, indicating the presence of some contaminating oxidized dye, serves to provide a fluorescence signal at time 0. To another 0.5 mL of a 2 mM solution of Compound **84** is added OPD buffer with hydrogen peroxide (0.5 mL), causing no detectable fluorescence increase. Addition of HRP (1 µL, 10⁻⁴ Units) causes an immediate production of bright yellow fluorescence.

### Cell labeling with fluorinated fluorescein diacetates:

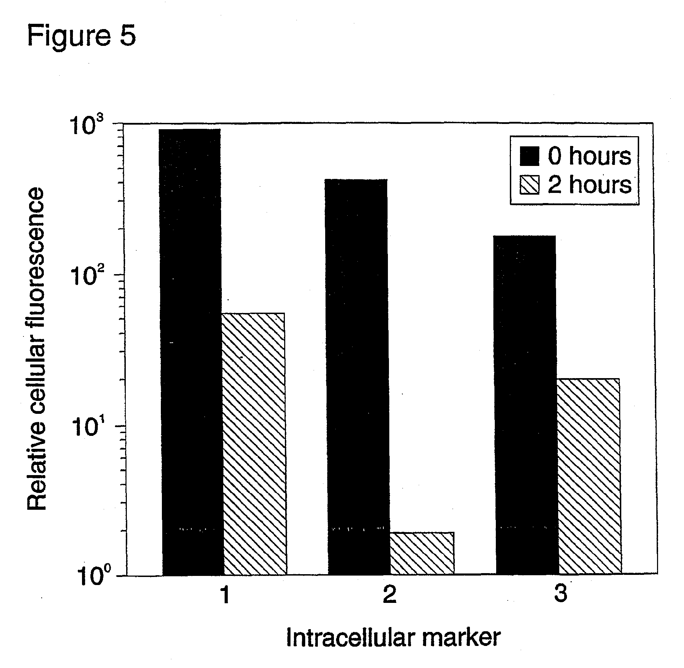

Cells from a human lymphoid B-cell culture in RPMI 1640 medium are treated with 1 µM Compound **25**, Compound **26**, or fluorescein diacetate (FDA) at 37 °C for 30 minutes. Following centrifugation and washing in PBS, the pellets are resuspended in RPMI 1640 medium for 15 min, recentrifuged, washed in PBS, fixed with 3.7% formaldehyde in PBS and analyzed by flow cytometry using 488 nm excitation. Cells stained with Compounds **25** and **26** show significantly higher fluorescence than those stained with FDA after two hours (Figure 5). Fluorescence in the cells can be detected for at least 24 hours and the dye does not transfer to other cells when the labeled cells are mixed with unlabeled cells. Cells stained with 2',4',5',7'-tetrafluorofluorescein diacetate are also weakly fluorescent. Alternatively, the stained and washed cells are viewed or analyzed without fixation.

### Detection of products of fluorinated dyes in cells:

Cells that have been stained with Compound **25** are gently lysed using PBS with 0.1% NP40. After removal of debris by centrifugation at 400 xg for 5 minutes, the supernatant is spin filtered by centrifugation for 30 minutes at 15,000 xg. The filtrate is analyzed using HPLC. The fluorescent products in the retained fraction are shown to contain both 4,5,6,7-tetrafluorofluorescein and its glutathione adduct (retention times 14.7 minutes and 11.6 minutes, respectively, as confirmed by HPLC of reference compounds prepared separately using 4,5,6,7-TFFDA). Using SDS gel electrophoresis, the cell lysate is shown to contain several proteins, only some of which are fluorescent.

### Quenching of the fluorescence of 2',7'-difluorofluorescein by rabbit polyclonal anti-fluorescein IgG (H+L) fraction:

Solutions of 5 x 10⁻⁹ M fluorescein and 5 x 10⁻⁹ M Compound **29** are prepared in 100 mM potassium phosphate buffer, pH 8.0. The fluorescence of each solution is read with excitation at 490 nm. Sequential additions of 0.5 mg/mL rabbit polyclonal anti-fluorescein IgG (H+L) fraction (Molecular Probes, Inc.) are added and the fluorescence measurements are repeated. The intensities are recorded in Table 15:

**Table 15:**

| Antibody volume (µL) | Fluorescence Intensity | |
|---|---|---|
| | fluorescein | Compound **29** |
| 0 | 100% | 100% |
| 2 | 84.6% | 83.6% |
| 4 | 68.8% | 67.3% |
| 6 | 54.3% | 51.9% |
| 8 | 38.5% | 36.5% |
| 10 | 22.9% | 23.8% |
| 12 | 11.7% | 13.4% |
| 15 | 4.4% | 6.3% |

The results show virtually identical binding and quenching of the two dyes.

### Procedure for pH titration of fluorinated fluorophores:

The dye of interest is first dissolved in a series of buffers that have each been calibrated using a pH meter. Acetate buffers are typically used in the range of pH 4-6, and phosphate buffers in the pH range 6-8. Absorption measurements are made using solutions that are approximately 10 µM in concentration, and fluorescence measurements are made using solutions that are approximately 1 µM in concentration. The absorption or emission data is then plotted vs. pH to determine pKa values. For example, Figure 3 shows the fluorescence emission data for 2',7'-difluorofluorescein (pKₐ ∼4.7) and fluorescein (pKₐ ∼6.4) plotted versus the pH of the solution. The data shows that fluorination of the fluorophore has lowered the pKa of fluorescein significantly. The pKa of the fluorinated fluorescein is even lower than that of 2',7'-dichlorofluorescein (pKₐ ∼5.1).

The disclosure includes the entire subject matter of WO 97/39064 from which the application is ultimately derived, including the subject matter of the following paragraphs:
1. A method of preparing a fluorinated resorcinol having the formula: 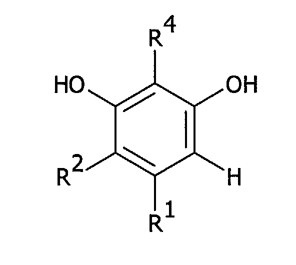 wherein
   R¹, R² and R⁴ are independently hydrogen, fluorine, chlorine, hydroxy, alkyl having 1-6 carbons or alkoxy having 1-6 carbons; provided at least one of R¹, R² and R⁴ is fluorine;
   comprising the steps of:
   a) treating a substituted nitrofluorobenzene having the formula 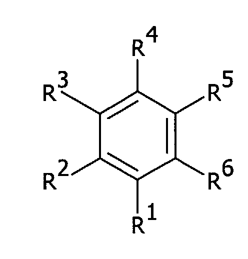 wherein
      R¹ and R² are independently hydrogen, fluorine, chlorine, alkyl having 1-6 carbons or alkoxy having 1-6 carbons;
      R⁴ is hydrogen, fluorine, chlorine, alkyl having 1-6 carbons, alkoxy having 1-6 carbons or nitro;
      R³ and R⁵ are independently alkoxy having 1-6 carbons, benzyloxy or F; and
      R⁶ is H or nitro;
      provided that
      exactly one of R⁴ and R⁶ is nitro;
      at least one of R³ and R⁵ is F; and
      at least one of R¹, R² and R⁴ is F;
      with a displacing ion that is an alkoxide ion having 1-6 carbons or a benzyloxide ion to yield a nitroresorcinol diether;
   b) reducing the nitroresorcinol diether to yield an aminoresorcinol diether;
   c) diazotizing the aminoresorcinol diether to yield a resorcinol diether diazonium salt;
   d) dediazotizing the resorcinol diether diazonium salt to yield a resorcinol diether; and
   e) cleaving the resorcinol diether to yield said fluorinated resorcinol.
2. A method, as in Claim 1, wherein the fluorinated resorcinol is 2-fluororesorcinol, 4-fluororesorcinol, 5-fluororesorcinol, 2,4-difluororesorcinol, 4,5-difluororesorcinol, 2,5-difluororesorcinol or 2,4,5-trifluororesorcinol.
3. A compound having the formula 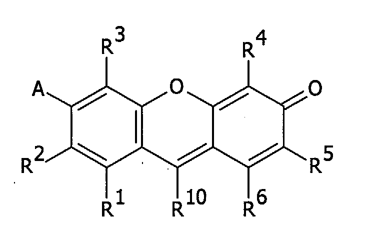 or the formula 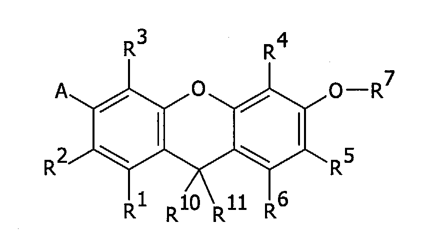 wherein
   R¹ and R⁶ are independently H, F, Cl, Br, I, C₁-C₁₈ alkyl or C₁-C₁₈ alkoxy;
   R², R³, R⁴ and R⁵ are independently H, F, Cl, Br, I, CN; or C₁-C₁₈ alkyl, C₁-C₁₈ alkoxy or C₁-C₁₈ alkylthio, where each alkyl, alkoxy or alkylthio is optionally further substituted by F, Cl, Br, I, sulfonic acid, salt of sulfonic acid, carboxylic acid, a salt of carboxylic acid, a carboxylic acid ester of a C₁-C₆ alcohol, a carboxylic acid ester of -CH₂-O-(C=O)-R¹⁸ where R¹⁸ is a C₁-C₆ alkyl, or amino, alkylamino, dialkylamino, or alkoxy, the alkyl portions of which independently have 1-6 carbons; or one or both of R³ and R⁴ are -CH₂N(CR¹⁹HCOOR¹⁷)₂, where R¹⁹ is H or a C₁-C₆ alkyl, R¹⁷ is H, a biologically compatible counterion, a linear or branched alkyl having 1-6 carbons, or -CH₂-O-(C=O)-R¹⁸;
   A is OR⁷ or NR⁸R⁹,
   where each R⁷ is independently H, C₁-C₁₈ alkyl or a C₁-C₁₈ acyl that is optionally substituted by amino, hydroxy, carboxylic acid, a salt of carboxylic acid, a carboxylic acid ester of a C₁-C₆ alcohol, a carboxylic acid ester of -CH₂-O-(C=O)-R¹⁸; or a trialkylsilyl wherein each alkyl group independently has 1-6 carbons;
   where each R⁸ and R⁹ are independently H, C₁-C₆ alkyl, C₁-C₆ carboxyalkyl, C₁-C₆ sulfoalkyl, a salt of C₁-C₆ carboxyalkyl, a salt of C₁-C₆ sulfoalkyl, or C₁-C₁₈ acyl wherein the alkyl portions are optionally substituted by amino, hydroxy, carboxylic acid, salt of carboxylic acid, a carboxylic acid ester of a C₁-C₆ alcohol, a carboxylic acid ester of -CH₂-O-(C=O)-R¹⁸, sulfonic acid, salt of sulfonic acid; or R⁸ in combination with R², or R⁹ in combination with R³, or both, form a saturated 5- or 6-membered ring that is optionally substituted by one or more methyls; or R⁸ in combination with R⁹ forms a saturated 5- or 6-membered heterocycle that is a piperidine, a morpholine, a pyrrolidine or a piperazine, each of which is optionally substituted by methyl, carboxylic acid, a salt of carboxylic acid, a carboxylic acid ester of a C₁-C₆ alcohol, or a carboxylic acid ester of -CH₂-O-(C=O)-R¹⁸;
   R¹⁰ is F, carboxylic acid, a salt of carboxylic acid, a carboxylic acid ester of a C₁-C₆ alcohol, or a carboxylic acid ester of -CH₂-O-(C=O)-R¹⁸; or R¹⁰ is C₁-C₁₈ alkyl, alkenyl or alkynyl that is optionally substituted one or more times by F, Cl, Br, carboxylic acid, a salt of carboxylic acid, a carboxylic acid ester of a C₁-C₆ alcohol, a carboxylic acid ester of -CH₂-O-(C=O)-R¹⁸, sulfonic acid, salt of sulfonic acid, amino, alkylamino, or dialkylamino, the alkyl groups of which have 1-6 carbons; or R¹⁰ has the formula 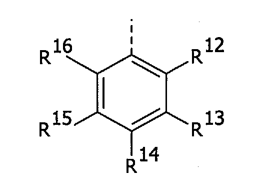 where R¹², R¹³, R¹⁴, R¹⁵ and R¹⁶ are independently H, F, Cl, Br, I; or sulfonic acid, salt of sulfonic acid, carboxylic acid, a salt of carboxylic acid, a carboxylic acid ester of a C₁-C₆ alcohol, a carboxylic acid ester of -CH₂-O-(C=O)-R¹⁸, CN, nitro, hydroxy, azido, amino, hydrazino; or C₁-C₁₈ alkyl, C₁-C₁₈ alkoxy, C₁-C₁₈ alkylthio, C₁-C₁₈ alkylamino, C₁-C₁₈ alkylester, C₁-C₁₈ alkylamido or C₁-C₁₈ arylamido, the alkyl or aryl portions of which are optionally substituted one or more times by F, Cl, Br, I, hydroxy, carboxylic acid, a salt of carboxylic acid, a carboxylic acid ester of a C₁-C₆ alcohol, a carboxylic acid ester of -CH₂-O-(C=O)-R¹⁸, sulfonic acid, salt of sulfonic acid, amino, alkylamino, dialkylamino or alkoxy, the alkyl portions of each having 1-6 carbons; or one pair of adjacent substituents R¹³ and R¹⁴, R¹⁴ and R¹⁵ or R¹⁵ and R¹⁶, when taken in combination, form a fused 6-membered aromatic ring that is optionally further substituted by carboxylic acid, a salt of carboxylic acid, a carboxylic acid ester of a C₁-C₆ alcohol, a carboxylic acid ester of -CH₂-O-(C=O)-R¹⁸; and
   R¹¹ is H, hydroxy, CN or a C₁-C₆ alkoxy; or R¹⁰ in combination with R¹¹ forms a 5-membered spirolactone ring or a 5-membered spirosultone ring; or R¹¹ in combination with R¹² forms a 5- or 6-membered spirolactone ring or a 5- or 6-membered spirosultone ring that is optionally and independently substituted by H, F or CH₃; or R¹⁰ when taken in combination with R¹¹ is a carbonyl oxygen;
   provided that at least one of R¹, R², R³, R⁴, R⁵, R⁶, R¹⁰, R¹², R¹³, R¹⁴, R¹⁵ or R¹⁶ is F, and, where only one of R¹, R², R³, R⁴, R⁵, R⁶, R¹⁰, R¹², R¹³, R¹⁴, R¹⁵ or R¹⁶ is F, R¹⁴ is not F.
4. A compound, as in paragraph 3, wherein at least one of R², R³, R⁴, R⁵, R⁷, R⁸, R⁹, R¹⁰, R¹², R¹³, R¹⁴, R¹⁵ and R¹⁶ is -L-Rₓ, or is modified to be -L-Rₓ, where each -L-Rₓ is optionally the same or different; and
   L is a single covalent bond, or L is a covalent linkage having 1-24 nonhydrogen atoms selected from the group consisting of C, N, O and S and is composed of any combination of single, double, triple or aromatic carbon-carbon bonds, carbon-nitrogen bonds, nitrogen-nitrogen bonds, carbon-oxygen bonds and carbon-sulfur bonds; and
   Rₓ is a reactive site.
5. A compound, as in paragraph 3, wherein at least one -L-Rₓ is modified to be -L-S_{c}, where each -L-S_{c} is optionally the same or different; and
   L is a single covalent bond, or L is a covalent linkage having 1-24 nonhydrogen atoms selected from the group consisting of C, N, O and S and is composed of any combination of single, double, triple or aromatic carbon-carbon bonds, carbon-nitrogen bonds, nitrogen-nitrogen bonds, carbon-oxygen bonds and carbon-sulfur bonds; and
   S_{c} is a conjugated substance.
6. A compound, as in paragraph 3, 4, or 5, wherein at least one of R⁷, R⁸ or R⁹ is modified to be a BLOCK;
   wherein each BLOCK moiety is independently a monovalent moiety derived by removal of a hydroxy group from phosphate or from sulfate, or a biologically compatible salt thereof; or a monovalent moiety derived by removal of a hydroxy group from a carboxy group of an aliphatic or aromatic carboxylic acid or of an amino acid, protected amino acid, peptide, or protected peptide; or a monovalent moiety derived by removal of a hydroxy group from an alcohol or from a mono- or polysaccharide, where said BLOCK is selected to be removable from said compound by action of an enzyme; or BLOCK is a photolabile caging group.
7. A compound, as in paragraph 3, 4, or 5, wherein at least one of R², R³, R⁴ or R⁵ is F.
8. A compound, as in paragraph 3, 4, or 5, wherein R² and R⁵ are F.
9. A compound, as in paragraph 3, 4, or 5, wherein at least three of R¹³, R¹⁴, R¹⁵, and R¹⁶ is F.
10. A compound, as in paragraph 3, 4, or 5, wherein R¹² is carboxylic acid, a salt of carboxylic acid, a sulfonic acid or a salt of sulfonic acid.
11. A compound, as in paragraph 3, 4, or 5, wherein R¹ and R⁶ are H and R², R³, R⁴, R⁵ are independently H, F, Cl, Br, I or C₁-C₆ alkoxy.
12. A compound, as in paragraph 3, 4, or 5, wherein A is OR⁷.
13. A compound, as in paragraph 12, wherein each R⁷ is H.
14. A compound, as in paragraph 3, 4, or 5 wherein R¹¹ is H.
15. A compound, as in paragraph 3, 4, or 5, wherein A is NR⁸R⁹.
16. A compound, as in paragraph 3, 4, or 5, wherein each of substituents R¹, R², R³, R⁴, R⁵, R⁶, R¹³, R¹⁴, R¹⁵ and R¹⁶ are either F or H.
17. A compound, as in paragraph 3, having the formula 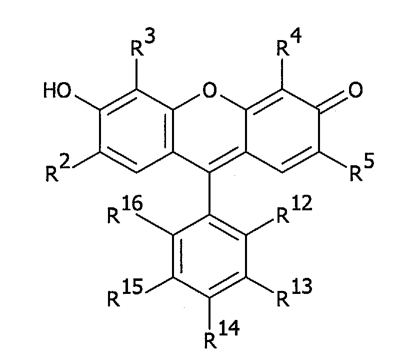 wherein
   R², R³, R⁴ and R⁵ are independently H or F;
   R¹² is carboxylic acid, a salt of carboxylic acid, a carboxylic acid ester of a C₁-C₆ alcohol, sulfonic acid or a salt of sulfonic acid;
   R¹³, R¹⁴, R¹⁵ and R¹⁶ are independently H; F; carboxylic acid; a salt of carboxylic acid; a carboxylic acid ester of a C₁-C₆ alcohol; a carboxylic acid ester of -CH₂-O-(C=O)-R^{18;} sulfonic acid; salt of sulfonic acid; or C₁-C₆ alkylthio that is optionally substituted by carboxylic acid, a salt of carboxylic acid, a carboxylic acid ester of a C₁-C₆ alcohol, or a carboxylic acid ester of -CH₂-O-(C=O)-R¹⁸.
18. A compound, as in paragraph 4 or 5, wherein each L independently contains 1-6 carbon atoms, is a single covalent bond, or the longest linear segment of L contains 4-10 nonhydrogen atoms including 1-2 heteroatoms.
19. A compound, as in paragraph 4 or 5, wherein exactly one of R¹², R¹³, R¹⁴, R¹⁵ and R¹⁶ is -L-Rₓ or -L-S_{c}.
20. A compound, as in paragraph 4 or 5, wherein exactly one of R², R³, R⁴, R⁵, R⁷, R⁸, R⁹ or R¹⁰ is -L-Rₓ or -L-S_{c}.
21. A compound, as in paragraph 4, wherein Rₓ is an acrylamide, an activated ester of a carboxylic acid, an acyl azide, an acyl halide, an acyl nitrile, hydroxy, an aldehyde, an alkyl halide, a sulfonate, an amine, an anhydride, an aniline, an aryl halide, an azide, an aziridine, a boronate, a carboxylic acid, a carbodiimide, a diazoalkane, an epoxide, a glycol, a haloacetamide, a halotriazine, a hydrazine, a hydroxylamine, an imido ester, an isocyanate, an isothiocyanate, a ketone, a maleimide, a phosphoramidite, a silyl halide, a sulfonyl halide, or a thiol group.
22. A compound, as in paragraph 21, wherein L is a single covalent bond and Rₓ is a carboxylic acid, an activated ester of a carboxylic acid, an amine, an azide, a haloacetamide, an alkyl halide, a sulfonyl halide, an isothiocyanate, or a maleimide group.
23. A compound, as in paragraph 5, wherein S_{c} is an amino acid, peptide, protein, polysaccharide, ion-complexing moiety, nucleotide, oligonucleotide, nucleic acid, drug, lipid, lipophilic polymer, non-biological organic polymer, animal cell, plant cell, bacterium, yeast, or virus.
24. A compound, as in paragraph 23, wherein S_{c} is a phospholipid, lipoprotein, lipopolysaccharide, or liposome.
25. A compound, as in paragraph 23, wherein S_{c} is an amino acid, peptide, protein, nucleotide, an oligonucleotide, a nucleic acid, an ion-complexing moiety, a polysaccharide, or a non-biological organic polymer or polymeric microparticle.
26. A compound, as in paragraph 25, wherein S_{c} is an antibody, antibody fragment, avidin, streptavidin, lectin, protein A or protein G.
27. A compound, as in paragraph 6, wherein A is OR⁷ and R⁷ is BLOCK.
28. A compound, as in paragraph 27, wherein BLOCK is an ester of phosphate, sulfate or an aliphatic or aromatic carboxylic acid.
29. A compound, as in paragraph 6, wherein BLOCK is an ether of an alcohol or a mono- or polysaccharide.
30. A compound, as in paragraph 6, wherein R⁷ and R⁸ are BLOCK and BLOCK is a photolabile caging group that is an *o*-nitroarylmethine, a 2-methoxy-5-nitrophenyl or a desyl moiety.
31. A compound, as in paragraph 6, wherein R⁹ is H; R⁸ is BLOCK where BLOCK is an amino acid, a peptide or a protected peptide; or R⁸ is a C₁-C₁₈ aliphatic carboxylic acid, arylalkanoic carboxylic acid or alkylaromatic carboxylic acid, the alkyl and aryl portion of which are optionally substituted one or more times by F, Cl, Br, I, hydroxy, carboxylic acid, a salt of carboxylic acid, a carboxylic acid ester of a C₁-C₆ alcohol, or a carboxylic acid ester of -CH₂-O-(C=O)-R¹⁸.
32. A method of staining a biological sample, comprising the steps of:
   a) preparing a dye solution comprising a dye compound as in any of paragraphs 4, 5, 6 or 7 in a concentration sufficient to yield a detectable optical response under the desired conditions;
   b) combining the sample of interest with the dye solution for a period of time sufficient for the dye compound to yield a detectable optical response upon illumination; and
   c) illuminating said sample at a wavelength selected to elicit said optical response.
33. A method of staining, as in paragraph 32, further comprising combining the sample with an additional detection reagent that has spectral properties that are detectably different from said optical response.
34. A method of staining, as in paragraph 32, further comprising the step of determining a characteristic of the sample by comparing the optical response with a standard response parameter.
35. A method of staining, as in paragraph 34, wherein the characteristic of the sample being determined is the activity of an oxidative enzyme in a sample, and where for said dye compound R¹¹ is H.
36. A method of staining, as in paragraph 34, wherein said sample comprises cells, and the characteristic of the sample being determined is the retention efficiency of said cells, and wherein the dye solution comprises a dye of the formula 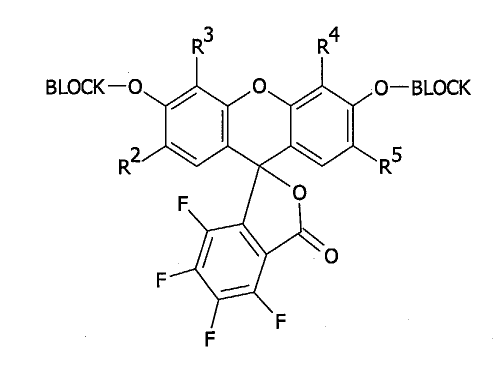 wherein
   R², R³, R⁴ and R⁵ are independently H, F, Cl, Br, I, CN, C₁-C₁₈ alkyl, C₁-C₁₈ alkoxy or C₁-C₁₈ alkylthio, where each alkyl, alkoxy or alkylthio is optionally further substituted by F, Cl, Br, I, amino, alkylamino, dialkylamino, or alkoxy, the alkyl portions of which independently have 1-6 carbons; or one or both of R³ and R⁴ are -CH₂N(CH₂COOR¹⁷)₂, where R¹⁷ is a linear or branched alkyl having 1-6 carbons, or -CH₂-O-(C=O)-R¹⁸ where R¹⁸ is a C₁-C₄ alkyl; and BLOCK is acetate;
   in at least a minimum concentration to give a detectable optical response that is fluorescence emission.
37. A method of staining, as in paragraph 32, wherein comparing comprises tracing the temporal or spatial location of the optical response within the biological sample.
38. A method of staining, as in paragraph 37, wherein for said dye compound
   R², R³, R⁴ and R⁵ are independently H or F;
   R¹² is carboxylic acid, a salt of carboxylic acid, a carboxylic acid ester of a C₁-C₆ alkyl, a carboxylic acid ester of -CH₂-O-(C=O)-R¹⁸, sulfonic acid or a salt of sulfonic acid;
   R¹³, R¹⁴, R¹⁵ and R¹⁶ are independently H; F; carboxylic acid; a salt of carboxylic acid; a carboxylic acid ester of a C₁-C₆ alkyl; a carboxylic acid ester of -CH₂-O-(C=O)-R^{18;} sulfonic acid; salt of sulfonic acid; or C₁-C₆ alkylthio that is optionally substituted by carboxylic acid, a salt of carboxylic acid, a carboxylic acid ester of a C₁-C₆ alkyl, or a carboxylic acid ester of -CH₂-O-(C=O)-R¹⁸.
39. A method of staining, as in paragraph 37, wherein for said dye compound
   one or both of R³ and R⁴ are -CH₂N(CR¹⁹HCOOR¹⁷)₂, where R¹⁷ is H, a biologically compatible counterion, a linear or branched alkyl having 1-6 carbons, or -CH₂-O-(C=O)-R¹⁸ where R¹⁸ is a C₁-C₄ alkyl; and R¹⁹ is H or CH₃;
   or
   one or R¹³, R¹⁴, R¹⁵ and R¹⁶ is carboxylic acid, a salt of carboxylic acid, a carboxylic acid ester of a C₁-C₆ alcohol, a carboxylic acid ester of -CH₂-O-(C=O)-R¹⁸ where R¹⁸ is a C₁-C₄ alkyl, sulfonic acid, salt of sulfonic acid, C₁-C₁₈ alkyl, C₁-C₁₈ alkoxy, C₁-C₁₈ alkylthio, C₁-C₁₈ alkylamino, C₁-C₁₈ alkylamido or C₁-C₁₈ arylamido.
40. A method of staining, as in paragraph 39, wherein one of R¹³, R¹⁴, R¹⁵ and R¹⁶ is C₁-C₁₈ alkyl, C₁-C₁₈ alkoxy, C₁-C₁₈ alkylthio, C₁-C₁₈ alkylamino, C₁-C₁₈ alkylamido or C₁-C₁₈ arylamido.
41. A method of staining, as in paragraph 37, wherein for said dye compound
   at least one of R¹³, R¹⁴, R¹⁵ and R¹⁶ is -L-Rₓ;
   where Rₓ is a haloacetamide, an alkyl halide, halomethylbenzamido or perfluorobenzamido.
42. A method of staining, as in paragraph 37, wherein for said dye compound
   at least one of R¹³, R¹⁴, R¹⁵ and R¹⁶ is -L-S_{c};
   where S_{c} is a protein, a polysaccharide, an ion-complexing moiety, a lipid, or a non-biological organic polymer or polymeric microparticle, that is optionally bound to one or more additional fluorophores that are the same or different.
43. A method of staining, as in paragraph 37, wherein for said dye compound
   at least one R⁷ is a BLOCK that is a monovalent moiety derived by removal of a hydroxy group from a carboxy group of a carboxylic acid, or BLOCK is a photolabile caging group.
44. A method of staining, as in paragraph 32 wherein said sample further comprises cells, and
wherein for said dye compound
at least one R7 is a BLOCK or H;
R¹¹ in combination with R¹² forms a 5- or 6-membered spirolactone ring or a 5- or 6-membered spirosultone ring; and
each of R¹³-R¹⁶ is F;
further comprising optionally washing the cells, after combining the sample with the dye compound, to remove residual dye compound that is outside said cells.

## Claims

1. A compound having the formula 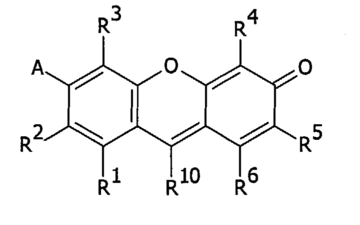 or the formula 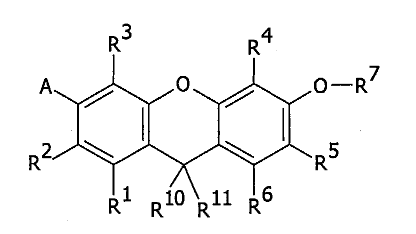 wherein
R¹ and R⁶ are independently H, F, Cl, Br, I, C₁-C₁₈ alkyl or C₁-C₁₈ alkoxy;
R², R³, R⁴ and R⁵ are independently H, F, Cl, Br, I, CN; or C₁-C₁₈ alkyl, C₁-C₁₈ alkoxy or C₁-C₁₈ alkylthio, where each alkyl, alkoxy or alkylthio is optionally further substituted by F, Cl, Br, I, sulfonic acid, salt of sulfonic acid, carboxylic acid, a salt of carboxylic acid, a carboxylic acid ester of a C₁-C₆ alcohol, a carboxylic acid ester of -CH₂-O-(C=O)-R¹⁸ where R¹⁸ is a C₁-C₆ alkyl, or amino, alkylamino, dialkylamino, or alkoxy, the alkyl portions of which independently have 1-6 carbons; or one or both of R³ and R⁴ are -CH₂N(CR¹⁹HCOOR¹⁷)₂, where R¹⁹ is H or a C₁-C₆ alkyl, R¹⁷ is H, a biologically compatible counterion, a linear or branched alkyl having 1-6 carbons, or -CH₂-O-(C=O)-R¹⁸;
A is OR⁷ or NR⁸R⁹,
where each R⁷ is independently H, C₁-C₁₈ alkyl or a C₁-C₁₈ acyl that is optionally substituted by amino, hydroxy, carboxylic acid, a salt of carboxylic acid, a carboxylic acid ester of a C₁-C₆ alcohol, a carboxylic acid ester of -CH₂-O-(C=O)-R¹⁸; or a trialkylsilyl wherein each alkyl group independently has 1-6 carbons;
where each R⁸ and R⁹ are independently H, C₁-C₆ alkyl, C₁-C₆ carboxyalkyl, C₁-C₆ sulfoalkyl, a salt of C₁-C₆ carboxyalkyl, a salt of C₁-C₆ sulfoalkyl, or C₁-C₁₈ acyl wherein the alkyl portions are optionally substituted by amino, hydroxy, carboxylic acid, salt of carboxylic acid, a carboxylic acid ester of a C₁-C₆ alcohol, a carboxylic acid ester of -CH₂-O-(C=O)-R¹⁸, sulfonic acid, salt of sulfonic acid; or R⁸ in combination with R², or R⁹ in combination with R³, or both, form a saturated 5- or 6-membered ring that is optionally substituted by one or more methyls; or R⁸ in combination with R⁹ forms a saturated 5- or 6-membered heterocycle that is a piperidine, a morpholine, a pyrrolidine or a piperazine, each of which is optionally substituted by methyl, carboxylic acid, a salt of carboxylic acid, a carboxylic acid ester of a C₁-C₆ alcohol, or a carboxylic acid ester of -CH₂-O-(C=O)-R¹⁸;
R¹⁰ is F, carboxylic acid, a salt of carboxylic acid, a carboxylic acid ester of a C₁-C₆ alcohol, or a carboxylic acid ester of -CH₂-O-(C=O)-R¹⁸; or R¹⁰ is C₁-C₁₈ alkyl, alkenyl or alkynyl that is optionally substituted one or more times by F, Cl, Br, carboxylic acid, a salt of carboxylic acid, a carboxylic acid ester of a C₁-C₆ alcohol, a carboxylic acid ester of -CH₂-O-(C=O)-R¹⁸, sulfonic acid, salt of sulfonic acid, amino, alkylamino, or dialkylamino, the alkyl groups of which have 1-6 carbons; or R¹⁰ has the formula 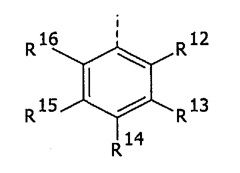 where R¹², R¹³, R¹⁴, R¹⁵ and R¹⁶ are independently H, F, Cl, Br, I; or sulfonic acid, salt of sulfonic acid, carboxylic acid, a salt of carboxylic acid, a carboxylic acid ester of a C₁-C₆ alcohol, a carboxylic acid ester of -CH₂-O-(C=O)-R¹⁸, CN, nitro, hydroxy, azido, amino, hydrazino; or C₁-C₁₈ alkyl, C₁-C₁₈ alkoxy, C₁-C₁₈ alkylthio, C₁-C₁₈ alkylamino, C₁-C₁₈ alkylester, C₁-C₁₈ alkylamido or C₁-C₁₈ arylamido, the alkyl or aryl portions of which are optionally substituted one or more times by F, Cl, Br, I, hydroxy, carboxylic acid, a salt of carboxylic acid, a carboxylic acid ester of a C₁-C₆ alcohol, a carboxylic acid ester of -CH₂-O-(C=O)-R¹⁸, sulfonic acid, salt of sulfonic acid, amino, alkylamino, dialkylamino or alkoxy, the alkyl portions of each having 1-6 carbons; or one pair of adjacent substituents R¹³ and R¹⁴, R¹⁴ and R¹⁵ or R¹⁵ and R¹⁶, when taken in combination, form a fused 6-membered aromatic ring that is optionally further substituted by carboxylic acid, a salt of carboxylic acid, a carboxylic acid ester of a C₁-C₆ alcohol, a carboxylic acid ester of -CH₂-O-(C=O)-R¹⁸; and
R¹¹ is H, hydroxy, CN or a C₁-C₆ alkoxy; or R¹⁰ in combination with R¹¹ forms a 5-membered spirolactone ring or a 5-membered spirosultone ring; or R¹¹ in combination with R¹² forms a 5- or 6-membered spirolactone ring or a 5- or 6-membered spirosultone ring that is optionally and independently substituted by H, F or CH₃; or R¹⁰ when taken in combination with R¹¹ is a carbonyl oxygen;
provided that at least one of R¹, R², R³, R⁴, R⁵, R⁶, R¹⁰, R¹², R¹³, R¹⁴, R¹⁵ or R¹⁶ is F, and, where only one of R¹, R², R³, R⁴, R⁵, R⁶, R¹⁰, R¹², R¹³, R¹⁴, R¹⁵ or R¹⁶ is F, R¹⁴ is not F.

2. A compound, as in Claim 1, wherein at least one of R², R³, R⁴, R⁵, R⁷, R⁸, R⁹, R¹⁰, R¹², R¹³, R¹⁴, R¹⁵ and R¹⁶ is -L-Rₓ, or is modified to be -L-Rₓ, where each -L-Rₓ is optionally the same or different; and
L is a single covalent bond, or L is a covalent linkage having 1-24 nonhydrogen atoms selected from the group consisting of C, N, O and S and is composed of any combination of single, double, triple or aromatic carbon-carbon bonds, carbon-nitrogen bonds, nitrogen-nitrogen bonds, carbon-oxygen bonds and carbon-sulfur bonds; and
Rₓ is a reactive site.

3. A compound, as in Claim 2, wherein at least one -L-Rₓ is modified to be -L-S_{c}, where each -L-S_{c} is optionally the same or different, wherein S_{c} is a conjugated substance.

4. A compound, as in any of Claims 1 to 3, wherein at least one of R⁷, R⁸ or R⁹ is modified to be a BLOCK;
wherein each BLOCK moiety is independently a monovalent moiety derived by removal of a hydroxy group from phosphate or from sulfate, or a biologically compatible salt thereof; or a monovalent moiety derived by removal of a hydroxy group from a carboxy group of an aliphatic or aromatic carboxylic acid or of an amino acid, protected amino acid, peptide, or protected peptide; or a monovalent moiety derived by removal of a hydroxy group from an alcohol or from a mono- or polysaccharide, where said BLOCK is selected to be removable from said compound by action of an enzyme; or BLOCK is a photolabile caging group.

5. A compound of claim 1 having the formula 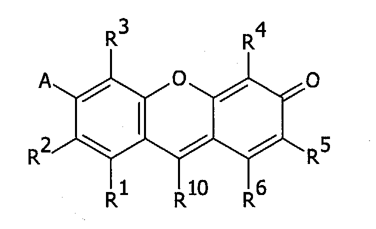 or the formula 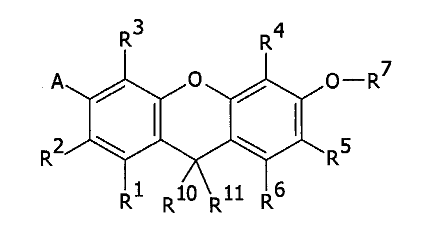 wherein
R¹ and R⁶ are independently H, F, Cl, Br, I, C₁-C₁₈ alkyl or C₁-C₁₈ alkoxy;
R², R³, R⁴ and R⁵ are independently H, F, Cl, Br, I, CN; or C₁-C₁₈ alkyl, C₁-C₁₈ alkoxy or C₁-C₁₈ alkylthio, where each alkyl, alkoxy or alkylthio is optionally further substituted by F, Cl, Br, I, sulfonic acid, salt of sulfonic acid, carboxylic acid, a salt of carboxylic acid, a carboxylic acid ester of a C₁-C₆ alcohol, a carboxylic acid ester of -CH₂-O-(C=O)-R¹⁸ where R¹⁸ is a C₁-C₆ alkyl, or amino, alkylamino, dialkylamino, or alkoxy, the alkyl portions of which independently have 1-6 carbons; or one or both of R³ and R⁴ are -CH₂N(CR¹⁹HCOOR¹⁷)₂, where R¹⁹ is H or a C₁-C₆ alkyl, R¹⁷ is H, a biologically compatible counterion, a linear or branched alkyl having 1-6 carbons, or -CH₂-O-(C=O)-R¹⁸;
R¹⁰ is F, carboxylic acid, a salt of carboxylic acid, a carboxylic acid ester of a C₁-C₆ alcohol, or a carboxylic acid ester of -CH₂-O-(C=O)-R¹⁸; or R¹⁰ is C₁-C₁₈ alkyl, alkenyl or alkynyl that is optionally substituted one or more times by F, Cl, Br, carboxylic acid, a salt of carboxylic acid, a carboxylic acid ester of a C₁-C₆ alcohol, a carboxylic acid ester of -CH₂-O-(C=O)-R¹⁸, sulfonic acid, salt of sulfonic acid, amino, alkylamino, or dialkylamino, the alkyl groups of which have 1-6 carbons; or R¹⁰ is an aryl moiety of the formula 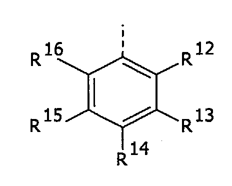 where R¹², R¹³, R¹⁴, R¹⁵ and R¹⁶ are independently H, F, Cl, Br, I; or sulfonic acid, salt of sulfonic acid, carboxylic acid, a salt of carboxylic acid, a carboxylic acid ester of a C₁-C₆ alcohol, a carboxylic acid ester of -CH₂-O-(C=O)-R¹⁸, CN, nitro, hydroxy, azido, amino, hydrazino; or C₁-C₁₈ alkyl, C₁-C₁₈ alkoxy, C₁-C₁₈ alkylthio, C₁-C₁₈ alkylamino, C₁-C₁₈ alkylester, C₁-C₁₈ alkylamido or C₁-C₁₈ arylamido, the alkyl or aryl portions of which are optionally substituted one or more times by F, Cl, Br, I, hydroxy, carboxylic acid, a salt of carboxylic acid, a carboxylic acid ester of a C₁-C₆ alcohol, a carboxylic acid ester of -CH₂-O-(C=O)-R¹⁸, sulfonic acid, salt of sulfonic acid, amino, alkylamino, dialkylamino or alkoxy, the alkyl portions of each having 1-6 carbons; or one pair of adjacent substituents R¹³ and R¹⁴, R¹⁴ and R¹⁵ or R¹⁵ and R¹⁶, when taken in combination, form a fused 6-membered aromatic ring that is optionally further substituted by carboxylic acid, a salt of carboxylic acid, a carboxylic acid ester of a C₁-C₆ alcohol, a carboxylic acid ester of -CH₂-O-(C=O)-R¹⁸; and
R¹¹ is H, hydroxy, CN or a C₁-C₆ alkoxy; or R¹⁰ in combination with R¹¹ forms a 5-membered spirolactone ring or a 5-membered spirosultone ring; or R¹¹ in combination with R¹² forms a 5- or 6-membered spirolactone ring or a 5- or 6-membered spirosultone ring that is optionally and independently substituted by H, F or CH₃; or R¹⁰ when taken in combination with R¹¹ is a carbonyl oxygen;
or wherein at least one of R², R³, R⁴, R⁵, R⁷, R¹⁰, R¹², R¹³, R¹⁴, R¹⁵ and R¹⁶ is -L-Rₓ or -L-S_{c}, where each -L-Rₓ and/or -L-S_{c} is the same or different; and
L is a single covalent bond, or L is a covalent linkage having 1-24 nonhydrogen atoms selected from the group consisting of C, N, O and S and is composed of any combination of single, double, triple or aromatic carbon-carbon bonds, carbon-nitrogen bonds, nitrogen-nitrogen bonds, carbon-oxygen bonds and carbon-sulfur bonds;
Rₓ is a reactive site; and
S_{c} is a conjugated substance;
A is OR⁷ and (i) at least one of R¹, R², R³, R⁴, R⁵ or R⁶ is F but the compound is not one in which R² and R⁵ are F and/or R³ and R⁴ are F, and/or (ii) at least three of R¹³ , R¹⁴, R¹⁵ and R¹⁶ are F; or
A is NR⁸R⁹ and (i) at least one of R¹, R², R³, R⁴, R⁵or R⁶is F, and/or (ii) at least three of R¹³, R¹⁴, R¹⁵ and R¹⁶ are F,
where each R⁷ is independently H; C₁-C₁₈ alkyl; a C₁-C₁₈ acyl that is optionally substituted by amino, hydroxy, carboxylic acid, a salt of carboxylic acid, a carboxylic acid ester of a C₁-C₆ alcohol, a carboxylic acid ester of -CH₂-O-(C=O)-R¹⁸; a trialkylsilyl wherein each alkyl group independently has 1-6 carbons; or a BLOCK,
where each R⁸ and R⁹ are independently H, C₁-C₆ alkyl, C₁-C₆ carboxyalkyl, C₁-C₆ sulfoalkyl, a salt of C₁-C₆ carboxyalkyl, a salt of C₁-C₆ sulfoalkyl, or C₁-C₁₈ acyl wherein the alkyl portions are optionally substituted by amino, hydroxy, carboxylic acid, salt of carboxylic acid, a carboxylic acid ester of a C₁-C₆ alcohol, a carboxylic acid ester of -CH₂-O-(C=O)-R¹⁸, sulfonic acid, salt of sulfonic acid; or R⁸ in combination with R², or R⁹ in combination with R³, or both, form a saturated 5- or 6-membered ring that is optionally substituted by one or more methyls; or R⁸ in combination with R⁹ forms a saturated 5- or 6-membered heterocycle that is a piperidine, a morpholine, a pyrrolidine or a piperazine, each of which is optionally substituted by methyl, carboxylic acid, a salt of carboxylic acid, a carboxylic acid ester of a C₁-C₆ alcohol, or a carboxylic acid ester of -CH₂-O-(C=O)-R¹⁸; or a BLOCK;
wherein each BLOCK moiety is independently a monovalent moiety derived by removal of a hydroxy group from phosphate or from sulfate, or a biologically compatible salt thereof; or a monovalent moiety derived by removal of a hydroxy group from a carboxy group of an aliphatic or aromatic carboxylic acid or of an amino acid, protected amino acid, peptide, or protected peptide; or a monovalent moiety derived by removal of a hydroxy group from an alcohol or from a mono- or polysaccharide, where said BLOCK is selected to be removable from said compound by action of an enzyme; or BLOCK is a photolabile caging group.

6. A compound, as in Claim 5, wherein at least one of R², R³, R⁴, R⁵, R⁷, R⁸, R⁹, R¹⁰, R¹², R¹³, R¹⁴, R¹⁵ and R¹⁶ is -L-Rₓ.

7. A compound, as in Claim 5 or Claim 6, wherein Rₓ is an acrylamide, an activated ester of a carboxylic acid, an acyl azide, an acyl halide, an acyl nitrile, hydroxy, an aldehyde, an alkyl halide, a sulfonate, an amine, an anhydride, an aniline, an aryl halide, an azide, an aziridine, a boronate, a carboxylic acid, a carbodiimide, a diazoalkane, an epoxide, a glycol, a haloacetamide, a halotriazine, a hydrazine, a hydroxylamine, an imido ester, an isocyanate, an isothiocyanate, a ketone, a maleimide, a phosphoramidite, a silyl halide, a sulfonyl halide, or a thiol group.

8. A compound, as in Claim 5 or Claim 6, wherein L is a single covalent bond and Rₓ is a carboxylic acid, an activated ester of a carboxylic acid, an amine, an azide, a haloacetamide, an alkyl halide, a sulfonyl halide, an isothiocyanate, or a maleimide group.

9. A compound, as in any of Claims 5 to 8, wherein each L independently contains 1-6 carbon atoms, is a single covalent bond, or the longest linear segment of L contains 4-10 nonhydrogen atoms including 1-2 heteroatoms.

10. A compound, as in any of Claims 5 to 9, wherein at least one of R², R³, R⁴, R⁵, R⁷, R⁸, R⁹, R¹⁰, R¹², R¹³, R¹⁴, R¹⁵ and R¹⁶ is -L-S_{c},.

11. A compound, as in any of Claims 5 to 9, wherein either
(i) exactly one of R¹², R¹³, R¹⁴, R¹⁵ and R¹⁶ is -L-Rₓ or -L-S_{c}; or
(ii) exactly one of R², R³, R⁴, R⁵, R⁷, R⁸, R⁹ or R¹⁰ is -L-Rₓ or -L-S_{c}.

12. A compound, as in Claim 9, wherein R¹² is an -L-Rₓ group which is carboxy or is such a group which has been converted to an -L-S_{c} group.

13. A compound, as in any of Claims 5 to 12, wherein S_{c} is
(i) an amino acid, peptide, protein, polysaccharide, ion-complexing moiety, nucleotide, oligonucleotide, nucleic acid, drug, lipid, lipophilic polymer, non-biological organic polymer, animal cell, plant cell, bacterium, yeast, or virus; or
(ii) a phospholipid, lipoprotein, lipopolysaccharide, or liposome; or
(iii) an amino acid, peptide, protein, nucleotide, an oligonucleotide, a nucleic acid, an ion-complexing moiety, a polysaccharide, or a non-biological organic polymer or polymeric microparticle; or
(iv) an antibody, antibody fragment, avidin, streptavidin, lectin, protein A or protein G.

14. A compound, as in any of Claims 5 to 13, wherein BLOCK is an ether of an alcohol or a mono- or polysaccharide.

15. A compound, as in any of Claims 5 to 13, wherein R⁷ and R⁸ are BLOCK and BLOCK is a photolabile caging group that is an *o*-nitroarylmethine, a 2-methoxy-5-nitrophenyl or a desyl moiety.

16. A compound, as in any of Claims 5 to 13, wherein R⁹ is H and R⁸ is BLOCK where BLOCK is an amino acid, a peptide or a protected peptide or R⁸ is a C₁-C₁₈ aliphatic carboxylic acid, arylalkanoic carboxylic acid or alkylaromatic carboxylic acid, the alkyl and aryl portion of which are optionally substituted one or more times by F, Cl, Br, I, hydroxy, carboxylic acid, a salt of carboxylic acid, a carboxylic acid ester of a C₁-C₆ alcohol, or a carboxylic acid ester of -CH₂-O-(C=O)-R¹⁸.

17. A compound, as in any of Claims 5 to 16, wherein A is OR⁷.

18. A compound, as in any of Claims 5 to 16, wherein when A is OR⁷ then at least three of R¹³, R¹⁴, R¹⁵ and R¹⁶ are F and claim 5 is modified so that it does not include the alternative that "at least one of R¹, R², R³, R⁴, R⁵ or R⁶ is F but the compound is not one in which R² and R⁵ are F and/or R³ and R⁴ are F".

19. A compound, as in Claim 17 or Claim 18, wherein each R⁷ is H

20. A compound, as in Claim 17 or Claim 18, wherein at least one R⁷ is a BLOCK.

21. A compound, as in Claim 20, wherein BLOCK is an ester of phosphate, sulfate or an aliphatic or aromatic carboxylic acid.

22. A compound, as in any of claims 5 to 16, wherein A is NR⁸R⁹.

23. A compound, as in Claim 22, wherein R⁵ is F.

24. A compound, as in any of Claims 5 to 22, wherein at least one of R², R³, R⁴ or R⁵ is F.

25. A compound, as in any of Claims 5 to 24, wherein R² and R⁵ are F.

26. A compound, as in any of claims 5 to 25, wherein R¹ and R⁶ are H and R², R³, R⁴, R⁵ are independently H, F, Cl, Br, I or C₁-C₆ alkoxy.

27. A compound, as in any of Claims 5 to 26 wherein R¹¹ is H.

28. A compound, as in any of Claims 5 to 27, wherein each of substituents R¹, R², R³, R⁴, R⁵, R⁶, R¹³, R¹⁴, R¹⁵ and R¹⁶ is either F or H.

29. A compound, as in any of Claims 5 to 28, which has the formula

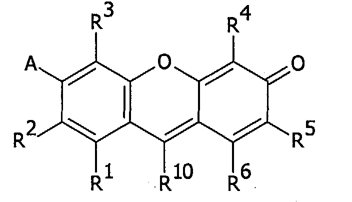

30. A compound, as in any of Claims 5 to 29, in which R¹⁰ has the formula

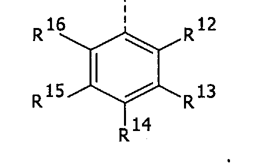

31. A compound, as in any of Claims 5 to 16, 18 or 24 to 28, which has the formula:

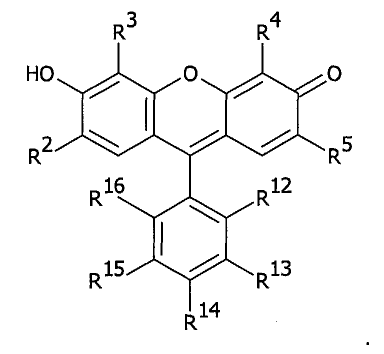

32. A compound, as in any of claims 5 to 20, wherein R¹² is carboxylic acid, a salt of carboxylic acid, a sulfonic acid or a salt of sulfonic acid.

33. A compound, as in any of Claims 5 to 32, in which at least three of R¹³, R¹⁴, R¹⁵ and R¹⁶ are F.

34. A compound, as in Claim 33, wherein R¹², R¹³, R¹⁴, R¹⁵ and R¹⁶ are F.

35. A compound, as in any of Claims 5 to 34, wherein each of substituents R¹, R², R³, R⁴, R⁵, R⁶, R¹³, R¹⁴, R¹⁵ and R¹⁶ is either F or H.

36. A compound, as in Claim 5 or claim 18, having the formula 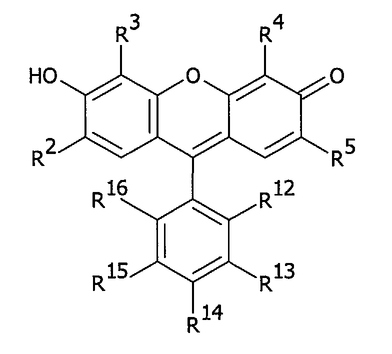 wherein
R², R³, R⁴ and R⁵ are independently H or F;
R¹² is carboxylic acid, a salt of carboxylic acid, a carboxylic acid ester of a C₁-C₆ alcohol, sulfonic acid or a salt of sulfonic acid;
R¹³, R¹⁴, R¹⁵ and R¹⁶ are independently H; F; carboxylic acid; a salt of carboxylic acid; a carboxylic acid ester of a C₁-C₆ alcohol; a carboxylic acid ester of -CH₂-O-(C=O)-R^{18;} sulfonic acid; salt of sulfonic acid; or C₁-C₆ alkylthio that is optionally substituted by carboxylic acid, a salt of carboxylic acid, a carboxylic acid ester of a C₁-C₆ alcohol, or a carboxylic acid ester of -CH₂-O-(C=O)-R¹⁸;
or wherein exactly one of R¹³, R¹⁴, R¹⁵ and R¹⁶ is -L-Rₓ as defined in any of claims 5 or 7 to 9 or -L-S_{C} as defined in any of Claims 5, 9 or 13.

37. A compound, as in Claims 36, wherein exactly one of R¹³, R¹⁴, R¹⁵ and R¹⁶ is -L-R_{X} or -L-S_{C}.

38. A compound, as in Claim 37, wherein said one of R¹³, R¹⁴, R¹⁵ and R¹⁶ is -L-Rₓ and Rₓ is as defined in Claim 8.

39. A compound, as in Claim 37, wherein said one of R¹³, R¹⁴, R¹⁵ and R¹⁶ is -L-S_{c} and S_{c} is as defined in Claim 13 (iii).

40. A compound, as in any of Claims 5 to 28, which has the formula:

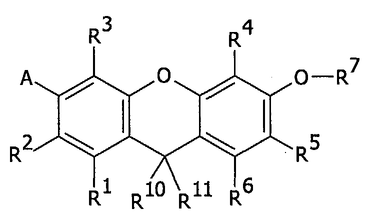

41. A compound of claim 40 wherein R¹¹ is H.

42. A compound having the formula 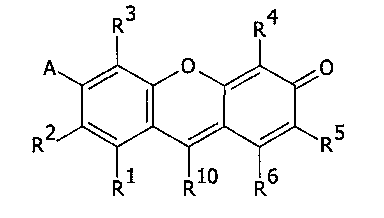 wherein
R¹, R², R³, R⁴, R⁵, R⁶ and A are as defined in Claim 5; and
exactly one of R², R³, R⁴, R⁵, R⁷, R⁸, R⁹ and R¹⁰ is -L-S_{c};
wherein L is a single covalent bond, or L is a covalent linkage having 1-24 nonhydrogen atoms selected from the group consisting of C, N, O and S and is composed of any combination of single, double, triple or aromatic carbon-carbon bonds, carbon-nitrogen bonds, nitrogen-nitrogen bonds, carbon-oxygen bonds and carbon-sulfur bonds; and
S_{c} is a conjugated substance that is an ion-complexing moiety.

43. A compound, as claimed in Claim 42, wherein the ion-complexing moiety is a crown ether, a BAPTA, an APTRA, a pyridine, or a phenanthroline.

44. A compound, as claimed in Claim 42 or Claim 43, wherein R² and R⁵ are each F.

45. A compound, as claimed in any of claims Claim 42 to 44, wherein A is as defined in Claim 18 or Claim 22

46. A method of staining a biological sample, comprising the steps of:
a) preparing a dye solution comprising a dye compound as in any of Claims 5 to 45, in a concentration sufficient to yield a detectable optical response under the desired conditions;
b) combining the sample of interest with the dye solution for a period of time sufficient for the dye compound to yield a detectable optical response upon illumination; and
c) illuminating said sample at a wavelength selected to elicit said optical response.

47. A method of staining, as in Claim 46, further comprising
(i) combining the sample with an additional detection reagent that has spectral properties that are detectably different from said optical response; and/or
(ii) the step of determining a characteristic of the sample by comparing the optical response with a standard response parameter.

48. A method of staining, as in Claim 47(ii), wherein the characteristic of the sample being determined is the activity of an oxidative enzyme in a sample, and where for said dye compound R¹¹ is H.

49. A method of staining, as in any of Claims 46, 47 or 48 which further comprises tracing the temporal or spatial location of the optical response within the biological sample.

50. A method of staining, as in Claim 49, wherein for the dye compound
R², R³, R⁴ and R⁵ are independently H or F;
R¹² is carboxylic acid, a salt of carboxylic acid, a carboxylic acid ester of a C₁-C₆ alkyl, a carboxylic acid ester of -CH₂-O-(C=O)-R¹⁸, sulfonic acid or a salt of sulfonic acid;
R¹³, R¹⁴, R¹⁵ and R¹⁶ are independently H; F; carboxylic acid; a salt of carboxylic acid; a carboxylic acid ester of a C₁-C₆ alkyl; a carboxylic acid ester of -CH₂-O-(C=O)-R^{18;} sulfonic acid; salt of sulfonic acid; or C₁-C₆ alkylthio that is optionally substituted by carboxylic acid, a salt of carboxylic acid, a carboxylic acid ester of a C₁-C₆ alkyl, or a carboxylic acid ester of -CH₂-O-(C=O)-R¹⁸.

51. A method of staining, as in any of Claims 46 to 50, wherein for the dye compound
one or both of R³ and R⁴ are -CH₂N(CR¹⁹HCOOR¹⁷)₂, where R¹⁷ is H, a biologically compatible counterion, a linear or branched alkyl having 1-6 carbons, or -CH₂-O-(C=O)-R¹⁸ where R¹⁸ is a C₁-C₄ alkyl; and R¹⁹ is H or CH₃;
or
one of R¹³, R¹⁴, R¹⁵ and R¹⁶ is carboxylic acid, a salt of carboxylic acid, a carboxylic acid ester of a C₁-C₆ alcohol, a carboxylic acid ester of -CH₂-O-(C=O)-R¹⁸ where R¹⁸ is a C₁-C₄ alkyl, sulfonic acid, salt of sulfonic acid, C₁-C₁₈ alkyl, C₁-C₁₈ alkoxy, C₁-C₁₈ alkylthio, C₁-C₁₈ alkylamino, C₁-C₁₈ alkylamido or C₁-C₁₈ arylamido;
and optionally wherein one of R¹³, R¹⁴, R¹⁵ and R¹⁶ is C₁-C₁₈ alkyl, C₁-C₁₈ alkoxy, C₁-C₁₈ alkylthio, C₁-C₁₈ alkylamino, C₁-C₁₈ alkylamido or C₁-C₁₈ arylamido.

52. A method of staining, as in Claim 43, wherein for the dye compound
(i) at least one of R¹³, R¹⁴, R¹⁵ and R¹⁶ is -L-Rₓ, where Rₓ is a haloacetamide, an alkyl halide, halomethylbenzamido or perfluorobenzamido; or
(ii) at least one of R¹³, R¹⁴, R¹⁵ and R¹⁶ is -L-S_{c}, where S_{c} is a protein, a polysaccharide, an ion-complexing moiety, a lipid, or a non-biological organic polymer or polymeric microparticle, that is optionally bound to one or more additional fluorophores that are the same or different; or
(iii)at least one R⁷ is a BLOCK that is a monovalent moiety derived by removal of a hydroxy group from a carboxy group of a carboxylic acid or that is a photolabile caging group.

53. A method of staining, as in Claim 46 or 47 wherein the sample further comprises cells, and wherein for the dye compound
at least one R⁷ is a BLOCK or H;
R¹¹ in combination with R¹² forms a 5- or 6-membered spirolactone ring or a 5- or 6-membered spirosultone ring; and
each of R¹³-R¹⁶ is F;
the method optionally further comprising washing the cells, after combining the sample with the dye compound, to remove residual dye compound that is outside said cells.

54. The use of a compound of claim 27 as a peroxidase substrate which fluoresces on oxidation.
